(19) Europäisches Patentamt
European Patent Office
Office européen des brevets

(11) EP 3 495 474 B1

(12) EUROPÄISCHE PATENTSCHRIFT

(45) Veröffentlichungstag und Bekanntmachung des Hinweises auf die Patenterteilung:
**30.09.2020 Patentblatt 2020/40**

(51) Int Cl.:
***C12N 9/14*** *(2006.01)* ***A23K 10/14*** *(2016.01)*

(21) Anmeldenummer: **19152160.8**

(22) Anmeldetag: **27.08.2014**

# (54) VERFAHREN ZUR HYDROLYTISCHEN SPALTUNG VON ZEARALENON UND/ODER ZEARALENON-DERIVATEN MITTELS EINES POLYPEPTIDS SOWIE VERWENDUNG

METHOD FOR THE HYDROLYSIS OF ZEARALENONE AND/OR ZEARALENONE DERIVATIVES USING A POLYPEPTIDE AND USE THEREOF

PROCÉDÉ DE DÉCOMPOSITION HYDROLYTIQUE DE LA ZÉARALÉNONE ET / OU DES DÉRIVÉES DE LA ZÉARALÉNONE AU MOYEN D'UN POLYPEPTIDE AINSI QU'UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.08.2013 AT 6672013**

(43) Veröffentlichungstag der Anmeldung:
**12.06.2019 Patentblatt 2019/24**

(62) Dokumentnummer(n) der früheren Anmeldung(en) nach Art. 76 EPÜ:
**14781432.1 / 3 039 134**

(73) Patentinhaber: **Erber Aktiengesellschaft**
**3131 Getzersdorf bei Traismauer (AT)**

(72) Erfinder:
- **FRUHAUF, Sebastian**
**3040 Neulengbach (AT)**
- **THAMHESL, Michaela**
**1180 Wien (AT)**
- **PFEFFER, Martin**
**3430 Tulln (AT)**
- **MOLL, Dieter**
**2000 Stockerau (AT)**
- **SCHATZMAYR, Gerd**
**3430 Tulln (AT)**
- **BINDER, Eva Maria**
**3430 Tulln (AT)**

(74) Vertreter: **Cunow, Gerda**
**Cunow Patentanwalts KG**
**Teschnergasse 33/1/3**
**1180 Wien (AT)**

(56) Entgegenhaltungen:
**WO-A1-2012/113827**

- **DATABASE UniProt [Online] 1. Mai 2013 (2013-05-01), "SubName: Full=Alpha/beta hydrolase {ECO:0000313|EMBL:EME22619.1};", XP002734871, gefunden im EBI accession no. UNIPROT:M2XGD3 Database accession no. M2XGD3**
- **DATABASE UniProt [Online] 22. September 2009 (2009-09-22), "SubName: Full=Alpha/beta hydrolase fold protein {ECO:0000313|EMBL:ACT60255.1};", XP002790249, gefunden im EBI accession no. UNIPROT:C6XP75 Database accession no. C6XP75**
- **DATABASE UniProt [Online] 22. September 2009 (2009-09-22), "SubName: Full=Uncharacterized protein {ECO:0000313|EMBL:ACU38472.1}; Flags: Precursor;", XP002734872, gefunden im EBI accession no. UNIPROT:C6WMV9 Database accession no. C6WMV9**
- **DATABASE UniProt [Online] 21. März 2012 (2012-03-21), "SubName: Full=Hydrolase {ECO:0000313|EMBL:EHN79176.1};", XP002734870, gefunden im EBI accession no. UNIPROT:H1Q8U7 Database accession no. H1Q8U7**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



**(Forts. nächste Seite)**

• YUANSHAN YU ET AL: "Oxidation of zearalenone by extracellular enzymes fromSM04 into smaller estrogenic products", WORLD JOURNAL OF MICROBIOLOGY AND BIOTECHNOLOGY, KLUWER ACADEMIC PUBLISHERS, DO, Bd. 27, Nr. 11, 26. April 2011 (2011-04-26), Seiten 2675-2681, XP019959940, ISSN: 1573-0972, DOI: 10.1007/S11274-011-0741-3

SEQUENCE LISTING

<110> Erber Aktiengesellschaft

<120> Polypeptid zur hydrolytischen Spaltung von Zearalenon und / oder Zearalenon Derivaten

<130> P05341PCT

<150> EP 3 039 134
<151> 2016-07-06

<160> 69

<170> PatentIn version 3.5

<210> 1
<211> 328
<212> PRT
<213> Rhodococcus erythropolis

<400> 1

```
Met Ala Glu Glu Gly Thr Arg Ser Glu Ala Ala Asp Ala Ala Thr Gln
1               5                   10                  15

Ala Arg Gln Leu Pro Asp Ser Arg Asn Ile Phe Val Ser His Arg Phe
            20                  25                  30

Pro Glu Arg Gln Val Asp Leu Gly Glu Val Val Met Asn Phe Ala Glu
        35                  40                  45

Ala Gly Ser Pro Asp Asn Pro Ala Leu Leu Leu Leu Pro Glu Gln Thr
    50                  55                  60

Gly Ser Trp Trp Ser Tyr Glu Pro Val Met Gly Leu Leu Ala Glu Asn
65                  70                  75                  80

Phe His Val Phe Ala Val Asp Ile Arg Gly Gln Gly Arg Ser Thr Trp
                85                  90                  95

Thr Pro Arg Arg Tyr Ser Leu Asp Asn Phe Gly Asn Asp Leu Val Arg
            100                 105                 110

Phe Ile Ala Leu Val Ile Lys Arg Pro Val Val Val Ala Gly Asn Ser
        115                 120                 125

Ser Gly Gly Leu Leu Ala Ala Trp Leu Ser Ala Tyr Ala Met Pro Gly
    130                 135                 140

Gln Ile Arg Ala Ala Leu Cys Glu Asp Ala Pro Phe Phe Ala Ser Glu
145                 150                 155                 160
```

```
Leu Val Pro Ala Tyr Gly His Ser Val Leu Gln Ala Ala Gly Pro Ala
                165                 170                 175

Phe Glu Leu Tyr Arg Asp Phe Leu Gly Asp Gln Trp Ser Ile Gly Asp
            180                 185                 190

Trp Lys Gly Phe Val Glu Ala Ala Lys Ala Ser Pro Ala Lys Ala Met
        195                 200                 205

Gln Leu Phe Pro Thr Pro Asp Glu Ala Pro Gln Asn Leu Lys Glu Tyr
    210                 215                 220

Asp Pro Glu Trp Gly Arg Ala Phe Phe Glu Gly Thr Val Ala Leu His
225                 230                 235                 240

Cys Pro His Asp Arg Met Leu Ser Gln Val Lys Thr Pro Ile Leu Ile
                245                 250                 255

Thr His His Ala Arg Thr Ile Asp Pro Glu Thr Gly Glu Leu Leu Gly
            260                 265                 270

Ala Leu Ser Asp Leu Gln Ala Glu His Ala Gln Asp Ile Ile Arg Ser
        275                 280                 285

Ala Gly Val Arg Val Asp Tyr Gln Ser His Pro Asp Ala Leu His Met
    290                 295                 300

Met His Leu Phe Asp Pro Ala Arg Tyr Ala Glu Ile Leu Thr Ser Trp
305                 310                 315                 320

Ser Ala Thr Leu Pro Ala Asn Asp
                325
```

<210> 2
<211> 308
<212> PRT
<213> Streptomyces violaceusniger

<400> 2

```
Met Ala Asp Pro Ala Gln Arg Asp Val Tyr Val Pro His Ala Tyr Pro
1               5                   10                  15

Glu Lys Gln Ala Asp Leu Gly Glu Ile Thr Met Asn Tyr Ala Glu Ala
            20                  25                  30

Gly Glu Pro Asp Met Pro Ala Val Leu Leu Ile Pro Glu Gln Thr Gly
        35                  40                  45
```

```
Ser Trp Trp Gly Tyr Glu Glu Ala Met Gly Leu Leu Ala Glu Asn Phe
    50                  55                  60

His Val Tyr Ala Val Asp Leu Arg Gly Gln Gly Arg Ser Ser Trp Ala
65                  70                  75                  80

Pro Lys Arg Tyr Ser Leu Asp Asn Phe Gly Asn Asp Leu Val Arg Phe
                85                  90                  95

Ile Ala Leu Val Val Lys Arg Pro Val Ile Val Ala Gly Asn Ser Ser
            100                 105                 110

Gly Gly Val Leu Ala Ala Trp Leu Ser Ala Tyr Ser Met Pro Gly Gln
        115                 120                 125

Val Arg Gly Ala Leu Cys Glu Asp Ala Pro Phe Phe Ala Ser Glu Leu
    130                 135                 140

Val Thr Thr Cys Gly His Ser Ile Arg Gln Ala Ala Gly Pro Met Phe
145                 150                 155                 160

Glu Leu Phe Arg Thr Tyr Leu Gly Asp Gln Trp Ser Val Gly Asp Trp
                165                 170                 175

Thr Gly Tyr Cys Arg Ala Ala Asp Ala Ser Ser Ser Pro Met Ala Arg
            180                 185                 190

Tyr Phe Val Ala Asp Glu Ile Pro Gln His Met Arg Glu Tyr Asp Pro
        195                 200                 205

Glu Trp Ala Arg Ala Phe Trp Glu Gly Thr Val Ala Leu His Cys Pro
    210                 215                 220

His Glu Gln Leu Leu Thr Gln Val Lys Thr Pro Val Leu Leu Thr His
225                 230                 235                 240

His Met Arg Asp Ile Asp Pro Asp Thr Gly His Leu Val Gly Ala Leu
                245                 250                 255

Ser Asp Glu Gln Ala Ala Arg Ala Arg Leu Leu Met Glu Ser Ala Gly
            260                 265                 270

Val Lys Val Asp Tyr Ala Ser Val Pro Asp Ala Leu His Met Met His
        275                 280                 285

Gln Phe Asp Pro Pro Arg Tyr Val Glu Ile Phe Thr Gln Trp Ala Ala
    290                 295                 300
```

Thr Leu Ala Ala

305

<210> 3
<211> 309
<212> PRT
<213> Streptomyces coelicolor

<400> 3

```
Met Val Thr Ser Pro Ala Leu Arg Asp Val His Val Pro His Ala Tyr
1               5                   10                  15

Pro Glu Gln Gln Val Asp Leu Gly Glu Ile Thr Met Asn Tyr Ala Glu
            20                  25                  30

Ala Gly Asp Pro Gly Arg Pro Ala Val Leu Leu Ile Pro Glu Gln Thr
        35                  40                  45

Gly Ser Trp Trp Ser Tyr Glu Glu Ala Met Gly Leu Leu Ala Glu His
    50                  55                  60

Phe His Val Tyr Ala Val Asp Leu Arg Gly Gln Gly Arg Ser Ser Trp
65                  70                  75                  80

Thr Pro Lys Arg Tyr Ser Leu Asp Asn Phe Gly Asn Asp Leu Val Arg
                85                  90                  95

Phe Ile Ala Leu Val Val Arg Arg Pro Val Val Val Ala Gly Asn Ser
            100                 105                 110

Ser Gly Gly Val Leu Ala Ala Trp Leu Ser Ala Tyr Ser Met Pro Gly
        115                 120                 125

Gln Ile Arg Gly Val Leu Cys Glu Asp Pro Pro Phe Phe Ala Ser Glu
    130                 135                 140

Leu Val Pro Ala His Gly His Ser Val Arg Gln Gly Ala Gly Pro Val
145                 150                 155                 160

Phe Glu Leu Phe Arg Thr Tyr Leu Gly Asp Gln Trp Ser Val Gly Asp
                165                 170                 175

Trp Glu Gly Phe Arg Ser Ala Ala Asp Ala Ser Ala Ser Pro Met Ala
            180                 185                 190

Arg Ser Phe Val Ala Asp Thr Ile Pro Gln His Leu Lys Glu Tyr Asp
        195                 200                 205
```

Pro Glu Trp Ala Arg Ala Phe Tyr Glu Gly Thr Val Gly Leu Asn Cys
    210                 215                 220

Pro His Glu Arg Met Leu Asn Arg Val Asn Thr Pro Val Leu Leu Thr
225                 230                 235                 240

His His Met Arg Gly Thr Asp Pro Glu Thr Gly Asn Leu Leu Gly Ala
                245                 250                 255

Leu Ser Asp Glu Gln Ala Ala Gln Val Arg Arg Leu Met Glu Ser Ala
            260                 265                 270

Gly Val Lys Val Asp Tyr Glu Ser Val Pro Asp Ala Ser His Met Met
        275                 280                 285

His Gln Ser Asp Pro Ala Arg Tyr Ala Glu Ile Leu Thr Pro Trp Thr
    290                 295                 300

Ala Ala Leu Ala Pro
305

<210> 4
<211> 309
<212> PRT
<213> Streptomyces rapamycinicus

<400> 4

Met Val Thr Ser Pro Ala Leu Arg Asp Val His Val Pro His Ala Tyr
1               5                   10                  15

Pro Glu Gln Gln Val Asp Leu Gly Glu Ile Thr Met Asn Tyr Ala Glu
            20                  25                  30

Ala Gly Asp Pro Asp Arg Pro Ala Val Leu Leu Ile Pro Glu Gln Thr
        35                  40                  45

Gly Ser Trp Trp Ser Tyr Glu Glu Ala Met Gly Leu Leu Ala Glu His
    50                  55                  60

Phe His Val Tyr Ala Val Asp Leu Arg Gly Gln Gly Arg Ser Ser Trp
65                  70                  75                  80

Thr Pro Lys Arg Tyr Ser Leu Asp Asn Phe Gly Asn Asp Leu Val Arg
                85                  90                  95

Phe Ile Ala Leu Val Val Lys Arg Pro Val Val Val Ala Gly Asn Ser
            100                 105                 110

```
Ser Gly Gly Val Leu Ala Ala Trp Leu Ser Ala Tyr Ser Met Pro Gly
        115                 120                 125

Gln Leu Arg Gly Val Leu Cys Glu Asp Pro Pro Phe Phe Ala Ser Glu
    130                 135                 140

Leu Val Pro Ala His Gly His Ser Val Arg Gln Gly Ala Gly Pro Val
145                 150                 155                 160

Phe Glu Leu Phe Arg Thr Tyr Leu Gly Asp Gln Trp Ser Val Ser Asp
                165                 170                 175

Trp Glu Gly Phe Cys Arg Ala Ala Gly Ala Ser Ala Ser Pro Met Ala
            180                 185                 190

Arg Ser Phe Val Ala Asp Gly Ile Pro Gln His Leu Lys Glu Tyr Asp
        195                 200                 205

Pro Glu Trp Ala Arg Ala Phe His Glu Gly Thr Val Gly Leu Asn Cys
    210                 215                 220

Pro His Glu Arg Met Leu Gly Arg Val Asn Thr Pro Val Leu Leu Thr
225                 230                 235                 240

His His Met Arg Gly Thr Asp Pro Glu Thr Gly Asn Leu Leu Gly Ala
                245                 250                 255

Leu Ser Asp Glu Gln Ala Ala Gln Ala Arg Leu Leu Met Glu Ser Ala
            260                 265                 270

Gly Val Arg Val Asp Tyr Glu Ser Val Pro Asp Ala Ser His Met Met
        275                 280                 285

His Gln Ser Asp Pro Ala Arg Tyr Ala Glu Ile Phe Thr Arg Trp Ala
    290                 295                 300

Ala Ala Leu Ala Pro
305
```

<210> 5
<211> 309
<212> PRT
<213> Streptomyces lividans

<400> 5

```
Met Val Thr Ser Pro Ala Leu Arg Asp Val His Val Pro His Ala Tyr
1               5                   10                  15
```

```
Pro Glu Gln Gln Val Asp Leu Gly Glu Ile Thr Met Asn Tyr Ala Glu
            20                  25                  30

Ala Gly Asp Pro Gly Arg Pro Ala Val Leu Leu Ile Pro Glu Gln Thr
        35                  40                  45

Gly Ser Trp Trp Ser Tyr Glu Glu Ala Met Gly Leu Leu Ala Glu His
    50                  55                  60

Phe His Val Tyr Ala Val Asp Leu Arg Gly Gln Gly Arg Ser Ser Trp
65                  70                  75                  80

Thr Pro Lys Arg Tyr Ser Leu Asp Asn Phe Gly Asn Asp Leu Val Arg
                85                  90                  95

Phe Met Ala Leu Val Val Arg Arg Pro Val Val Val Ala Gly Asn Ser
            100                 105                 110

Ser Gly Gly Val Leu Ala Ala Trp Leu Ser Ala Tyr Ser Met Pro Gly
        115                 120                 125

Gln Ile Arg Gly Val Leu Cys Glu Asp Pro Pro Phe Phe Ala Ser Glu
    130                 135                 140

Leu Val Pro Ala His Gly His Ser Val Arg Gln Gly Ala Gly Pro Val
145                 150                 155                 160

Phe Glu Leu Phe Arg Thr Tyr Leu Gly Asp Gln Trp Ser Val Gly Asp
                165                 170                 175

Trp Glu Gly Phe Arg Ser Ala Ala Gly Ala Ser Ala Ser Pro Met Ala
            180                 185                 190

Arg Ser Phe Val Ala Asp Thr Ile Pro Gln His Leu Lys Glu Tyr Asp
        195                 200                 205

Pro Glu Trp Ala Arg Ala Phe Tyr Glu Gly Thr Val Gly Leu Asn Cys
    210                 215                 220

Pro His Glu Arg Met Leu Asn Arg Val Asn Thr Pro Val Leu Leu Thr
225                 230                 235                 240

His His Met Arg Gly Thr Asp Pro Glu Thr Gly Asn Leu Leu Gly Ala
                245                 250                 255

Leu Ser Asp Glu Gln Ala Ala Gln Ala Arg Arg Leu Met Glu Ser Ala
            260                 265                 270
```

```
Gly Val Lys Val Asp Tyr Glu Ser Val Pro Asp Ala Ser His Met Met
        275                 280                 285

His Gln Ser Asp Pro Ala Arg Tyr Ala Glu Ile Leu Thr Pro Trp Ala
    290                 295                 300

Ala Ala Leu Ala Pro
305
```

<210> 6
<211> 309
<212> PRT
<213> Streptomyces coelicoflavus

<400> 6

```
Met Val Thr Ser Pro Ala Leu Arg Asp Val His Val Pro His Ala Tyr
1               5                   10                  15

Pro Glu Gln Gln Val Asp Leu Gly Glu Ile Thr Met Asn Tyr Ala Glu
            20                  25                  30

Ala Gly Asp Pro Asp Arg Pro Ala Val Leu Leu Ile Pro Glu Gln Thr
        35                  40                  45

Gly Ser Trp Trp Ser Tyr Glu Glu Ala Met Gly Leu Leu Ser Glu His
    50                  55                  60

Phe His Val Tyr Ala Val Asp Leu Arg Gly Gln Gly Arg Ser Ser Trp
65                  70                  75                  80

Thr Pro Lys Arg Tyr Ser Leu Asp Asn Phe Gly Asn Asp Leu Val Arg
                85                  90                  95

Phe Ile Ala Leu Val Val Lys Arg Pro Val Val Val Ala Gly Asn Ser
            100                 105                 110

Ser Gly Gly Val Leu Ala Ala Trp Leu Ser Ala Tyr Ser Met Pro Gly
        115                 120                 125

Gln Leu Arg Gly Val Leu Cys Glu Asp Pro Pro Phe Phe Ala Ser Glu
    130                 135                 140

Leu Val Pro Ala His Gly His Ser Val Arg Gln Gly Ala Gly Pro Val
145                 150                 155                 160

Phe Glu Leu Phe Arg Thr Tyr Leu Gly Asp Gln Trp Ser Val Gly Asp
                165                 170                 175
```

Trp Glu Gly Phe Cys Arg Ala Ala Gly Ala Ser Ala Ser Pro Met Ala
180 185 190

Arg Ser Phe Val Ala Asp Gly Ile Pro Gln His Leu Gln Glu Tyr Asp
195 200 205

Pro Glu Trp Ala Arg Val Phe Tyr Glu Gly Thr Val Gly Leu Ser Cys
210 215 220

Pro His Glu Arg Met Leu Gly Gln Val Lys Thr Pro Val Leu Leu Thr
225 230 235 240

His His Met Arg Gly Ile Asp Pro Glu Thr Gly Asn Leu Leu Gly Ala
245 250 255

Leu Ser Asp Glu Gln Ala Leu Arg Ala Arg Arg Leu Met Asp Ser Ala
260 265 270

Gly Val Thr Val Asp Tyr Glu Ser Val Pro Asp Ala Ser His Met Met
275 280 285

His Gln Ser Ala Pro Ala Arg Tyr Val Glu Ile Phe Thr Arg Trp Ala
290 295 300

Ala Ala Leu Ala Pro
305

<210> 7
<211> 300
<212> PRT
<213> Rhodococcus triatome

<400> 7

Met Pro His Asp Tyr Glu Glu Lys Leu Val Asp Leu Gly Glu Ile Asp
1 5 10 15

Leu Asn Tyr Ala Glu Ala Gly Ser Pro Asp Lys Pro Ala Leu Leu Leu
20 25 30

Ile Pro Ser Gln Ser Glu Ser Trp Trp Gly Tyr Glu Glu Ala Met Gly
35 40 45

Leu Leu Ala Glu Asp Tyr His Val Phe Ala Val Asp Met Arg Gly Gln
50 55 60

Gly Arg Ser Thr Trp Thr Pro Gly Arg Tyr Ser Leu Asp Asn Phe Gly
65 70 75 80

```
Asn Asp Leu Val Arg Phe Ile Asp Leu Val Ile Gly Arg Thr Val Ile
                85                  90                  95

Val Ser Gly Asn Ser Ser Gly Gly Val Val Ala Ala Trp Leu Ala Ala
            100                 105                 110

Phe Ser Leu Pro Gly Gln Val Arg Ala Ala Leu Ala Glu Asp Ala Pro
        115                 120                 125

Phe Phe Ala Ser Glu Leu Asp Pro Lys Val Gly His Thr Ile Arg Gln
    130                 135                 140

Ala Ala Gly His Ile Phe Val Asn Trp Arg Asp Tyr Leu Gly Asp Gln
145                 150                 155                 160

Trp Ser Val Gly Asp Tyr Ala Gly Phe Leu Lys Ala Met Lys Ser Ser
                165                 170                 175

Glu Val Pro Met Leu Arg Gln Val Pro Leu Pro Glu Thr Ala Pro Gln
            180                 185                 190

Asn Leu Leu Glu Tyr Asp Pro Glu Trp Ala Arg Ala Phe Tyr Glu Gly
        195                 200                 205

Thr Val Ala Gln Thr Cys Pro His Asp Tyr Met Leu Ser Gln Val Lys
    210                 215                 220

Val Pro Met Leu Val Thr His His Ala Arg Met Ile Asp Glu Ala Thr
225                 230                 235                 240

Ser Gly Leu Val Gly Ala Met Ser Asp Leu Gln Val Gln Lys Ala Ala
                245                 250                 255

Glu Ile Ile Arg Gly Thr Gly Val Gln Val Asp Val Val Asp Leu Pro
            260                 265                 270

Glu Ala Pro His Ile Leu His Gln Leu Ala Pro Lys Glu Tyr Val Glu
        275                 280                 285

Ile Leu Asn Asn Trp Val Glu Lys Leu Pro Pro Val
    290                 295                 300
```

<210> 8
<211> 307
<212> PRT
<213> Hirschia baltica

<400> 8

```
Met Ile Gln Asn Asn Lys Thr Ala Pro Tyr Lys Tyr Lys Glu Lys Leu
1               5                   10                  15

Val Asp Leu Gly Glu Ile Lys Met Asn Tyr Ile Val Ala Gly Ala Asp
            20                  25                  30

Val Ser Pro Ala Leu Leu Leu Ile Pro Gly Gln Thr Glu Ser Trp Trp
        35                  40                  45

Gly Phe Glu Ala Ala Ile Glu Lys Leu Glu Ser Asn Phe Gln Val Phe
    50                  55                  60

Ala Ile Asp Leu Arg Gly Gln Gly Lys Ser Thr Gln Thr Pro Gly Arg
65                  70                  75                  80

Tyr Ser Leu Asn Leu Met Gly Asn Asp Leu Val Arg Phe Ile Ser Leu
                85                  90                  95

Val Ile Lys Arg Pro Val Ile Val Ser Gly Asn Ser Ser Gly Gly Leu
            100                 105                 110

Leu Ala Ala Trp Leu Ser Ala Tyr Ala Met Pro Asn Gln Ile Arg Ala
        115                 120                 125

Ile His Cys Glu Asp Ala Pro Phe Phe Thr Ala Glu Lys Ala Pro Leu
    130                 135                 140

Tyr Gly His Ala Ile Gln Gln Ala Ala Gly Pro Ile Phe Ser Leu Met
145                 150                 155                 160

Ser Lys Phe Leu Gly Asp Gln Trp Ser Ile Asn Asn Trp Glu Gly Leu
                165                 170                 175

Lys Ala Ala Gln Ala Lys Asp Thr His Pro Ala Asn Lys Met Ile Ser
            180                 185                 190

Gln Val Glu Gln Pro Pro Gln His Leu Lys Glu Tyr Asp Pro Glu Trp
        195                 200                 205

Gly Arg Ala Phe Ile Glu Gly Lys Phe Asn Leu Asn Ser Pro His His
    210                 215                 220

Thr Leu Leu Ser Asp Ile Lys Thr Pro Met Leu Tyr Thr His His Met
225                 230                 235                 240

Arg Phe Glu Asp Pro Gln Thr Gly Leu Leu Ile Gly Ala Thr Ser Asp
```

```
                245                 250                 255

Phe Gln Ala Ser Lys Ile Lys Glu Ile Ala Leu Lys Thr Gly Asn Ser
            260                 265                 270

Phe Glu Leu Ile Asp Ala Pro Asp Ala Phe His Ser Met His Glu Ala
        275                 280                 285

Asp Pro Gln Arg Phe Val Asp Ile Leu Thr Ser Trp Ile Glu Arg Leu
    290                 295                 300

Asn Leu Gln
305
```

<210> 9
<211> 321
<212> PRT
<213> Nocardia brasiliensis

<400> 9

```
Met Gly Ile Ser Glu Ala Ala Asp Arg Ala Asp Thr Phe Val Ala His
1               5                   10                  15

Lys Phe Glu Glu Gln Leu Val Asp Leu Gly Glu Ile Arg Met Asn Tyr
            20                  25                  30

Val Ala Ala Gly Asp Pro Thr Ser Pro Ala Leu Leu Leu Ile Pro Ala
        35                  40                  45

Gln Gly Glu Ser Trp Trp Gly Tyr Glu Asn Ala Ile Thr Leu Leu Ala
    50                  55                  60

Asn Asp Phe Arg Val Phe Ala Ile Asp Leu Arg Gly Gln Gly Arg Ser
65                  70                  75                  80

Thr Trp Thr Pro Gly Arg Tyr Asn Leu Asn Thr Trp Gly Asn Asp Val
                85                  90                  95

Glu Arg Phe Ile Asp Leu Val Ile Gly Arg Pro Thr Leu Val Ala Gly
            100                 105                 110

Asn Ser Ser Gly Gly Val Ile Ala Ala Trp Leu Ala Ala Tyr Ala Lys
        115                 120                 125

Pro Gly Gln Ile Arg Gly Ala Met Leu Glu Asp Pro Pro Leu Phe Ala
    130                 135                 140

Ser Gln Ala Ala Pro Pro Tyr Gly Pro Gly Ile Met Gln Thr Leu Gly
```

145 150 155 160

Pro Ile Phe Val Leu Trp Ala Lys Trp Leu Gly Pro Gln Trp Ser Val
165 170 175

Gly Asp Trp Asp Gly Met Val Ala Ala Ala Pro Arg Glu Leu Pro Glu
180 185 190

Phe Leu His Pro Gly Ile Ala Phe Leu Phe Gly Asp Gly Thr Gly Glu
195 200 205

Gly Ala Ala Ala Thr Pro Pro Gln His Leu Lys Glu Tyr Asp Pro Glu
210 215 220

Trp Ala Gln Ala Trp Ala Thr Asp Val Ala Asn Ala Gly Cys Asp His
225 230 235 240

Ala Thr Met Leu Ala Gln Asn Arg Val Pro Val Leu Leu Thr His His
245 250 255

Phe His Leu Thr Asp Pro Asp Thr Gly Gln Leu Met Gly Ala Met Thr
260 265 270

Asp Ile Gln Ala Gln Gln Ala Arg Arg Leu Leu Ala Ala Thr Gly Gln
275 280 285

Pro Val Thr Phe Thr Ala Leu Asp Ala Pro His Thr Met His Asp Pro
290 295 300

Glu Pro Glu Arg Tyr Phe Glu Val Leu Thr Glu Trp Ala Ser Ala Leu
305 310 315 320

Asp

<210> 10
<211> 319
<212> PRT
<213> Mycobacterium vaccae

<400> 10

```
Met Gly Arg Tyr Ala Gly Val Phe Gly Pro His Ala Pro Glu Ser Thr
1               5                   10                  15

Tyr Val Gly His Ala Tyr Pro Glu Gln Leu Phe Asp Thr Gly Glu Val
            20                  25                  30

Arg Leu Asn Tyr Ala Val Ala Gly Asp Ala Ser Ala Ser Pro Leu Leu
```

```
            35                  40                  45

Leu Ile Pro Gly Gln Thr Glu Ser Trp Trp Gly Tyr Glu Pro Ala Met
    50                  55                  60

Gly Leu Leu Ala Glu His Phe His Val His Ala Val Asp Leu Arg Gly
65                  70                  75                  80

Gln Gly Arg Ser Thr Arg Thr Pro Arg Arg Tyr Thr Leu Asp Asn Ile
                85                  90                  95

Gly Asn Asp Leu Val Arg Phe Leu Asp Gly Val Ile Gly Arg Pro Ala
            100                 105                 110

Phe Val Ser Gly Leu Ser Ser Gly Gly Leu Leu Ser Ala Trp Leu Ser
        115                 120                 125

Ala Phe Ala Glu Pro Gly Gln Val Leu Ala Ala Cys Tyr Glu Asp Pro
    130                 135                 140

Pro Phe Phe Ser Ser Glu Leu Asp Pro Val Ile Gly Pro Gly Leu Met
145                 150                 155                 160

Ser Thr Val Gly Pro Leu Phe Ala Leu Tyr Val Lys Tyr Leu Gly Asp
                165                 170                 175

Gln Trp Ser Ile Gly Asp Trp Asp Gly Phe Val Ala Gly Ala Pro Gln
            180                 185                 190

Glu Leu Ala Gly Trp Gln Ala His Val Ala Leu Ala Gly Gly Thr Ala
        195                 200                 205

Glu Pro Pro Gln His Leu Lys Glu Tyr Asp Pro Glu Trp Gly Arg Ala
    210                 215                 220

Phe Val Gly Gly Thr Phe Thr Thr Gly Cys Pro His Gln Val Met Leu
225                 230                 235                 240

Ser Gln Val Lys Val Pro Val Leu Phe Thr His His Phe Arg Met Leu
                245                 250                 255

Asp Asp Glu Ser Gly Ser Leu Ile Gly Ala Ala Thr Asp Asp Gln Ala
            260                 265                 270

Ala Arg Val Val Glu Leu Val Glu Asn Ser Gly Ala Pro Leu Thr Tyr
        275                 280                 285
```

Arg Ser Phe Pro Met Met Gly His Ser Met His Ala Gln Asp Pro Ala
    290                 295                 300

Leu Phe Ala Gly Thr Leu Val Asp Trp Phe Thr Ala Ala Arg Ser
305                 310                 315

<210> 11
<211> 319
<212> PRT
<213> Mycobacterium gilvum

<400> 11

Met Gly Arg Tyr Ala Gly Val Phe Gly Pro His Ala Pro Glu Ala Thr
1               5                   10                  15

Tyr Val Glu His Gly Tyr Pro Glu Arg Leu Phe Asp Thr Gly Glu Val
            20                  25                  30

Gln Leu Asn Tyr Val Val Ala Gly Asp Ala Ala Ala Pro Pro Leu Leu
        35                  40                  45

Leu Ile Pro Gly Gln Ser Glu Ser Trp Trp Gly Tyr Glu Ala Ala Ile
    50                  55                  60

Pro Leu Leu Ala Arg His Phe His Val His Ala Val Asp Leu Arg Gly
65                  70                  75                  80

Gln Gly Arg Ser Thr Arg Thr Pro Gly Arg Tyr Thr Leu Asp Asn Val
                85                  90                  95

Gly Asn Asp Leu Val Arg Phe Leu Asp Gly Val Ile Gly Arg Pro Ala
            100                 105                 110

Phe Val Ser Gly Leu Ser Ser Gly Gly Leu Ala Ser Ala Trp Leu Ser
        115                 120                 125

Ala Phe Ala Lys Pro Gly Gln Val Val Ala Ala Cys Trp Glu Asp Pro
    130                 135                 140

Pro Phe Phe Ser Ser Glu Thr Ala Pro Ile Val Gly Pro Pro Ile Thr
145                 150                 155                 160

Asp Ser Ile Gly Pro Leu Phe Gly Met Trp Ala Arg Tyr Leu Gly Asp
                165                 170                 175

Gln Trp Ser Val Gly Asp Trp Asp Gly Phe Val Ala Ala Val Pro Thr
            180                 185                 190

```
Glu Leu Ala Asp Trp Gln Ala His Val Ala Leu Val Val Gly Thr Ala
        195                 200                 205

Asp Pro Pro Gln Asn Leu Arg Glu Tyr Asp Pro Glu Trp Gly Lys Ala
    210                 215                 220

Phe Ile Thr Gly Thr Phe Ala Ala Ser Cys Pro His His Val Met Leu
225                 230                 235                 240

Ser Lys Val Lys Val Pro Val Leu Tyr Thr His His Phe Arg Met Ile
                245                 250                 255

Asp Glu Gly Ser Gly Gly Leu Ile Gly Ala Cys Ser Asp Ile Gln Ala
            260                 265                 270

Gly Arg Val Thr Gln Leu Ala Lys Ser Gly Gly Arg Ser Val Thr Tyr
        275                 280                 285

Arg Ser Phe Pro Met Met Ala His Ser Met His Gly Gln Asp Pro Ala
    290                 295                 300

Leu Phe Ser Glu Thr Leu Val Glu Trp Phe Ser Arg Phe Thr Gly
305                 310                 315
```

<210> 12
<211> 322
<212> PRT
<213> Gordonia effusa

<400> 12

```
Met Pro Lys Ser Glu Ala Ala Asp Arg Ala Asp Ser Phe Val Ser His
1               5                   10                  15

Asp Phe Lys Glu Asn Ile Val Asp Leu Gly Glu Ile Arg Met Asn Tyr
            20                  25                  30

Val Val Gln Gly Asn Lys Lys Ser Pro Ala Leu Leu Leu Ile Pro Ala
        35                  40                  45

Gln Gly Glu Ser Trp Trp Gly Tyr Glu Ala Ala Ile Pro Leu Leu Ala
    50                  55                  60

Lys His Phe Gln Val Phe Ala Ile Asp Leu Arg Gly Gln Gly Arg Thr
65                  70                  75                  80

Thr Trp Thr Pro Gly Arg Tyr Thr Leu Asp Ile Phe Gly Asn Asp Val
                85                  90                  95
```

```
Val Arg Phe Ile Asp Leu Val Ile Gly Arg Glu Thr Leu Ile Ala Gly
            100                 105                 110

Asn Ser Ser Gly Gly Leu Ile Gly Ala Trp Leu Ala Ala Phe Ala Lys
        115                 120                 125

Pro Gly Gln Val Arg Ala Val Met Leu Glu Asp Pro Pro Leu Phe Ala
    130                 135                 140

Ser Glu Ile Arg Pro Pro Tyr Gly Pro Gly Ile Trp Gln Gly Leu Gly
145                 150                 155                 160

Pro Met Phe Ala Ala Trp Ala Lys Trp Leu Gly Pro Gln Trp Ser Ile
                165                 170                 175

Gly Asp Trp Asp Gly Met Val Lys Ala Leu Pro Asp Glu Leu Pro Glu
            180                 185                 190

Asp Leu Leu Pro Gly Ile Gly Phe Met Leu Gly Asp Gly Glu Ser Asp
        195                 200                 205

Gly Ala Ala Pro Thr Pro Pro Gln His Leu Lys Glu Tyr Asp Pro Glu
    210                 215                 220

Trp Gly Ala Ser Trp Ala Ser Gly Phe Ala Asn Thr Gly Cys Glu His
225                 230                 235                 240

Glu Ala Val Ile Ser Gln Val Arg Val Pro Val Leu Leu Thr His His
                245                 250                 255

Phe Arg Gln Ile Asn Glu Glu Thr Gly His Leu Met Gly Ala Leu Ser
            260                 265                 270

Asp Leu Gln Ala Ala Gln Val Arg His Ile Ile Glu Glu Val Ala Gly
        275                 280                 285

Gln Glu Val Thr Tyr Val Ser Leu Asp Ala Pro His Thr Met His Glu
    290                 295                 300

Pro Gln Pro Glu Arg Tyr Thr Asp Val Leu Leu Asp Trp Val Lys Lys
305                 310                 315                 320

Leu Gly
```

<210> 13
<211> 328
<212> PRT

<213> Togninia minima

<400> 13

```
Met Asn Tyr Ala Thr Ala Gly Ser Ser Asp Lys Pro Ala Leu Leu Leu
1               5                   10                  15

Val Pro Gly Gln Ser Glu Ser Trp Trp Gly Tyr Glu Met Ala Met Trp
            20                  25                  30

Leu Leu Lys Asp Asp Tyr Gln Val Phe Ala Val Asp Met Arg Gly Gln
        35                  40                  45

Gly Gln Ser Thr Trp Thr Pro Gly Arg Tyr Ser Leu Asp Thr Phe Gly
    50                  55                  60

Asn Asp Leu Val Lys Phe Ile Asp Ile Val Ile Lys Arg Pro Val Val
65                  70                  75                  80

Val Ser Gly Leu Ser Ser Gly Gly Val Val Ser Ala Trp Leu Ser Ala
                85                  90                  95

Phe Ala Lys Pro Gly Gln Ile Arg Ala Ala Val Tyr Glu Asp Pro Pro
            100                 105                 110

Leu Phe Ala Ser Gln Ser Lys Pro Ala Ile Gly Gln Ser Val Met Gln
        115                 120                 125

Thr Val Ala Gly Pro Phe Phe Asn Leu Trp Tyr Lys Trp Leu Gly Ala
    130                 135                 140

Gln Trp Thr Ile Gly Asp Gln Ala Gly Met Val Ala Ala Met Pro Lys
145                 150                 155                 160

Glu Ile Pro Ala Trp Ile Leu Gln Tyr Leu Gly Asn Thr Thr Ser Gly
                165                 170                 175

Pro Thr Gly Leu Asp Leu Thr Leu Asn Glu Tyr Asp Pro Glu Trp Gly
            180                 185                 190

His Gly Phe Val Ser Gly Thr Val Asp Ala Thr Cys Asp His Glu Ala
        195                 200                 205

Met Leu Thr His Val Lys Val Pro Val Leu Phe Thr His His Ser Arg
    210                 215                 220

Ala Ile Asp Pro Tyr Thr Gly Asn Leu Ile Gly Ser Val Ser Asp Thr
225                 230                 235                 240
```

Gln Val Ser Tyr Ala Gln Gly Leu Ile Thr Thr Asn Gly Asn Gln Ser
245 250 255

Phe Thr Leu Lys Asn Phe Pro Leu Ala Ser His Asp Met His Asn Ser
260 265 270

Asp Pro Ala Thr Tyr Val Ser Ala Ile Thr Thr Trp Met Ala Ser Leu
275 280 285

Gly Ile Gly Ser Ala Val Ile Pro Gly Pro Val Lys Val Ala Ser Ala
290 295 300

Ser Ala Gln Val Ser Ala Ala Ser Thr Ala Pro Pro Ser Cys Thr Ser
305 310 315 320

Thr Ser Ala Pro Ser Thr Gly His
325

<210> 14
<211> 280
<212> PRT
<213> Actinosynnema mirum

<400> 14

Met Thr Val Val Asp Pro Pro Ala Pro Arg Asp Phe Pro Glu Leu Leu
1 5 10 15

Val Asp Leu Gly Glu Val Val Leu Asn His Ala Glu Ala Gly Ser Pro
20 25 30

Asp Arg Pro Ala Leu Val Pro Val Pro Glu Gln Gly Gly Ser Trp Trp
35 40 45

Ser Tyr Glu Arg Val Met Pro Leu Pro Ala Arg Asp Phe His Val Phe
50 55 60

Ala Val Asp Leu Arg Gly Arg Gly Arg Ser Thr Arg Thr Pro Arg Arg
65 70 75 80

Tyr Ser Leu Asp Asp Phe Gly Asn Asp Leu Val Arg Phe Leu Ala Leu
85 90 95

Val Val Arg Arg Pro Ala Val Val Ala Gly Asn Ser Ser Gly Gly Val
100 105 110

Leu Ala Ala Trp Ser Ser Ala Tyr Ala Met Pro Gly Gln Val Arg Ala
115 120 125

Val Leu Leu Glu Asp Pro Pro Leu Phe Ser Ser Glu Leu Thr Pro Val
    130                 135                 140

Cys Gly Pro Gly Val Arg Gln Ala Ala Gly Pro Leu Phe Glu Leu Leu
145                 150                 155                 160

Ser Thr His Leu Gly Asp Gln Trp Gly Gly Gly Arg Pro Gly Arg Val
                165                 170                 175

His Gly Gly Val Pro Arg Leu Gly Leu Ala Ala Ala Ala Ala Val Arg
            180                 185                 190

Val Ala Arg Arg Ala Ala Ala Thr Asp Ala Arg Gly Arg Pro Gly Ala
        195                 200                 205

Ala Arg Gly Arg Pro Ala Gly Val Gly Gly Ala Ala Arg Arg Gly Arg
    210                 215                 220

Gly Gly Arg Glu Arg Thr Gly Thr Thr Thr Val Leu Ser Gly Leu Thr
225                 230                 235                 240

Gly Ser Arg Thr Ser Gly Thr Gly Arg Cys Arg Lys Pro Phe Arg Leu
                245                 250                 255

Arg Gln Trp Trp Ala Gly Gly Ala Arg Gly Pro Pro Pro Pro Arg Gln
            260                 265                 270

Ile Arg Ala Asp Val Arg Thr Arg
        275                 280

<210> 15
<211> 326
<212> PRT
<213> Kutzneria albida

<400> 15

Met Ser Val Pro Val Thr Pro Ser Ala Arg Asn Val Phe Val Pro His
1               5                   10                  15

Ala Phe Pro Glu Lys Gln Ile Asp Leu Gly Glu Val Val Leu Asn Tyr
            20                  25                  30

Ala Glu Ala Gly Thr Pro Asp Lys Pro Ala Leu Leu Leu Leu Pro Glu
        35                  40                  45

Gln Thr Gly Ser Trp Trp Ser Tyr Glu Pro Ala Met Gly Leu Leu Ala
    50                  55                  60

```
Glu His Phe His Val Phe Ala Val Asp Leu Arg Gly Gln Gly Arg Ser
65                  70                  75                  80

Thr Trp Thr Pro Gly Arg Tyr Ser Leu Asp Asn Phe Gly Asn Asp Leu
                85                  90                  95

Val Arg Phe Ile Ala Leu Ala Ile Arg Arg Pro Val Val Val Ala Gly
            100                 105                 110

Cys Ser Ser Gly Gly Val Leu Ala Ala Trp Leu Ser Ala Tyr Ala Leu
        115                 120                 125

Pro Gly Gln Ile Arg Gly Ala Leu Cys Glu Asp Ala Pro Leu Phe Ala
    130                 135                 140

Ser Glu Leu Thr Pro Ala His Gly His Gly Val Arg Gln Gly Ala Gly
145                 150                 155                 160

Pro Val Phe Glu Leu Tyr Arg Asp Tyr Leu Gly Asp Gln Trp Ser Val
                165                 170                 175

Gly Asp Trp Ala Gly Leu Val Ala Ala Ala Gln Ala Ser Pro Ala Lys
            180                 185                 190

Met Met Ser Leu Phe Lys Met Pro Gly Glu Pro Pro Gln Asn Leu Arg
        195                 200                 205

Glu Tyr Asp Pro Glu Trp Ala Arg Val Phe Phe Glu Gly Thr Val Gly
    210                 215                 220

Leu His Cys Pro His Asp Arg Met Leu Ser Gln Val Lys Thr Pro Val
225                 230                 235                 240

Leu Ile Thr His His Ala Arg Thr Thr Asp Pro Glu Thr Gly Glu Phe
                245                 250                 255

Leu Gly Ala Leu Ser Glu Leu Gln Ala Glu Arg Ala Gln Ala Ile Ile
            260                 265                 270

Arg Ala Ala Gly Val Pro Val Asp Tyr Gln Ser Phe Pro Asp Ala Ala
        275                 280                 285

His Ala Met His Thr Thr Glu Pro Ala Arg Tyr Ala Ala Val Leu Thr
    290                 295                 300

Ala Trp Ala Ala Lys Leu Pro Pro Val Ala Asp Thr Ser Pro Ser Ala
305                 310                 315                 320
```

```
Ala Ala Ser Ala His Val
                    325
```

<210> 16
<211> 987
<212> DNA
<213> Rhodococcus erythropolis

<220>
<221> misc_feature
<223> Artificial DNA sequence encodes polypeptid with SEQ ID NO: 1

<400> 16

atggccgaag aaggaactag gtccgaagca gcggatgctg ccacacaagc gagacagcta 60

cccgattcgc ggaacatctt tgtctcgcac cgatttccgg aaaggcaggt cgatctcggt 120

gaagtggtga tgaacttcgc ggaggcgggc tctccggaca acccggcact gctcctcctc 180

cccgagcaga ccgggtcgtg gtggagttac gagccagtga tgggtcttct ggcagagaac 240

tttcatgtct ttgccgtcga tatccgtggg caaggtcgca gtacctggac gccacggcga 300

tacagcctgg acaacttcgg caatgatctg gtgcgtttca tcgctctggt catcaagcgc 360

cctgtcgtcg tggcagggaa ctcctcgggg gggctgctgg ccgcctggct ctcggcgtac 420

gcgatgcccg gccagatccg tgcagcattg tgtgaggacg caccgttctt tgcgtcggag 480

ttggtccccg catacggtca ctcggttctg caggcggcgg gtccggcatt cgagttgtac 540

cgggacttcc tcggggacca gtggtcgatt ggggactgga aagggttcgt tgaggcagcc 600

aaagcgtcgc cggcaaaggc tatgcaatta tttccgaccc cggatgaggc gccgcagaat 660

ctcaaggaat acgacccgga atgggggcgc gcattcttcg aagggactgt ggcactgcac 720

tgcccacacg acaggatgct ctcgcaagtc aagacaccaa ttctcatcac tcaccacgcg 780

cggacgatcg accccgagac gggcgagctg ttgggcgcgc tctccgacct tcaggcagag 840

catgcgcagg acatcattcg gtctgcgggc gttcgggtgg actatcagtc gcaccccgac 900

gcgcttcaca tgatgcatct gttcgatccc gctcgttacg cggagatctt gacatcctgg 960

tccgcaacac tgcctgcgaa cgactag 987

<210> 17
<211> 987
<212> DNA
<213> Artificial sequence

<220>
<223> ORF was codon optimized and thus differce from natural occuring DNA sequence.

<400> 17
atggcagaag aaggcacccg tagcgaagca gcagatgcag caacccaggc acgtcagctg 60

ccggatagcc gtaacatttt tgttagccat cgttttccgg aacgtcaggt tgatctgggt 120
gaagttgtta tgaattttgc agaagcaggt agtccggata atccggcatt actgctgctg 180
ccggaacaga ccggtagttg gtggtcttat gaaccggtta tgggtctgct ggcagaaaac 240
tttcatgttt ttgcagttga tattcgtggt cagggtcgta gcacctggac accgcgtcgt 300
tatagcctgg ataattttgg taatgatctg gtgcgtttta ttgccctggt tattaaacgt 360
ccggttgttg ttgcaggtaa tagcagcggt ggcctgctgg ctgcatggct gagcgcctat 420
gcaatgcctg gtcagattcg tgcagcactg tgtgaagatg caccgttttt tgcaagcgaa 480
ctggttcctg cctatggtca tagcgttctg caggcagcag gtccggcatt tgaactgtat 540
cgtgattttc tgggtgatca gtggtcaatt ggtgattgga aaggttttgt tgaagcagca 600
aaagcaagtc cggctaaagc aatgcagctg tttccgacac cggatgaagc accgcagaat 660
ctgaaagaat atgatccgga atggggtcgt gcattttttg aaggcaccgt tgcactgcat 720
tgtccgcatg atcgtatgct gagccaggtt aaaaccccga ttctgattac ccatcatgca 780
cgtaccatcg atccggaaac cggtgaactg ctgggtgcac tgagtgatct gcaggccgaa 840
catgcacagg atattattcg tagtgccggt gttcgtgttg attatcagag ccatcctgat 900
gcactgcaca tgatgcacct gtttgatccg gcacgttatg cagaaattct gaccagttgg 960
agcgcaaccc tgcctgcaaa tgattaa 987

<210> 18
<211> 927
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 2

<400> 18

atggcagatc cggcacagcg tgatgtttat gttccgcatg catatccgga aaaacaggca 60
gatctgggtg aaattaccat gaattatgcc gaagccggtg aacctgatat gcctgcagtt 120
ctgctgattc cggaacagac cggtagttgg tggggttatg aagaagcaat gggtctgctg 180
gcagaaaact ttcatgttta tgcagttgat ctgcgtggtc agggtcgtag cagctgggca 240
ccgaaacgtt atagcctgga taattttggt aatgatctgg tgcgttttat tgccctggtt 300
gttaaacgtc cggttattgt tgcaggtaat agcagcggtg gtgttctggc agcatggctg 360
agcgcatata gcatgcctgg tcaggttcgt ggtgcactgt gtgaagatgc accgtttttt 420

```
gcaagcgaac tggttaccac ctgtggtcat agcattcgtc aggcagcagg tccgatgttt       480
gaactgtttc gtacctatct gggcgatcag tggtcagttg gtgattggac cggctattgt       540
cgtgcagcag atgcaagcag cagcccgatg gcacgttatt ttgttgcaga tgaaattccg       600
cagcacatgc gtgaatatga tccggaatgg gcacgtgcat tttgggaagg caccgttgca       660
ctgcattgtc cgcatgaaca gctgctgacc caggttaaaa caccggtgct gctgacacat       720
cacatgcgcg atattgatcc tgataccggt catctggttg gtgccctgag tgatgaacag       780
gcagcccgtg cacgtctgct gatggaaagt gccggtgtta aagttgatta tgcaagcgtt       840
ccggatgcac tgcacatgat gcaccagttt gatccgcctc gttatgttga aatctttacc       900
cagtgggcag caaccctggc agcataa                                           927
```

<210> 19
<211> 930
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 3

<400> 19

atggttacca gtccggcact gcgtgatgtt catgttccgc atgcatatcc ggaacagcag 60
gttgatctgg gtgaaattac catgaattat gccgaagccg gtgatccggg tcgtccggca 120
gttctgctga tcccggaaca gaccggtagt tggtggtctt atgaagaagc aatgggtctg 180
ctggcagaac attttcatgt ttatgcagtt gatctgcgtg gtcagggtcg tagcagctgg 240
accccgaaac gttatagcct ggataatttt ggtaatgatc tggtgcgttt tattgccctg 300
gttgttcgtc gtccggttgt tgttgcaggt aatagcagcg gtggtgttct ggcagcatgg 360
ctgagcgcat atagcatgcc tggtcagatt cgtggtgtgc tgtgtgaaga tccgcctttt 420
tttgcaagcg aactggttcc ggcacatggt catagcgttc gtcagggtgc aggtccggtt 480
tttgaactgt ttcgtaccta tctgggcgat cagtggtcag ttggtgattg ggaaggtttt 540
cgtagcgcag cagatgcaag cgcaagcccg atggcacgta gctttgttgc agataccatt 600
ccgcagcatc tgaaagaata tgatccggaa tgggcacgtg cattttatga aggcaccgtt 660
ggtctgaatt gtccgcatga acgtatgctg aatcgtgtta atacaccggt gctgctgacc 720
catcacatgc gtggcaccga tccggaaacc ggtaatctgc tgggtgcact gagtgatgaa 780
caggcagcac aggtgcgtcg tctgatggaa agtgccggtg ttaaagttga ttatgaaagc 840
gttccggatg caagccacat gatgcaccag agcgatccgg cacgttatgc agaaattctg 900

accccgtgga ccgcagcact ggcaccgtaa 930

<210> 20
<211> 930
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 4

<400> 20

```
atggttacca gtccggcact gcgtgatgtt catgttccgc atgcatatcc ggaacagcag        60
gttgatctgg gtgaaattac catgaattat gccgaagccg gtgatcctga tcgtccggca       120
gttctgctga tcccggaaca gaccggtagt tggtggtcat atgaagaagc aatgggtctg       180
ctggcagaac attttcatgt ttatgcagtt gatctgcgtg gtcagggtcg tagcagctgg       240
accccgaaac gttatagcct ggataatttt ggtaatgatc tggtgcgttt tattgccctg       300
gttgttaaac gtccggttgt tgttgcaggt aatagcagcg gtggtgttct ggcagcatgg       360
ctgagcgcat atagcatgcc tggtcagctg cgtggtgtgc tgtgtgaaga tccgcctttt       420
tttgcaagcg aactggttcc ggcacatggt catagcgttc gtcagggtgc aggtccggtt       480
tttgaactgt ttcgtaccta tctgggcgat cagtggtcag ttagcgattg ggaaggtttt       540
tgtcgtgcag ccggtgcaag cgcaagcccg atggcacgta gctttgttgc agatggtatt       600
ccgcagcatc tgaaagaata tgatccggaa tgggcacgtg catttcatga aggcaccgtt       660
ggtctgaatt gtccgcatga acgtatgctg ggtcgtgtta atacaccggt gctgctgacc       720
catcatatgc gtggcaccga tccggaaacc ggtaatctgc tgggtgcact gagtgatgaa       780
caggcagcac aggcacgtct gctgatggaa agtgccggtg ttcgtgttga ttatgaaagc       840
gttccggatg caagccatat gatgcaccag agcgatccgg cacgttatgc agaaatcttt       900
acccgttggg cagcagccct ggcaccgtaa                                        930
```

<210> 21
<211> 930
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO : 5

<400> 21

atggttacca gtccggcact gcgtgatgtt catgttccgc atgcatatcc ggaacagcag 60

gttgatctgg gtgaaattac catgaattat gccgaagccg gtgatccggg tcgtccggca 120

gttctgctga tcccggaaca gaccggtagt tggtggtctt atgaagaagc aatgggtctg 180

ctggcagaac attttcatgt ttatgcagtt gatctgcgtg gtcagggtcg tagcagctgg 240

accccgaaac gttatagcct ggataatttt ggtaatgatc tggtgcgttt tatggcactg 300

gttgttcgtc gtccggttgt tgttgcaggt aatagcagcg gtggtgttct ggcagcatgg 360

ctgagcgcat atagcatgcc tggtcagatt cgtggtgtgc tgtgtgaaga tccgcctttt 420

tttgcaagcg aactggttcc ggcacatggt catagcgttc gtcagggtgc aggtccggtt 480

tttgaactgt ttcgtaccta tctgggcgat cagtggtcag ttggtgattg ggaaggtttt 540

cgtagcgcag ccggtgcaag cgcaagcccg atggcacgta gctttgttgc agataccatt 600

ccgcagcatc tgaaagaata tgatccggaa tgggcacgtg cattttatga aggcaccgtt 660

ggtctgaatt gtccgcatga acgtatgctg aatcgtgtta atacaccggt gctgctgacc 720

catcacatgc gtggcaccga tccggaaacc ggtaatctgc tgggtgcact gagtgatgaa 780

caggcagcac aggcacgtcg tctgatggaa agtgccggtg ttaaagttga ttatgaaagc 840

gttccggatg caagccacat gatgcaccag agcgatccgg cacgttatgc agaaattctg 900

accccgtggg cagcagccct ggcaccgtaa 930

<210> 22
<211> 930
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO : 6

<400> 22

atggttacca gtccggcact gcgtgatgtt catgttccgc atgcatatcc ggaacagcag 60

gttgatctgg gtgaaattac catgaattat gccgaagccg gtgatcctga tcgtccggca 120

gttctgctga tcccggaaca gaccggtagt tggtggtctt atgaagaagc aatgggtctg 180

ctgagcgaac attttcatgt ttatgcagtt gatctgcgtg gtcagggtcg tagcagctgg 240

accccgaaac gttatagcct ggataatttt ggtaatgatc tggtgcgttt tattgccctg 300

gttgttaaac gtccggttgt tgttgcaggt aatagcagcg gtggtgttct ggcagcatgg 360

ctgagcgcat atagcatgcc tggtcagctg cgtggtgtgc tgtgtgaaga tccgcctttt 420

tttgcaagcg aactggttcc ggcacatggt catagcgttc gtcagggtgc aggtccggtt 480

tttgaactgt ttcgtaccta tctgggcgat cagtggtcag ttggtgattg ggaaggtttt 540

tgtcgtgcag ccggtgcaag cgcaagcccg atggcacgta gctttgttgc agatggtatt 600

ccgcagcatc tgcaagaata tgatccggaa tgggcacgtg ttttttatga aggcaccgtt 660

ggtctgagct gtccgcatga acgtatgctg ggtcaggtta aaacaccggt gctgctgacc 720

catcacatgc gtggtatcga tccggaaacc ggtaatctgc tgggtgcact gagtgatgaa 780

caggccctgc gtgcacgtcg tctgatggat agtgccggtg ttaccgttga ttatgaaagc 840

gttccggatg caagccacat gatgcaccag agcgcaccgg cacgttatgt tgaaatcttt 900

acccgttggg cagcagccct ggcaccgtaa 930

<210> 23
<211> 903
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO : 7

<400> 23

atgccgcacg attatgaaga aaaactggtt gatctgggcg aaatcgatct gaattatgca 60

gaagcaggta gtccggataa accggcactg ctgctgattc cgagccagag cgaaagttgg 120

tggggctatg aagaagcaat gggtctgctg gccgaagatt atcatgtttt tgcagttgat 180

atgcgtggtc agggtcgtag cacctggaca ccgggtcgtt atagcctgga taattttggt 240

aatgatctgg tgcgctttat cgatctggtt attggtcgta ccgttattgt tagcggtaat 300

agcagcggtg gtgttgttgc agcatggctg gcagcattta gcctgcctgg tcaggttcgt 360

gcagcactgg cagaagatgc accgtttttt gcaagcgaac tggacccgaa agtgggtcat 420

accattcgtc aggcagcagg tcatattttt gttaactggc gtgattatct gggtgatcag 480

tggtcagttg gtgattatgc aggttttctg aaagcaatga aaagcagcga agttccgatg 540

ctgcgtcagg ttccgctgcc ggaaaccgca ccgcagaatc tgctggaata tgatccggaa 600

tgggcacgtg cattttatga aggcaccgtt gcacagacct gtccgcatga ttatatgctg 660

agccaggtta aagtgcctat gctggttacc catcatgcac gtatgattga tgaagcaacc 720

agcggtctgg ttggtgcaat gagcgatctg caggttcaga aagcagcaga aattattcgt 780

ggcaccggtg ttcaggttga tgttgttgat ctgccggaag caccgcatat tctgcatcag 840

ctggcaccga aagaatatgt ggaaattctg aataactggg tggaaaaact gcctccggtt 900

taa 903

<210> 24
<211> 924
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 8

<400> 24

atgatccaga acaataaaac cgcaccgtat aaatacaaag aaaaactggt tgatctgggc 60

gaaatcaaaa tgaactatat tgttgccggt gcagatgtta gtccggcact gctgctgatt 120

ccgggtcaga ccgaaagttg gtggggtttt gaagcagcaa ttgagaaact ggaaagcaac 180

tttcaggtgt ttgcaattga tctgcgtggt cagggtaaaa gcacccagac accgggtcgt 240

tatagcctga atctgatggg taatgatctg gttcgtttta ttagcctggt tattaaacgt 300

ccggttattg ttagcggtaa tagcagcggt ggtctgctgg cagcatggct gagcgcctat 360

gcaatgccga atcagattcg tgcaattcat tgtgaagatg caccgttttt taccgcagaa 420

aaagcaccgc tgtatggtca tgcaattcag caggcagcag gtccgatttt tagcctgatg 480

agcaaatttc tgggtgatca gtggtcaatt aacaattggg aaggtctgaa agcagcacag 540

gcaaaagata cccatccggc aaacaaaatg attagccagg ttgaacagcc tccgcagcat 600

ctgaaagaat atgatccgga atggggtcgt gcatttattg aaggcaaatt taacctgaac 660

agtccgcatc ataccctgct gagcgacatt aaaaccccga tgctgtatac ccatcacatg 720

cgttttgaag atccgcagac aggtctgctg attggtgcaa ccagcgattt tcaggcaagc 780

aaaatcaaag aaattgccct gaaaaccggc aatagcttcg aactgattga tgcaccggat 840

gcatttcata gtatgcatga agccgatccg cagcgttttg ttgatattct gaccagctgg 900

attgaacgtc tgaatctgca gtaa 924

<210> 25
<211> 966
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 9

<400> 25

```
atgggtatta gcgaagcagc agatcgtgca gatacctttg ttgcacataa atttgaagaa      60
cagctggttg atctgggtga aattcgtatg aattatgttg cagccggtga tccgaccagt     120
ccggcactgc tgctgattcc ggcacagggt gaaagttggt ggggttatga aaatgcaatt     180
accctgctgg caaatgattt tcgtgttttt gcaattgatc tgcgtggtca gggtcgtagc     240
acctggacac cgggtcgtta taatctgaat acctggggta atgatgtgga acgctttatt     300
gatctggtta ttggtcgtcc gaccctggtt gcaggtaata gcagcggtgg tgttattgca     360
gcatggctgg cagcctatgc aaaaccgggt cagattcgtg gtgcaatgct ggaagatccg     420
cctctgtttg caagccaggc agcaccgcct tatggtccgg gtattatgca gaccctgggt     480
ccgatttttg ttctgtgggc aaaatggctg ggtccgcagt ggtcagttgg tgattgggat     540
ggtatggttg cagcggcacc gcgtgaactg ccggaatttc tgcatccggg tatcgcattt     600
ctgtttggtg atggcaccgg tgaaggtgca gcagcaaccc ctccgcagca tctgaaagaa     660
tatgatccgg aatgggcaca ggcatgggca accgatgttg caaatgcagg ttgtgatcat     720
gcaaccatgc tggcacagaa tcgtgttccg gttctgctga cccatcattt tcatctgacc     780
gatccggata caggccagct gatgggtgca atgaccgata ttcaggcaca gcaggcacgt     840
cgtctgctgg cagcaaccgg tcagccggtt acctttaccg cactggatgc accgcatacc     900
atgcatgatc ctgaacctga acgttatttt gaagttctga ccgaatgggc aagtgcactg     960
gattaa                                                                966
```

<210> 26
<211> 960
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 10

<400> 26

```
atgggtcgtt atgccggtgt ttttggtccg catgcaccgg aaagcaccta tgttggtcat      60
gcatatccgg aacaactgtt tgataccggt gaagttcgtc tgaattatgc agttgccggt     120
gatgcaagcg caagtccgct gctgctgatt ccgggtcaga ccgaaagttg gtggggttat     180
```

gaaccggcaa tgggtctgct ggcagaacat tttcatgttc atgcagttga tctgcgtggt 240
cagggtcgta gcacccgtac accgcgtcgt tataccctgg ataatattgg taatgatctg 300
gtgcgttttc tggatggtgt tattggtcgt ccggcatttg ttagcggtct gagcagcggt 360
ggtctgctga gcgcatggct gagcgccttt gcagaaccgg gtcaggttct ggcagcatgt 420
tatgaagatc cgcctttttt tagcagcgaa ctggacccgg tgattggtcc gggtctgatg 480
agcaccgttg gtccgctgtt tgcactgtat gttaaatatc tgggtgatca gtggtcaatt 540
ggtgattggg atggttttgt tgcaggcgca ccgcaagaac tggcaggttg gcaggcacat 600
gttgcactgg caggcggtac agcagaaccg cctcagcatc tgaaagaata tgatccggaa 660
tggggtcgtg catttgttgg tggcaccttt accaccggtt gtccgcatca ggttatgctg 720
agccaggtta aagttccggt tctgtttacc catcattttc gtatgctgga tgatgaaagc 780
ggtagcctga ttggtgcagc aaccgatgat caggcagcac gtgttgttga actggttgaa 840
aatagtggtg caccgctgac ctatcgtagc tttccgatga tgggtcatag tatgcatgca 900
caagatccgg cactgtttgc aggcaccctg gttgattggt ttaccgcagc acgtagctaa 960

<210> 27
<211> 960
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 11

<400> 27

```
atgggtcgtt atgccggtgt ttttggtccg catgcaccgg aagcaaccta tgttgaacat       60
ggttatccgg aacgtctgtt tgataccggt gaagtgcagc tgaattatgt tgttgccggt      120
gatgcagcag caccgcctct gctgctgatt ccgggtcaga gcgaaagttg gtggggttat      180
gaagcagcaa ttccgctgct ggcacgtcat tttcatgttc atgcagttga tctgcgtggt      240
cagggtcgta gcacccgtac accgggtcgc tataccctgg ataatgttgg taatgatctg      300
gtgcgttttc tggatggtgt tattggtcgt ccggcatttg ttagcggtct gagcagcggt      360
ggtctggcaa gcgcatggct gagcgcattt gcaaaaccgg gtcaggttgt tgcagcatgt      420
tgggaagatc cgcctttttt tagcagcgaa accgcaccga ttgttggtcc gcctattacc      480
gatagcattg gtccgctgtt tggtatgtgg gcacgttatc tgggtgatca gtggtcagtt      540
ggtgattggg atggttttgt tgccgcagtt ccgaccgaac tggcagattg gcaggcacat      600
gttgcactgg ttgttggcac cgcagatcct ccgcagaatc tgcgtgaata tgatccggaa      660
tggggtaaag catttattac cggcaccttt gcagcaagct gtccgcatca tgttatgctg      720
agcaaagtta aagttccggt tctgtatacc catcactttc gcatgattga tgaaggtagt      780
ggtggtctga ttggtgcatg tagcgatatt caggcaggtc gtgttaccca gctggcaaaa      840
tcaggtggtc gtagcgttac ctatcgtagc tttccgatga tggcacatag catgcatggt      900
caagatccgg cactgtttag cgaaaccctg gttgaatggt ttagccgttt taccggttaa      960
```

<210> 28
<211> 969
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 12

<400> 28

```
atgccgaaaa gcgaagcagc agatcgtgca gatagctttg ttagccatga tttcaaagaa        60
aacattgtgg atctgggcga aatccgcatg aattatgttg ttcagggcaa caaaaaaagt       120
ccggcactgc tgctgattcc ggcacagggt gaaagttggt ggggttatga agcagcaatt       180
ccgctgctgg caaaacattt tcaggttttt gcaattgatc tgcgtggtca gggtcgtacc       240
acctggacac cgggtcgtta taccctggat atttttggta atgatgtggt gcgctttatc       300
gatctggtta ttggtcgtga aaccctgatt gcaggtaata gcagcggtgg tctgattggt       360
gcatggctgg cagcatttgc aaaaccgggt caggttcgtg cagttatgct ggaagatccg       420
cctctgtttg caagcgaaat tcgtccgcct tatggtccgg gtatttggca gggtctgggt       480
ccgatgtttg cagcatgggc aaaatggctg ggtccgcagt ggtcaattgg tgattgggat       540
ggtatggtta aagcactgcc ggatgaactg ccggaagatc tgctgcctgg tattggtttt       600
atgctgggtg atggtgaaag tgatggtgca gcaccgaccc ctccgcagca tctgaaagaa       660
tatgatccgg aatggggtgc aagctgggca agcggttttg ccaataccgg ttgtgaacat       720
gaagcagtta ttagccaggt gcgtgttccg gttctgctga cccatcattt tcgtcagatt       780
aatgaagaaa ccggtcatct gatgggtgca ctgagcgatc tgcaggcagc acaggttcgt       840
catatcattg aagaagttgc aggtcaagag gttacctatg ttagcctgga tgcaccgcat       900
accatgcatg aaccgcagcc ggaacgttat accgatgttc tgctggattg ggttaaaaaa       960
ctgggttaa                                                               969
```

<210> 29
<211> 987
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 13

<400> 29

```
atgaattatg caaccgcagg tagcagcgat aaaccggcac tgctgctggt tccgggtcag      60
agcgaaagtt ggtggggtta tgaaatggca atgtggctgc tgaaagatga ttatcaggtt     120
tttgcagttg atatgcgtgg tcagggtcag agtacctgga caccgggtcg ttatagcctg     180
gatacctttg gtaatgatct ggtgaaattc atcgatatcg tgattaaacg tccggttgtt     240
gttagcggtc tgagcagcgg tggtgttgtg agcgcatggc tgagcgcatt tgcaaaacct     300
ggtcagattc gtgcagcagt ttatgaagat ccgcctctgt ttgcaagcca gagcaaaccg     360
gcaattggtc agagtgttat gcagaccgtt gcaggtccgt tttttaacct gtggtataaa     420
tggctgggtg cacagtggac cattggtgat caggcaggta tggttgcagc aatgccgaaa     480
gaaattccgg catggattct gcagtatctg ggtaatacca ccagtggtcc gaccggtctg     540
gatctgacac tgaatgaata tgatccggaa tggggtcatg gttttgttag tggcaccgtt     600
gatgcaacct gtgatcatga agcaatgctg acccatgtta aagttccggt tctgtttacc     660
catcatagcc gtgcaattga tccgtatacc ggtaatctga ttggtagcgt tagcgatacc     720
caggttagct atgcacaggg tctgattacc accaatggca atcagagctt taccctgaaa     780
aactttccgc tggcaagcca tgatatgcat aattctgatc cggcaaccta tgttagcgca     840
attaccacct ggatggcaag cctgggtatt ggtagtgcag ttattccggg tccggttaaa     900
gttgcaagcg caagcgcaca ggttagcgca gcaagcaccg caccgcctag ctgtaccagc     960
accagcgcac cgagcaccgg tcattaa                                         987
```

<210> 30
<211> 843
<212> DNA
<213> Artificial sequence

<220>
<223> ORF was codon optimized and thus differce from natural occuring DNA sequence.

<220>
<221> misc_feature
<223> Artificial DNA sequence encodes polypeptid with SEQ ID NO: 14

<400> 30

```
atgaccgttg ttgatccgcc tgcaccgcgt gattttccgg aactgctggt tgatctgggt      60
gaagttgttc tgaatcatgc agaagcaggt agtccggatc gtccggcact ggttccggtg     120
ccggaacagg gtggtagttg gtggtcttat gaacgtgtta tgccgctgcc tgcacgcgat     180
tttcatgttt ttgcagttga tctgcgtggt cgtggtcgta gcacccgtac accgcgtcgt     240
tatagcctgg atgattttgg taatgatctg gttcgttttc tggccctggt tgttcgccgt     300
ccggcagttg ttgcaggtaa tagcagcggt ggtgttctgg cagcatggtc aagcgcctat     360
gcaatgcctg gtcaggttcg tgcagttctg ctggaagatc cgcctctgtt tagcagcgaa     420
ctgacaccgg tttgtggtcc gggtgttcgt caggcagcag gtccgctgtt tgaactgctg     480
agcacccatc tgggcgatca gtggggtggt ggtcgtccgg gtcgtgttca tggtggcgtt     540
ccgcgtctgg gtctggcagc cgcagcagca gttcgtgttg cacgtcgtgc agcagcaacc     600
gatgcacgtg gtcgccctgg tgcagcacgt ggacgtcctg ccggtgttgg tggtgcagct     660
cgtcgcggtc gcggtggtcg tgaacgcacc ggtacaacca ccgttctgag cggtctgacc     720
ggtagccgta ccagcggcac cggtcgttgt cgtaaaccgt ttcgtctgcg tcagtggtgg     780
gcaggcggtg cccgtggtcc tcctccgcct cgtcagattc gcgcagatgt tcgtacccgt     840
taa                                                                   843
```

<210> 31
<211> 981
<212> DNA
<213> Artifical Sequence

<220>
<221> misc_feature
<223> Artificial DNA sequence encodes polypeptid with SEQ ID NO: 15

<400> 31

```
atgagcgttc cggttacccc gagcgcacgt aatgtttttg ttccgcatgc atttccagag      60
aaacaaattg atctgggtga agtggttctg aattatgcag aagcaggtac accggataaa     120
ccggcattac tgctgctgcc ggaacagacc ggtagttggt ggtcttatga accggcaatg     180
ggtctgctgg cagaacattt tcatgttttt gcagttgatc tgcgtggtca gggtcgtagc     240
acctggacac cgggtcgtta tagcctggat aattttggta atgatctggt gcgttttatt     300
gcactggcaa ttcgtcgtcc ggttgttgtt gcaggttgta gcagcggtgg tgttctggca     360
gcatggctga gcgcctatgc actgcctggt cagattcgtg gtgcactgtg tgaagatgca     420
ccgctgtttg caagcgaact gacaccggca catggtcatg gtgttcgtca gggtgcaggt     480
ccggtttttg aactgtatcg tgattatctg ggcgatcagt ggtcagttgg tgattgggca     540
ggtctggttg cagcagcaca ggcaagtccg gcaaaaatga tgagcctgtt taaaatgcct     600
```

```
ggtgaaccgc ctcagaatct gcgtgaatat gatccggaat gggcacgtgt ttttttgaa       660
ggcaccgttg gtctgcattg tccgcatgat cgtatgctga gccaggttaa aacaccggtt      720
ctgattaccc atcatgcacg taccaccgat ccggaaaccg gtgaatttct gggtgcactg      780
agcgaactgc aggcagaacg tgcacaggcc attattcgtg cagccggtgt tccggttgat      840
tatcagagct ttccggatgc agcacatgca atgcatacca cagaaccggc acgttatgca      900
gcagttctga ccgcatgggc agcaaaactg cctccggttg cagataccag cccgtcagca      960
gcagcaagcg cacatgttta a                                                981
```

<210> 32
<211> 7
<212> PRT
<213> Artificial sequence

<220>
<223> amino acid motif

<220>
<221> PEPTIDE
<222> (1)..(7)

<400> 32

```
Ala Gly Asn Ser Ser Gly Gly
1               5
```

<210> 33
<211> 7
<212> PRT
<213> Artificial sequence

<220>
<223> amino acid motif

<220>
<221> PEPTIDE
<222> (1)..(7)

<400> 33

```
Arg Thr Ile Asp Pro Glu Thr
1               5
```

<210> 34
<211> 7
<212> PRT
<213> Artificial sequence

<220>
<223> amino acid motif

<220>
<221> PEPTIDE

<222> (1)..(7)

<400> 34

**Asp Ala Leu His Met Met His**
**1 5**

<210> 35
<211> 7
<212> PRT
<213> Artificial sequence

<220>
<223> amino acid motif

<220>
<221> PEPTIDE
<222> (1)..(7)

<400> 35

**Ala Gly Asp Ser Ser Gly Gly**
**1 5**

<210> 36
<211> 7
<212> PRT
<213> Artificial sequence

<220>
<223> amino acid motif

<220>
<221> PEPTIDE
<222> (1)..(7)

<400> 36

**Ala Gly Asp Ser Ser Leu Gly**
**1 5**

<210> 37
<211> 7
<212> PRT
<213> Artificial sequence

<220>
<223> amino acid motif

<220>
<221> PEPTIDE
<222> (1)..(7)

<400> 37

**Ala Gly Gln Ser Ser Gly Gly**

1 5

<210> 38
<211> 7
<212> PRT
<213> Artificial sequence

<220>
<223> amino acid motif

<220>
<221> PEPTIDE
<222> (1)..(7)

<400> 38

Ala Gly His Ser Ser Gly Gly
1 5

<210> 39
<211> 7
<212> PRT
<213> Artificial sequence

<220>
<223> amino acid motif

<220>
<221> PEPTIDE
<222> (1)..(7)

<400> 39

Ala Gly Ser Ser Ser Gly Gly
1 5

<210> 40
<211> 7
<212> PRT
<213> Artificial sequence

<220>
<223> amino acid motif

<220>
<221> PEPTIDE
<222> (1)..(7)

<400> 40

Ser Gly Asn Ser Ser Gly Gly
1 5

<210> 41
<211> 7
<212> PRT
<213> Artificial sequence

<220>
<223> amino acid motif

<220>
<221> PEPTIDE
<222> (1)..(7)

<400> 41

```
Ser Gly Asp Ser Ser Gly Gly
1               5
```

<210> 42
<211> 7
<212> PRT
<213> Artificial sequence

<220>
<223> amino acid motif

<220>
<221> PEPTIDE
<222> (1)..(7)

<400> 42

```
Ser Gly Gln Ser Ser Gly Gly
1               5
```

<210> 43
<211> 7
<212> PRT
<213> Artificial sequence

<220>
<223> amino acid motif

<220>
<221> PEPTIDE
<222> (1)..(7)

<400> 43

```
Ser Gly His Ser Ser Gly Gly
1               5
```

<210> 44
<211> 7
<212> PRT
<213> Artificial sequence

<220>
<223> amino acid motif

<220>
<221> PEPTIDE
<222> (1)..(7)

<400> 44

Ser Gly Ser Ser Ser Gly Gly
1               5

<210> 45
<211> 7
<212> PRT
<213> Artificial sequence

<220>
<223> amino acid motif

<220>
<221> PEPTIDE
<222> (1)..(7)

<400> 45

Arg Thr Ile Asp Pro Glu Thr
1               5

<210> 46
<211> 7
<212> PRT
<213> Artificial sequence

<220>
<223> amino acid motif

<220>
<221> PEPTIDE
<222> (1)..(7)

<400> 46

Arg Asp Ile Asp Pro Asp Thr
1               5

<210> 47
<211> 7
<212> PRT
<213> Artificial sequence

<220>
<223> amino acid motif

<220>
<221> PEPTIDE
<222> (1)..(7)

<400> 47

Arg Gly Thr Asp Pro Glu Thr
1               5

<210> 48
<211> 7

<212> PRT
<213> Artificial sequence

<220>
<223> amino acid motif

<220>
<221> PEPTIDE
<222> (1)..(7)

<400> 48

**Arg Gly Ile Asp Pro Glu Thr**
**1 5**

<210> 49
<211> 7
<212> PRT
<213> Artificial sequence

<220>
<223> amino acid motif

<220>
<221> PEPTIDE
<222> (1)..(7)

<400> 49

**Asp Ala Leu His Met Met His**
**1 5**

<210> 50
<211> 7
<212> PRT
<213> Artificial sequence

<220>
<223> amino acid motif

<220>
<221> PEPTIDE
<222> (1)..(7)

<400> 50

**Asp Ala Ser His Met Met His**
**1 5**

<210> 51
<211> 11
<212> PRT
<213> Artificial sequence

<220>
<223> amino acid motif

<220>

<221> PEPTIDE
<222> (1)..(11)

<400> 51

```
Val Val Ala Gly Asn Ser Ser Gly Gly Leu Leu
1               5                   10
```

<210> 52
<211> 11
<212> PRT
<213> Artificial sequence

<220>
<223> amino acid motif

<220>
<221> PEPTIDE
<222> (1)..(11)

<400> 52

```
Ile Val Ala Gly Asn Ser Ser Gly Gly Val Leu
1               5                   10
```

<210> 53
<211> 11
<212> PRT
<213> Artificial sequence

<220>
<223> amino acid motif

<220>
<221> PEPTIDE
<222> (1)..(11)

<400> 53

```
His Ala Arg Thr Ile Asp Pro Glu Thr Gly Glu
1               5                   10
```

<210> 54
<211> 11
<212> PRT
<213> Artificial sequence

<220>
<223> amino acid motif

<220>
<221> PEPTIDE
<222> (1)..(11)

<400> 54

```
His Met Arg Asp Ile Asp Pro Asp Thr Gly His
1               5                   10
```

<210> 55
<211> 11
<212> PRT
<213> Artificial sequence

<220>
<223> amino acid motif

<220>
<221> PEPTIDE
<222> (1)..(11)

<400> 55

**His Met Arg Gly Thr Asp Pro Glu Thr Gly Asn**
**1 5 10**

<210> 56
<211> 11
<212> PRT
<213> Artificial sequence

<220>
<223> amino acid motif

<220>
<221> PEPTIDE
<222> (1)..(11)

<400> 56

**His Pro Asp Ala Leu His Met Met His Leu Phe**
**1 5 10**

<210> 57
<211> 11
<212> PRT
<213> Artificial sequence

<220>
<223> amino acid motif

<220>
<221> PEPTIDE
<222> (1)..(11)

<400> 57

**Val Pro Asp Ala Leu His Met Met His Gln Phe**
**1 5 10**

<210> 58
<211> 11
<212> PRT
<213> Artificial sequence

<220>
<223> amino acid motif

<220>
<221> PEPTIDE
<222> (1)..(11)

<400> 58

```
Val Pro Asp Ala Ser His Met Met His Gln Ser
1               5                   10
```

<210> 59
<211> 21
<212> PRT
<213> Artificial sequence

<220>
<223> amino acid motif

<220>
<221> PEPTIDE
<222> (1)..(21)

<400> 59

```
Ile Lys Arg Pro Val Val Val Ala Gly Asn Ser Ser Gly Gly Leu Leu
1               5                   10                  15

Ala Ala Trp Leu Ser
            20
```

<210> 60
<211> 21
<212> PRT
<213> Artificial sequence

<220>
<223> amino acid motif

<220>
<221> PEPTIDE
<222> (1)..(21)

<400> 60

```
Val Lys Arg Pro Val Ile Val Ala Gly Asn Ser Ser Gly Gly Val Leu

1               5                   10                  15

Ala Ala Trp Leu Ser
            20
```

<210> 61
<211> 21
<212> PRT
<213> Artificial sequence

<220>
<223> amino acid motif

<220>
<221> PEPTIDE
<222> (1)..(21)

<400> 61

```
Val Arg Arg Pro Val Val Val Ala Gly Asn Ser Ser Gly Gly Val Leu
1               5                   10                  15

Ala Ala Trp Leu Ser
            20
```

<210> 62
<211> 21
<212> PRT
<213> Artificial sequence

<220>
<223> amino acid motif

<220>
<221> PEPTIDE
<222> (1)..(21)

<400> 62

```
Val Lys Arg Pro Val Val Val Ala Gly Asn Ser Ser Gly Gly Val Leu
1               5                   10                  15

Ala Ala Trp Leu Ser
            20
```

<210> 63
<211> 21
<212> PRT
<213> Artificial sequence

<220>
<223> amino acid motif

<220>
<221> PEPTIDE
<222> (1)..(21)

<400> 63

```
Ile Leu Ile Thr His His Ala Arg Thr Ile Asp Pro Glu Thr Gly Glu
1               5                   10                  15

Leu Leu Gly Ala Leu
            20
```

<210> 64
<211> 21
<212> PRT
<213> Artificial sequence

<220>
<223> amino acid motif

<220>
<221> PEPTIDE
<222> (1)..(21)

<400> 64

```
Val Leu Leu Thr His His Met Arg Asp Ile Asp Pro Asp Thr Gly His
1               5                   10                  15

Leu Val Gly Ala Leu
            20
```

<210> 65
<211> 21
<212> PRT
<213> Artificial sequence

<220>
<223> amino acid motif

<220>
<221> PEPTIDE
<222> (1)..(21)

<400> 65

```
Val Leu Leu Thr His His Met Arg Gly Thr Asp Pro Glu Thr Gly Asn
1               5                   10                  15

Leu Leu Gly Ala Leu
            20
```

<210> 66
<211> 21
<212> PRT
<213> Artificial sequence

<220>
<223> amino acid motif

<220>
<221> PEPTIDE
<222> (1)..(21)

<400> 66

```
Val Leu Leu Thr His His Pro Asp Ala Leu His Met Met His Leu Phe
1               5                   10                  15

Leu Leu Gly Ala Leu
            20
```

<210> 67
<211> 21
<212> PRT
<213> Artificial sequence

<220>
<223> amino acid motif

<220>
<221> PEPTIDE
<222> (1)..(21)

<400> 67

```
Val Asp Tyr Gln Ser His Pro Asp Ala Leu His Met Met His Leu Phe
1               5                   10                  15

Asp Pro Ala Arg Tyr
            20
```

<210> 68
<211> 21
<212> PRT
<213> Artificial sequence

<220>
<223> amino acid motif

<220>
<221> PEPTIDE
<222> (1)..(21)

<400> 68

```
Val Asp Tyr Ala Ser Val Pro Asp Ala Leu His Met Met His Gln Phe
1               5                   10                  15

Asp Pro Pro Arg Tyr
            20
```

<210> 69
<211> 21
<212> PRT
<213> Artifical sequence

<220>
<221> PEPTIDE
<222> (1)..(21)

<400> 69

```
Val Asp Tyr Glu Ser Val Pro Asp Ala Ser His Met Met His Gln Ser
1               5                   10                  15

Ala Pro Ala Arg Tyr
            20
```

## Beschreibung

Die vorliegende Erfindung bezieht sich auf ein Verfahren zur hydrolytischen Spaltung von Zearalenon und/oder von wenigstens einem Zearalenon-Derivat mit einem Polypeptid, sowie einen Zusatzstoff enthaltend ein derartiges Polypeptid als auch auf eine Verwendung eines derartigen Polypeptids.

Mykotoxine sind von filamentösen Pilzen produzierte Sekundärmetabolite. Ein wichtiger Vertreter derselben ist das weltweit verbreitete Zearalenon (ZEN), früher bekannt als F-2 Toxin, das durch eine Vielzahl von *Fusarien*-Pilzen produziert wird. Diese Pilze befallen unter Anderem Kulturpflanzen, wie verschiedene Getreidearten, wobei in der Regel der Pilzbefall vor der Ernte auftritt, wobei das Pilzwachstum bzw. die Mykotoxinproduktion vor oder bei nicht sachgemäßer Lagerung auch nach der Ernte erfolgen kann. Die FAO schätzt, dass weltweit 25 % der agrarischen Produkte mit Mykotoxinen kontaminiert sind, was zu erheblichen wirtschaftlichen Einbußen führt. In einer kürzlich weltweit durchgeführten Studie wurden von Jänner 2009 bis Dezember 2011 insgesamt 23.781 Proben analysiert, wobei 81 % positiv auf mindestens ein Mykotoxin und 45 % positiv auf ZEN getestet wurden. ZEN konnte in allen Regionen der Welt ebenso wie in allen getesteten Getreide- und Futtermittelklassen, wie beispielsweise Mais, Sojamehl, Weizen, Weizenkleie, DDGS (Trockenschlempe) sowie in Fertigfuttermischungen mit einer Häufigkeit von bis zu 100 % gefunden werden.

ZEN ist ein nicht steroides, östrogenes, makrozyklisches, über den Polyketid-Stoffwechselweg synthetisiertes Lacton mit der Strukturformel:

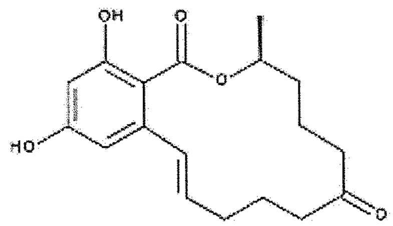

und dem IUPAC Nomenklaturnamen (2E,11S)-15,17-dihydroxy-11-methyl-12-oxabicyclo[12.4.0]octadeca-1(18),2,14,16-tetraene-7,13-dion.

In der Natur kommen jedoch auch eine Vielzahl von ZEN-Derivaten vor, die durch enzymatische oder chemische Modifikationen von ZEN entstehen. Beispiele hierfür sind glykosidische oder sulfathältige ZEN-Konjugate, die durch Pilze, Pflanzen oder dem Säugetiermetabolismus gebildet werden, sowie ZEN-Metabolite, die unter anderem im menschlichen oder tierischen Organismus gebildet werden. Im Folgenden werden unter ZEN-Derivaten als in der Natur vorkommende oder durch chemische oder biochemische Synthese hergestellte ZEN-Konjugate oder ZEN-Metabolite, aber im speziellen α-Zearalenol (α-ZEL; (2E,7R,11S)-7,15,17-trihydroxy-11-methyl-12-oxabicyclo [12.4.0] octadeca-1(18),2,14,16-tetraen-13-on), β-Zearalenol (β-ZEL; (2E,7S,11S)-7,15,17-trihydroxy-11-methyl-12-oxabicyclo[12.4.0] octadeca-1(18),2,14,16-tetraen-13-on), α-Zearalanol (α-ZAL; (7R,11S)-7,15,17-trihydroxy-11-methyl-12-oxabicyclo[12.4.0]octadeca 1(18),14,16-trien-13-on), β-Zearalanol (β-ZAL; (7S,11S)-7,15,17-trihydroxy-11-methyl-12-oxabicyclo [12.4.0] octadeca-1(14),15,17-trien-13-on), Zearalenon-14-Sulfat (Z14S; [(2E,11S)-15-hydroxy-11-methyl-7,13-dioxo-12-oxabicyclo[12.4.0]octadeca-1(18),2,14,16-tetraen-17-yl] hydrogen-sulfat), Zearalenon-14-glycosid (Z14G; (2E,11S)-15-hydroxy-11-methyl-17-[(3R,4S,5S,6R)-3,4,5-trihydroxy-6-(hydroxymethyl)tetrahydropyran-2-yl]oxy-12-oxabicyclo[12.4.0]octadeca-1(18)2,14,16-tetraene-7, 13-dion), sowie Zearalanon (ZAN; (11S)-15,17-dihydroxy-11-methyl-12-oxa-bicyclo [12.4.0] octadeca-1(18), 14,16-triene-7,13-dion) verstanden.

ZEN ebenso wie auch ZEN-Derivate, vor allem α-ZEL, β-ZEL, Z14S, α-ZAL, β-ZAL, Z14G und ZAN können aufgrund ihrer hohen chemischen und physikalischen Stabilität auch in verarbeiteten Lebens- oder Futtermitteln, wie z.B. Brot oder Bier nachgewiesen werden.

ZEN bindet an den Östrogenrezeptor und kann hormonelle Störungen verursachen, wobei es unmittelbar nach der oralen Aufnahme absorbiert und von Säugetieren in die zwei stereoisomeren Metaboliten α-ZEL bzw. β-ZEL umgewandelt wird. Hierbei weisen z.B. α-ZEL, aber auch α-ZAL bzw. ZAN eine weitaus stärkere östrogene Wirkung auf als ZEN. Konjugierte ZEN-Derivate weisen mitunter eine geringere Östrogenität als ZEN auf, jedoch kann gegebenenfalls aus diesen ZEN-Derivaten im Verdauungstrakt wieder ZEN freigesetzt werden.

Obwohl ZEN eine relativ geringe akute Toxizität aufweist und einen oralen LD50-Wert von bis zu 20.000 mg/kg Körpergewicht besitzt, können bei längerer Einnahme subakute und/oder subchronische toxische Wirkungen, wie beispielsweise teratogene, karzinogene, östrogene und immunsupressive Wirkungen bei Tieren oder Menschen auftreten. Mit ZEN kontaminiertes Futtermittel führt zu Entwicklungsstörungen bei Säugetieren, wobei Schweine, und im speziellen Jungtiere äußerst sensitiv gegenüber ZEN sind. ZEN-Konzentrationen im Futtermittel von über 0,5 ppm führen zu Entwicklungsstörungen, wobei z.B. Konzentrationen von über 1,5 ppm zu Hyperöstrogenität bei Schweinen führen können und Konzentrationen von 12 ppm ZEN für Fehlgeburten bei Rindern verantwortlich gemacht wurden. Da Zearalenon schnell über die Schleimhäute, insbesondere über die Magen- aber auch über die Mundschleimhaut aufgenommen wird, ist eine unverzügliche und vor allem quantitative Deaktivierung notwendig. Bereits 30 Minuten nach oraler Gabe von ZEN kann dieses im Blut nachgewiesen werden. Hierbei weist der Einsatz isolierter Enzyme gegenüber Mikroorganismen Vorteile, wie eine höhere spezifische Aktivität oder eine schnellere Wirkung auf. Aufgrund der schädlichen Wirkung von ZEN gibt es in der Europäischen Union verbindliche ZEN-Obergrenzen in Lebensmitteln sowie Empfehlungen für ZEN-Obergrenzen in Futtermitteln (EC NO: 1881/2006).

Die primäre Strategie, um die ZEN-Kontamination von Lebensmitteln oder Futtermitteln zu reduzieren, ist die Einschränkung des Pilzwachstums, beispielsweise durch Einhaltung der "guten landwirtschaftlichen Praxis". Dazu zählt unter anderem, dass Saatgut frei von Schädlingen und Pilzbefall ist, oder dass landwirtschaftliche Abfallprodukte rechtzeitig vom Feld entfernt werden. Des Weiteren kann durch den Einsatz von Fungiziden das Pilzwachstum auf dem Feld reduziert werden. Nach der Ernte sollte das Erntegut bei einer Restfeuchtigkeit von unter 15 % und geringer Temperatur gelagert werden, um das Pilzwachstum zu verhindern. Ebenso sollte vor der Weiterverarbeitung durch Pilzbefall kontaminiertes Gut entfernt werden. Trotz dieser Liste von Maßnahmen berichteten I. Rodriges und K. Naehrer (2012), dass selbst in Regionen mit den höchsten landwirtschaftlichen Standards, wie den USA und Zentraleuropa in den Jahren 2009 bis 2011 29 % bzw. 39 % der getesteten Maisproben mit ZEN kontaminiert waren.

Weitere Möglichkeiten zur Entfernung von ZEN aus Futter- oder Lebensmitteln sind die Adsorption bzw. die Transformation des Mykotoxins. Hierfür ist es erforderlich, dass die Bindung des Mykotoxins zum Adsorbens stark und spezifisch über einen weiten pH-Bereich ist und während des gesamten Verdauungsprozesses im gastrointestinalen Bereich stabil bleibt. Obwohl einige nicht-biologische Adsorptionsmittel, wie z.B. Aktivkohle, Silikate oder synthetische Polymere, wie Cholestryamin, für Aflatoxine effizient eingesetzt werden können, ist ihr Einsatz für andere Mykotoxine beschränkt. Der wesentliche Nachteil der Adsorptionsmittel ist die nicht-spezifische Bindung von anderen Molekülen, die teilweise essentiell für die Ernährung sind. Biologische Adsorptionsmittel, wie z.B. Hefe oder Hefeextrakte sind in der Literatur ebenfalls beschrieben, haben jedoch eine ähnliche Limitierung wie nicht-biologische Adsorptionsmittel.

Auch die Detoxifikation von ZEN durch physikalische und chemische Behandlungen ist limitiert. Durch thermische Behandlung kann ZEN nicht effektiv deaktiviert werden, allerdings kann der ZEN-Gehalt durch Extrusion und Behandlung mit Oxidationsmitteln, z.B. für 16 h bei 80 °C mit 10 %iger Wasserstoffperoxidlösung um 83,9 % reduziert werden. Der Einsatz von Extrusionsverfahren und Oxidationsmitteln, wie Ozon oder Wasserstoffperoxid in der Futter- und Lebensmittelherstellung ist auf Grund der hohen Kosten, dem Qualitätsverlust, der teilweise geringen Effizienz und der geringen Spezifität begrenzt.

Auch die Biotransformation von ZEN mittels Mikroorganismen, wie z.B. *Trichosporon mycotoxinivorans, Gliocladium roseum* oder *Bacillus subtilis* Stämmen bzw. daraus isolierten Enzymen wie Hydrolasen oder Peroxidasen sind z.B. in E. Vekiru et al., in Appl. and Environ. Microb., 2010, 76, 7, 2353-2359 beschrieben worden.

Aus der EP 0 938 575 B1 sind ZEN abbauende Eigenschaften von Bakterien der Gattung *Rhodococcus* und *Nocardia,* insbesondere *R. globerulus, R. erythropolis* und *N. globerula* bekannt geworden.

Der WO 02/076205 ist die ZEN abbauende Wirkung von aus *Gliocladium roseum* isolierten Enzymen, unter anderem der $\alpha/\beta$-Hydrolase, Zearalenonhydrolase 1 (ZHD1), entnehmbar, welche den Abbau von ZEN mittels einer katalytischen Triade katalysieren.

Aus der WO 2012/113827 sind rekombinante Zonasen, nämlich ZEN abbauende Enzyme zu entnehmen, die im Magen-Darm-Trakt stabil bleiben, insbesondere sind darin Mikroorganismen, wie *Thermobifidia fusca, Streptomyces exfoliatus, Acidovorans delafieldii* und *Streptomyces sp.* beschrieben.

Der DATABASE UniProt 16. November 2011, "SubName: Full=Alpha/beta hydrolase fold containing protein {ECO:0000313|EMBL:AEM80235.1}", gefunden im EBI accession no. UNIPROT:G2P9U4 ist eine $\alpha/\beta$-Hydrolase Proteinsequenz zu entnehmen, über deren Einsatzmöglichkeiten bzw. Eigenschaften jedoch keine Aussage getroffen ist.

Polypeptide oder Enzyme, die in der Lage sind ZEN und/oder wenigstens ein ZEN Derivat zu hydrolysieren, können auch als Zonasen bezeichnet werden.

Die im Nachfolgenden verwendeten Ausdrücke sind der Fachsprache entnommen und werden jeweils, außer es ist etwas anderes angeführt, in den herkömmlichen Bedeutungen verwendet. So bezieht sich der Ausdruck "Polynukleotid" auf jegliche Arten von genetischem Material aller Längen und Sequenzen, wie z.B. Einzelstrang und Doppelstrang DNS- und RNS- Moleküle, inklusive regulatorischer Elemente, Strukturelemente, Gruppen von Genen, Plasmiden, gesamte Genome und Fragmente davon. Die Bezeichnung "Polypeptid" umfasst Proteine, wie beispielsweise Enzyme, Antikörper sowie Polypeptide mit bis zu 500 Aminosäuren, wie beispielsweise Peptidinhibitoren, Domänen von Proteinen aber auch kurze Polypeptide mit geringen Sequenzlängen, z.B. weniger als 10 Aminosäuren, wie Rezeptoren, Liganden, Peptidhormone, Tags und dgl. Die Bezeichnung "Position" in einem Polynukleotid oder Polypeptid bezieht sich auf eine einzelne, spezifische Base oder Aminosäure in der Sequenz des Polynukleotids oder des Polypeptids.

Die vorliegende Erfindung zielt nun darauf ab, ein Verfahren zur Verfügung zu stellen, mit dem eine schnelle und zuverlässige hydrolytische Spaltung von ZEN und/oder von wenigstens einem ZEN-Derivat ermöglicht wird.

Zur Lösung dieser Aufgabe ist die vorliegende Erfindung im Wesentlichen dadurch gekennzeichnet, dass Zearalenon und/oder wenigstens ein Zearalenon-Derivat durch das Polypeptid das eine Hydrolase mit einer Aminosäuresequenz gewählt aus der Gruppe der Sequenz ID-Nrn. 7, 8, 14 und 15, insbesondere der Sequenz ID Nr. 14 oder eine funktionelle Variante davon ist, hydrolysiert wird, wobei eine Sequenzidentität zwischen der funktionellen Variante und wenigstens einer der Aminosäuresequenzen wenigstens 70 % beträgt.

Die Bezeichnung "Sequenzidentität" entsprechend der vorliegenden Erfindung bezieht sich auf eine prozentuale Sequenzidentität. Für Aminosäuresequenzen und Nukleotidsequenzen kann die Sequenzidentität visuell bestimmt werden, bevorzugt aber mit einem Computerprogramm errechnet werden. Der Sequenzvergleich wird auch innerhalb von Sequenzabschnitten durchgeführt, wobei als Abschnitt eine durchgängige Sequenz der Referenzsequenz zu verstehen ist und bevorzugt eine konservierte Region der Sequenz umfasst.

Im vorliegenden Fall wurde die Sequenzidentität mit Hilfe des Programms NCBI BLAST (Basic Local Alignment Search Tool), insbesondere mit BLASTP für Polypeptide und BLASTN für Polynukleotide, welche auf der Homepage des "National Center for Biotechnology Information" (NCBI; http://www.ncbi.nim.nih.gov/) zur Verfügung gestellt werden, durchgeführt. Damit ist es möglich zwei oder mehrere Sequenzen miteinander nach dem Algorithmus von Altschul et al., 1997 (Nucleic Acids Res., 25:3389-3402) zu vergleichen. Für den Zweck dieser Erfindung wurden die Programme in der Version vom 15. Mai 2013 verwendet. Als Programmeinstellungen wurden die Basiseinstellungen herangezogen, insbesondere aber für den Aminosäuresequenzvergleich: "max target sequence" = 100; "expected treshold" = 10; "word size" = 3; "matrix" = BLOSOM62; "gap costs" = "Existence: 11; Extention: 1"; "computational adjustment" = "Conditional compositional score matrix adjustment "; sowie für den Nukleotidsequenzvergleich Word Size: 11; Expect value: 10; Gap costs: Existence = 5 , Extension = 2; Filter = low complexity activated; Match/Mismatch Scores: 2,-3; Filter String: L; m.

Die Ausdrücke "funktionelle Polypeptidvariante" oder "funktionelle Variante" beziehen sich zum einen auf "allelische Varianten" des Polypeptids und auf "funktionelle Fragmente" des Polypeptids, zum anderen auf "Modifikation" des Polypeptids, wobei die enzymatische Funktion im Wesentlichen unverändert ist. Die Bezeichnung "allelische Variante" bezieht sich auf ein Polypeptid, das durch in der Natur zufällig vorkommende Mutation(en) der Nukleotidsequenz entstanden ist und eine Veränderung der Aminosäuresequenz bewirkt, wobei die enzymatische Funktion davon nicht beeinflusst ist. "Modifikationen" können beispielhaft C- oder N-terminale Fusionen mit Polypeptiden oder mutierte Polypeptide sein, wobei Mutationen durch Substitution, Insertion oder Deletion von wenigstens einer Aminosäure, insbesondere durch ortsspezifische Mutagenese bzw. zufällige Mutagenese, Rekombination und/oder irgendeiner anderen proteinengineering Methode erhalten werden können. Die Ausdrücke Substitution, Insertion und Deletion sind in der in der Gentechnik üblichen und dem Fachmann geläufigen Bedeutung verwendet. Der Ausdruck "funktionelles Fragment" bezieht sich auf einen Teil bzw. eine Teilsequenz eines Polypeptids oder einen Teil bzw. eine Teilsequenz einer funktionellen Variante davon, wobei die enzymatische Funktion im Wesentlichen beibehalten wird. Eine enzymatische Funktion ist dann im Wesentlichen beibehalten, wenn der enzymatische Reaktionsmechanismus unverändert bleibt, d.h. das Mykotoxin an derselben Stelle hydrolysiert wird und die spezifische Restaktivität "funktionelle Variante" wenigstens 5 %, bevorzugt wenigstens 10 %, insbesondere wenigstens 50 %, bezogen auf das ursprüngliche Polypeptid, beträgt.

Durch die Wahl einer derartigen Aminosäuresequenz, oder einer funktionellen Variante davon wurde eine überraschend schnelle und vollständige Hydrolyse von ZEN und/oder von wenigstens einem ZEN-Derivat festgestellt.

Wie es einer bevorzugten Weiterentwicklung der Erfindung entspricht, wird die hydrolytische Spaltung mit einem Polypeptid durchgeführt, das wenigstens einen konservierten Aminosäuresequenzabschnitt oder eine funktionelle Variante davon enthält, wobei die funktionelle Variante des Aminosäuresequenzabschnitts eine Sequenzidentität von wenigstens 70 %, bevorzugt wenigstens 84 %, noch bevorzugter wenigstens 92 % und am bevorzugtesten wenigstens 98 % aufweist und der wenigstens eine konservierte Aminosäuresequenzabschnitt aus der Gruppe der Aminosäuresequenzen +24 bis +50, +52 bis +77, +79 bis +87, +89 bis +145, +150 bis +171, +177 bis +193, +223 bis +228, +230 bis +237, +239 bis +247, +249 bis +255, +257 bis +261, +263 bis +270, +272 bis +279, +297 bis +301 der Sequenz mit der Sequenz ID-Nr. 1 gewählt ist. Durch das Vorhandensein wenigstens eines derartigen konservierten Aminosäuresequenzabschnittes gelingt es ein Polypeptid zur Verfügung zu stellen das neben der schnellen und vollständigen Hydrolyse von ZEN und/oder von wenigstens einem ZEN Derivat auch einen besonders hohen Aktivitätswert im Vergleich zu bis dato bekannten ZEN abbauenden Polypeptiden aufweist.

Gleichbleibend gute Ergebnisse konnten erzielt werden, wenn die funktionelle Variante wenigstens eine Aminosäuremodifikation gewählt aus der Gruppe Substitution, Deletion und Insertion von einer oder mehreren Aminosäuren aufweist.

Indem das Polypeptid eine spezifische Aktivität von wenigstens 0,01 U/mg, bevorzugt wenigstens 0,1 U/mg, insbesondere wenigstens 1 U/mg aufweist; und/oder einen $K_M$-Wert der hydrolytischen Spaltung von ZEN von höchstens 50 $\mu$M, bevorzugt höchstens 3,5 $\mu$M, insbesondere höchstens 0,5 $\mu$M aufweist; und/oder einen $k_{cat}$-Wert der hydrolytischen Spaltung von ZEN von wenigstens 0,05 $s^{-1}$, bevorzugt wenigstens 0,6 $s^{-1}$, insbesondere wenigstens 5 $s^{-1}$ besitzt; und/oder einen $v_{max}$-Wert der hydrolytischen Spaltung von ZEN von wenigstens 0,00001 $\mu M^{-1} s^{-1}$, bevorzugt wenigstens 0,0001 $\mu M^{-1} s^{-1}$, insbesondere wenigstens 0,001 $\mu M^{-1} s^{-1}$ besitzt, können ZEN und/oder ZEN-Derivate besonders rasch und vollständig hydrolysiert, insbesondere detoxifiziert werden.

Weiterhin kann das Polypeptid eine Aminosäuresequenz der Sequenz ID Nr. 15 oder eine funktionelle Variante davon enthalten, wobei die funktionelle Variante wenigstens 70 % Sequenzidentität zu der Aminosäuresequenz aufweist,

und eine pH Stabilität des Polypeptids bei pH 5,0 wenigstens 15 %, bevorzugt 50 % und insbesondere bevorzugt wenigstens 90 % beträgt. Hierdurch kann gewährleistet werden, dass das Polypeptid Zearalenon und/oder wenigstens ein Zearalenon-Derivat auch im sauren Milieu, so wie dies zum Beispiel im Magen von Säugern der Fall ist, spaltet bzw. detoxifiziert. Die pH Stabilität von Polypeptiden ist dabei definiert als die prozentuale Restaktivität der Polypeptide bei pH 5,0 relativ zu der Aktivität beim jeweiligen pH Optimum.

Weiterhin kann das Polypeptid eine Aminosäuresequenz der Sequenz ID Nrn. 15 oder eine funktionelle Variante davon enthalten, wobei die funktionelle Variante wenigstens 70 % Sequenzidentität zu der Aminosäuresequenz aufweist wodurch das Polypeptid die höchste enzymatische Aktivität in einem Temperaturbereich zwischen 30°C und 75°C, bevorzugt zwischen 38°C und 55°C, insbesondere bevorzugt zwischen 38°C und 52°C aufweist. Hierdurch wird gewährleistet, dass Zearalenon und/oder wenigstens ein Zearalenon Derivat auch bei mesophilen Temperaturen, insbesondere bei der Körpertemperatur von Mensch und Nutztieren, durch das Polypeptid hydrolysiert bzw. detoxifiziert wird. Die Temperatur bei der das Polypeptid die höchsten enzymatische Aktivität aufweist wird als Temperaturoptimum des Polypeptids definiert.

Schließlich kann das Polypeptid eine Aminosäuresequenz der Sequenz ID-Nr. 15 oder eine funktionelle Variante davon enthalten, wobei die funktionelle Variante wenigstens 70 % Sequenzidentität zu der Aminosäuresequenz aufweist, und das Polypeptid bis zu einer Temperatur von 90°C, bevorzugt 75°C und insbesondere bevorzugt 60°C temperaturstabil ist. Dadurch ist gewährleistet, dass das Polypeptid und dessen enzymatische Funktion selbst bei erhöhter Temperaturbeanspruchung, wie dies zum Beispiel während des Transportes im Container oder während des Pelletierens von Futter der Fall sein kann, im Wesentlichen intakt bleibt. Die Temperaturstabilität von Polypeptiden ist definiert als die Temperatur, bei der die Polypeptide nach 15-minütiger Vorinkubation eine 50 %ige Restaktivität verglichen mit der Aktivität beim jeweiligen Temperaturoptimum besitzen.

Das Polypeptid kann so ausgewählt sein, dass es eine zur sauerstoffunabhängigen und cofaktorfreien hydrolytischen Spaltung der Estergruppierung des Zearalenons und/oder der ZEN-Derivate geeignete $\alpha/\beta$-Hydrolase ist, welches eine die hydrolytische Spaltung katalysierende Aminosäuren-Triade, bestehend aus Serin, einer sauren Aminosäure, gewählt aus Glutaminsäure und Asparaginsäure, insbesondere Asparaginsäure sowie Histidin aufweist und die katalytische Triade z,B. S128, D264 und H303 ist, wobei die Positionierung relativ zur Sequenz ID-Nr. 1 dargestellt ist.

Die Hydrolyse von ZEN und von ZEN-Derivaten gelingt mit jedem der Polypeptide der Sequenz ID Nrn. 1 bis 15 an der Estergruppe des Zearalenons oder seiner Derivate nach dem folgenden Reaktionsmechanismus:

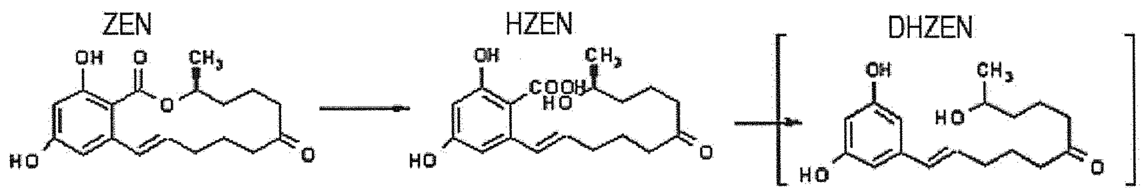

Die Hydrolyse von ZEN zu ungiftigem hydrolysiertem Zearalenon (HZEN) bzw. hydrolysierten ZEN-Derivaten erfolgt durch die erfindungsgemäßen Polypeptide, insbesondere die $\alpha/\beta$-Hydrolasen. Die weitere Decarboxylierung von HZEN zu decarboxyliertem, hydrolysiertem ZEN (DHZEN) bzw. decarboxylierten, hydrolysierten ZEN-Derivaten erfolgt in der Regel spontan.

Insbesondere gelingt es mittels der oben genannten katalytischen Triade ZEN und ZEN-Derivate vollständig zu hydrolysieren, wobei die Abbaureaktion eine gute pH-Stabilität, insbesondere bei pH-Werten im sauren Bereich aufweist.

Weiterhin hat sich herausgestellt, dass es gelingt, mit einem Polypeptid, das in einem aus 3 Aminosäuren vor und 3 Aminosäuren nach dem Serin der oben genannten katalytischen Triade bestehenden Sequenzabschnitt, wenigstens eine polare Aminosäure, gewählt aus Y, Q, N, T, K, R, E, D und wenigstens eine nicht polare Aminosäure, gewählt aus F, M, L, I, V, A, G, P enthält, gleichbleibend gute Ergebnisse zu erzielen und überdies wenigstens einen enzymkinetischen Parameter zu verbessern.

In einer bevorzugten Weiterbildung der Erfindung wird das Verfahren mit einem Polypeptid durchgeführt, das wenigstens eine Mutation der Aminosäuresequenz in Bezug auf die Sequenz ID-Nr. 1 an wenigstens einer der folgenden Positionen 22, 23, 25, 26, 27, 29, 31, 32, 35, 37, 42, 43, 46, 51, 53, 54, 57, 60, 69, 72, 73, 78, 80, 84, 88, 95, 97, 99, 114, 118, 119, 123, 132, 141, 146, 148, 149, 154, 163, 164, 165, 169, 170, 172, 176, 180, 182, 183, 190, 191, 194, 196, 197, 198, 201, 204, 205, 206, 207, 208, 209, 210, 212, 213, 214, 216, 217, 220, 221, 222, 229, 231, 233, 238, 240, 244, 245, 246, 248, 249, 251, 254, 256, 260, 262, 263, 266, 269, 271, 277, 280, 281, 282, 283, 284, 285, 286, 287, 292, 296, 298, 302, 307, 308, 309, 311, 314, 317, 319, 321, 323, 325 und 326 aufweist.

Gemäß einer Weiterbildung der Erfindung wird das Verfahren mit einem Polypeptid durchgeführt, das wenigstens eine Mutation gewählt aus der Gruppe: D22A, S23Q, S23L, N25D, I26V, F27Y, F27H, S29P, R31A, F32Y, R35K, R35Q, V37A, V42I, V43T, F46Y, S51E, S51D, D53G, N54M, N54R, L57V, L60I, S69G, P72E, V73A, A78S, N80H,

F84Y, I88L, T95S, T97A, R99K, I114M, I118V, K119R, V123I, L132V, A141S, I146V, I146L, A148G, A149V, A154P, P163T, A164T, Y165C, Y165H, V169I, L170R, A172G, A176M, A176V, Y180F, D182T, F183Y, I190V, G191S, K194T, K194E, F196Y, V197C, V197R, E198R, E198S, K201D, K201G, P204S, P204A, A205S, K206P, A207M, M208A, Q209R, L210A, L210S, ΔP212, T213V, P214A, E216T, E216G, A217I, N220H, L221M, K222R, K222Q, G229A, A231V, F233W, F233Y, F233H, A238G, H240N, H240S, D244E, R245Q, M246L, S248T, S248N, S248G, Q249R, K251N, I254V, I256L, A260M, T262D, T262G, I263T, E266D, E269H, E269N, L271V, L277E, E280A, E280L, H281R, H281Q, A282V, Q283R, D284L, D284R, I285L, I286M, R287E, R287D, R292K, R292T, Q296A, Q296E, H298V, L302S, L307Q, F308S, D309A, A311P, A314V, L317F, S319Q, S319P, S319R, S321A, S321T, T323A, P325A, A326P in der Aminosäuresequenz in Bezug auf Sequenz ID-Nr. 1 aufweist. Mit einem derartigen Polypeptid gelingt es ZEN innerhalb kurzer Zeit vollständig zu hydrolysieren, insbesondere zu detoxifizieren, wobei die spezifische Aktivität des Polypeptides wenigstens 6,00 U/mg, bevorzugt wenigstens 7,00 U/mg, insbesondere wenigstens 8,00 U/mg beträgt. Die Einheit "U" oder auch "Unit" ist ein Maß für die absolute katalytische Aktivität und wird definiert durch die Hydrolyse von 1 μmol ZEN pro Minute bei 32 °C in 50 mM Tris-HCl Puffer (pH 8,2), wobei - als "katalytische Aktivität" die enzymatische Umsetzung eines Substrats unter definierten Reaktionsbedingungen verstanden wird und unter "spezifischer Aktivität" das Verhältnis der katalytischen Aktivität und der Polypeptid-Massenkonzentration (Masse pro Volumseinheit) verstanden wird.

Indem das Polypeptid so ausgebildet ist, dass das Polypeptid wenigstens eines der folgenden Aminosäuremotive gewählt aus der Gruppe der Sequenz ID-Nrn. 32 bis 69 enthält, gelingt es Polypeptide zur Verfügung zu stellen, die eine spezifische Aktivität von wenigstens 7,00 U/mg, bevorzugt wenigstens 8,00 U/mg aufweisen. Überraschenderweise hat sich gezeigt, dass, wenn wenigstens eines der folgenden Aminosäuremotive mit der Sequenz mit den Sequenz ID-Nrn. 51 bis 58 enthalten ist, die enzymatische Aktivität des Polypeptids, beispielsweise gegenüber einem Motiv enthaltend 7 Aminosäuren noch weiter gesteigert wird. Eine noch höhere spezifische Aktivität wird erreicht, wenn wenigstens eines der folgenden Aminosäuremotive mit der Sequenz mit den Sequenz ID-Nrn. 59 bis 69 enthalten ist.

Schließlich kann das Polypeptid wenigstens eine konservative Aminosäuresubstitution an wenigstens einer Position enthalten, wobei die konservative Aminosäuresubstitution gewählt ist aus Substitutionen von G zu A; oder A zu G, S; oder V zu I, L, A, T, S; oder I zu V, L, M; oder L zu I, M, V; oder M zu L, I, V; oder P zu A, S, N; oder F zu Y, W, H; oder Y zu F, W, H; oder W zu Y, F, H; oder R zu K, E, D; oder K zu R, E, D; oder H zu Q, N, S; oder D zu N, E, K, R, Q; oder E zu Q, D, K, R, N; oder S zu T, A; oder T zu S, V, A; oder C zu S, T, A; oder N zu D, Q, H, S; oder Q zu E, N, H, K, R. Wobei sich die Bezeichnung konservative Aminosäuresubstitution auf die Substitution von Aminosäuren durch andere Aminosäuren, die vom Fachmann als konservativ angesehen werden, das heißt die ähnliche spezifische Eigenschaften besitzen, bezieht. Solche spezifische Eigenschaften sind beispielsweise die Größe, Polarität, Hydrophobizität, Ladung oder der pKs-Wert der Aminosäure. Unter einer konservativen Mutation wird z.B. eine Substitution einer sauren Aminosäure gegen eine andere saure Aminosäure, eine basische Aminosäure gegen eine andere basische Aminosäure oder eine polare gegen eine andere polare Aminosäure verstanden.

Mit derartigen konservativen Aminosäuresubstitutionen gelingt es funktionelle Polypeptidvarianten herzustellen, deren spezifische Aktivität im Vergleich zum parentalen Polypeptid in etwa gleich stark ist, bevorzugt jedoch um wenigstens 0,1 U/mg gesteigert ist.

Indem das Verfahren so geführt wird, dass das Polypeptid in einem Zearalenon und/oder wenigstens ein Zearalenon Derivat spaltendem, Hilfsstoffe enthaltenden, Zusatzstoff zu Futtermitteln für Schweine, Geflügel und Aquakultur, in Nahrungsmittel oder in Trockenschlempe eingesetzt wird, gelingt die biochemische Umwandlung von ZEN und/oder von wenigstens einem ZEN-Derivat zu hydrolysiertem ZEN und/oder zu hydrolysiertem ZEN-Derivat. Das Verfahren kann beispielsweise auch für die stereoselektive Hydrolyse von ZEN und/oder von ZEN-Derivaten in industriellen Prozessen herangezogen werden.

In einer bevorzugten Weiterbildung der Erfindung ist das Verfahren so weitergebildet, dass die Hilfsstoffe aus wenigstens einem inerten Träger, sowie gegebenenfalls weiteren Bestandteilen, wie Vitaminen und/oder Mineralstoffen und/oder Enzymen und/oder weiteren Komponenten zur Detoxifizierung von Mykotoxinen gewählt werden. Dadurch kann beispielsweise in Futter- oder Lebensmitteln sichergestellt werden, dass die gegebenenfalls enthaltenen Mengen an ZEN und/oder an ZEN-Derivaten mit Sicherheit soweit hydrolysiert, insbesondere detoxifiziert werden, dass eine schädliche Wirkung auf den Organismus des dieses Futter- oder Lebensmittel aufnehmenden Subjekts ausbleibt.

Hierbei kann ein Polypeptid auch in einer Enzympräparation vorliegen, die neben wenigstens einem Polypeptid zusätzlich wenigstens ein Enzym enthält, das beispielsweise am Abbau von Proteinen beteiligt ist, wie z.B. Proteasen, oder das beim Metabolismus von Stärke oder Faser oder Fett oder Glycogen beteiligt ist, wie z.B. Amylase, Cellulase oder Glucanase ist, sowie beispielsweise Hydrolasen, lipolytische Enzyme, Mannosidasen, Oxidasen, Oxidoreductasen, Phytasen, Xylanasen und/oder Kombinationen davon.

Weitere Einsatzbereiche sind Enzympräparationen, die neben wenigstens einem Polypeptid zusätzlich wenigstens eine Komponente zur Detoxifizierung von Mykotoxinen enthalten, wie ein Mykotoxin-abbauendes Enzym, wie z.B. Alfatoxin Oxidase, Ergotamin Hydrolasen, Ergotamin Amidasen, Zearalenon Esterasen, Zearalenon Lactonasen, Ochratoxin Amidasen, Fumonisin Carboxylesterasen, Fumonisin Aminotransferasen, Aminopolyol Aminoxidasen, Deoxynivalenol Epoxidhydrolasen; und/oder wenigstens einen Mykotoxin-abbauenden Mikroorganismus, wie *Bacillus sub-*

*tilis;* und/oder wenigstens eine Mykotoxin-bindende Komponente, beispielweise mikrobielle Zellwände oder anorganische Materialien, wie Bentonite enthalten.

Gemäß einer besonders bevorzugten Weiterbildung der Erfindung wird das Verfahren so geführt, dass wenigstens ein Polypeptid in dem Zusatzstoff in einer Konzentration von höchstens 10.000 U/g, bevorzugt höchstens 1.000 U/g, bevorzugter höchstens 100 U/g und am bevorzugtesten höchstens 10 U/g eingesetzt wird, wodurch es gelingt, ZEN und/oder ZEN-Derivate schnell und insbesondere bereits vor deren Resorption durch den Körper, eines, ein kontaminiertes Futter- oder Lebensmittel verzehrenden Subjekts, insbesondere eines Säugern, in nicht bzw. weniger toxische Metabolite, insbesondere HZEN und DHZEN, umzuwandeln.

Gemäß einer Weiterbildung der Erfindung liegt der Zusatzstoff in verkapselter oder gecoateter Form vor, wobei für das Verkapseln oder Coaten Standardmethoden, wie z.B. in der WO 92/12645 beschrieben, herangezogen werden können. Durch das Verkapseln bzw. Coaten gelingt es, das Polypeptid ohne Veränderung, insbesondere ohne Abbau und Schädigung an seinen Einsatzort zu transportieren, so dass erst nach Auflösung der Schutzhülle, beispielsweise im Verdauungstrakt von Tieren, das Polypeptid zu wirken beginnt, wodurch ein noch gezielterer, rascher und vollständiger Abbau von ZEN und/oder von ZEN-Derivaten auch im sauren, proteasereichen und anaeroben Milieu erzielt werden kann. Des Weiteren gelingt es durch die Verkapselung oder das Coaten auch die Temperaturstabilität der Polypeptide im Zusatzstoff zu erhöhen.

Gemäß einer bevorzugten Weiterentwicklung ist das Verfahren so geführt, dass darin das Polypeptid oder der Zusatzstoff mit einem mit Zearalenon und/oder mit wenigstens einem Zearalenon-Derivat kontaminierten Futter- oder Nahrungsmittel versetzt wird, dass das kontaminierte Futter- oder Nahrungsmittel mit Feuchtigkeit in Kontakt gebracht wird, und dass das Polypeptid oder der Zusatzstoff das im kontaminierten Futter- oder Nahrungsmittel enthaltende Zearalenon und/oder wenigstens ein Zearalenon-Derivat hydrolysiert. Bei feuchten Futter- oder Nahrungsmitteln, wie Maische oder Breien, wird die Hydrolyse des Zearalenons und/oder des wenigstens einen Zearalenon-Derivats vor der oralen Aufnahme im feuchten Futter- oder Nahrungsmittel stattfinden. Durch dieses Verfahren kann sichergestellt werden, dass die schädigenden Wirkungen von Zearalenon und von Zearalenon-Derivaten auf Mensch und Tier weitestgehend eliminiert werden. Als Feuchtigkeit wird hierbei die Anwesenheit von Wasser oder wasserhaltigen Flüssigkeiten verstanden, wobei darunter auch z.B. Speichel oder andere im Verdauungstrakt vorhandenen Flüssigkeiten fallen. Als Verdauungstrakt wird die Mundhöhle, der Pharynx (Rachen), die Speiseröhre und der Magen-Darm-Trakt oder Äquivalente davon definiert, wobei unterschiedliche Bezeichnungen bei Tieren vorkommen können bzw. einzelne Bestandteile nicht im Verdauungstrakt von Tieren vorhanden sein können.

Das erfinderische Verfahren kann auch so geführt werden, dass das Futter- oder Nahrungsmittel vor der oralen Aufnahme pelletiert wird.

Gemäß einer Weiterbildung der Erfindung ist das Verfahren so geführt, dass wenigstens 70 %, bevorzugt wenigstens 80 %, insbesondere wenigstens 90 % des Zearalenons und/oder wenigstens eines Zearalenon Derivats hydrolysiert werden. Dadurch können subakute und/oder subchronische toxische Wirkungen, wie beispielsweise teratogene, karzinogene, östrogene und immunsupressive Wirkungen bei Tieren oder Menschen, vorgebeugt werden.

Die vorliegende Erfindung zielt des Weiteren auf eine Verwendung eines wenigstens ein Polypeptid mit einer Aminosäuresequenz gewählt aus der Gruppe der Sequenz ID-Nrn. 7, 8, 14 und 15 oder einer funktionellen Variante davon, wobei eine Sequenzidentität zwischen der funktionellen Variante und wenigstens einer der Aminosäuresequenzen wenigstens 70 % beträgt, enthaltenden Zusatzstoffes zur hydrolytischen Spaltung von Zearalenon und/oder wenigsten einem Zearalenon-Derivat in Futtermitteln, insbesondere für Schweine, Geflügel und Aquakultur, in Nahrungsmitteln oder in Trockenschlempe ab. Durch die erfindungsgemäße Verwendung des Zusatzstoffes gelingt es das im Nahrungs- oder Futtermittel bzw. in Trockenschlempe enthaltene ZEN und/oder die ZEN-Derivate zu hydrolysieren bzw. zu detoxifizieren, wobei eine derartige Entgiftung bereits bei Polypeptidkonzentrationen von etwa 1 U/g kontaminiertem Futter- oder Nahrungsmittel gelingt.

Die Erfindung wird nachfolgend anhand von Ausführungsbeispielen sowie Zeichnungen näher erläutert. In diesen zeigt:

Fig. 1, die nicht Teil der Erfindung ist, den zeitlichen Abbau von ZEN und die Zunahme der Metabolite HZEN und DHZEN durch das Polypeptid mit der Sequenz ID-Nr. 1, wobei in Fig. 1A das Polypeptid nicht getagt ist, in Fig. 1B das Polypeptid einen C-terminalen 6xHis Tag und in Fig. 1C einen N-terminalen 6xHis Tag besitzt,

Fig. 2, die nicht Teil der Erfindung ist, die Michaelis-Menten Kinetik des Polypeptids mit der Sequenz ID-Nr. 1,

Fig. 3 den zeitlichen Abbau von ZEN und die Zunahme der Metabolite HZEN und DHZEN durch die aufgereinigten Polypeptide mit den Sequenz ID-Nrn. 1 (Fig. 3A), 7 (Fig. 3B) und 15 (Fig. 3C), wobei alle Sequenzen einen C-terminalen 6xHis Tag besitzen.

Beispiel 1: Modifikation, Klonierung und Expression von Polynukleotiden, die für Polypeptide codieren, welche ZEN und/oder wenigstens ein ZEN-Derivat hydrolytisch spalten können.

Aminosäuresubstitutionen, -insertionen oder -deletionen wurden durch Mutation der Nukleotidsequenzen mittels PCR unter Verwendung des "Quick-change Site-directed Mutagenesis Kits" (Stratagene) laut Anleitung durchgeführt. Alternativ dazu wurden auch komplette Nukleotidsequenzen bezogen (GeneArt). Die mittels PCR-Mutagenese generierten bzw. von GeneArt bezogenen Nukleotidsequenzen enthielten auf Aminosäureebene optional zusätzlich einen C- oder N-terminalen 6xHis-Tag und wurden mittels Standardmethoden in Expressionsvektoren zur Expression in *E. coli* oder *P. pastoris* integriert, in *E. coli* oder *P. pastoris* transformiert, sowie in *E. coli* oder *P. pastoris* exprimiert (J.M. Cregg, Pichia Protocols, second Edition, ISBN-10: 1588294293, 2007; J. Sambrook et al. 2012, Molecular Cloning, A Laboratory Manual 4th Edition, Cold Spring Harbor), wobei für diese Aufgabe auch jede andere geeignete Wirtszelle herangezogen werden kann.

Die Bezeichnung "Expressionsvektor" bezieht sich auf ein DNS Konstrukt das in der Lage ist *in vivo* oder *in vitro* ein Gen zu exprimieren. Im Besonderen werden darunter DNS Konstrukte verstanden, die geeignet sind, die das Polypeptid codierende Nukleotidsequenz in die Wirtszelle zu transferieren, um dort ins Genom zu integrieren oder frei im extrachromosomalen Raum vorzuliegen und die das Polypeptid codierende Nukleotidsequenz intrazellulär zu exprimieren und gegebenenfalls das Polypeptid aus der Zelle auszuschleusen.

Die Bezeichnung "Wirtszelle" bezieht sich auf alle Zellen, die entweder eine zu exprimierende Nukleotidsequenz oder einen Expressionsvektor enthalten und in der Lage sind, ein erfindungsgemäßes Polypeptid herzustellen. Im Besonderen werden darunter prokaryotische und/oder eukaryotische Zellen verstanden, bevorzugt *P. pastoris, E. coli, Bacillus subtilis, Streptomyces, Hansenula, Trichoderma, Lactobacillus, Aspergillus,* Pflanzenzellen und/oder Sporen von *Bacillus, Trichoderma* oder *Aspergillus.*

Das lösliche Zelllysat im Falle von *E. coli* beziehungsweise der Kulturüberstand im Falle von P. *pastoris* wurden zur Bestimmung der katalytischen Eigenschaften der Polypeptide herangezogen. Zur Bestimmung des $K_M$-Werts, von $v_{max}$, $k_{cat}$ und der spezifischen Aktivität wurden die Polypeptide mittels Standardmethoden chromatographisch über Nickel-Sepharosesäulen selektiv angereichert. Die Bestimmung der Proteinkonzentration erfolgte mittels Standardmethoden, entweder mit der BCA Methode (Pierce BCA Protein Assay KitProd # 23225), bevorzugt jedoch photometrisch mit den spezifischen Extinktionskoeffizienten für die jeweiligen Proteine, die mit dem Programm "ProtParam", online verfügbar auf http://web.expasy.org/protparam (Gasteiger E. et al.; Protein Identification and Analysis Tools on the ExPASy Server*;* (In) John M. Walker (ed): The Proteomics Protocols Handbook, Humana Press, 2005, pp. 571-607) berechnet wurden.

Beispiel 2: Bestimmung der Sequenzidentität sowie der konservierten Aminosäuresequenzabschnitte

Die Bestimmung der prozentualen Sequenzidentität über die gesamte Polypeptidlänge der Polypeptide mit den Aminosäuresequenzen mit den Sequenz ID-Nrn. 1 bis 15 relativ zueinander (Tabelle 1) wurde mit Hilfe des Programms BLAST (Basic Local Alignment Search Tool), insbesondere mit BLASTP, welches auf der Homepage des "National Center for Biotechnology Information" (NCBI; http://www.ncbi.nlm.nih.gov/) benutzt werden kann, durchgeführt. Damit ist es möglich zwei oder mehrere Sequenzen miteinander nach dem Algorithmus von Altschul et al., 1997 (Nucleic Acids Res. (1997) 25:3389-3402) zu vergleichen. Als Programmeinstellungen wurden die Basiseinstellungen herangezogen, insbesondere aber: "max target sequence" = 100; "expected treshold" = 10; "word size" = 3; "matrix" = BLOSOM62; "gap costs" = "Existence: 11; Extention: 1"; "computational adjustment" = "Conditional compositional score matrix adjustment ".

Zur Bestimmung der konservierten Aminosäuresequenzabschnitte wurden die Polypeptide mit den Sequenz ID Nrn. 1 bis 6, die eine Sequenzidentität von wenigstens 70 % zueinander besitzen, mit Hilfe der Software COBALT (J.S. Papadopoulos und R. Agarwala, 2007, COBALT: constraint-based alignment tool for multiple protein sequences, Bioinformatics 23:1073-79.) unter Heranziehung der Standardparameter, insbesondere der Parameter ("Gap penalties": -11, -1; "End-Gap Penalties": -5, -1; "Use RPS BLAST": on; "Blast E-value": 0,003; "Find Conserved columns and Recompute": on; "use query clusters": on; "word size": 4; "may cluster distance": 0,8; "Alphabet": regular; "Homology conversation setting": 3 bits) abgeglichen. Das Resultat dieser Analyse stellt die konservierten Aminosäuren dar. Als konservierte Aminosäuresequenzabschnitte wurden die folgenden Bereiche von wenigstens 5 aufeinanderfolgenden konservierten Aminosäuren definiert, nämlich in Bezug auf die Sequenz mit der Sequenz ID-Nr. 1 die Abschnitte A von Position +24 bis Position +50, B von Position +52 bis Position +77, C von Position +79 bis Position +87, D von Position +89 bis Position +145, E von Position +150 bis Position +171, F von Position +177 bis Position +193, G von Position +223 bis Position +228, H von Position +230 bis Position +237, I von Position +239 bis Position +247, J von Position +249 bis Position +255, K von Position +257 bis Position +261, L von Position +263 bis Position +270, M von Position +272 bis Position +279, N von Position +297 bis Position +301 und O von Position +303 bis Position +313.

Die Bestimmungen der prozentualen Sequenzidentität der Polypeptide zueinander sowie der konservierten

Aminosäuresequenzabschnitte der einzelnen Polypeptide relativ zu den konservierten Aminosäuresequenzabschnitten der Sequenz mit der Sequenz ID-Nr. 1 wurden, wie oben beschrieben, beispielhaft durchgeführt. Die Ergebnisse sind in Tabellen 1 und 2 dargestellt.

Tabelle 1: Prozentuale Sequenzidentität der Polypeptide zueinander.

| | SEQ ID-Nr 1 | SEQ ID-Nr 2 | SEQ ID-Nr. 3 | SEQ ID-Nr. 4 | SEQ ID-Nr. 5 | SEQ ID-Nr. 6 | SEQ ID-Nr. 7 |
|---|---|---|---|---|---|---|---|
| SEQ ID-Nr. 1 | - | 70% | 71% | 71% | 71% | 71% | 64% |
| SEQ ID-Nr. 2 | 70% | - | 81% | 83% | 81% | 83% | 63% |
| SEQ ID-Nr. 3 | 71% | 81% | - | 95% | 99% | 92% | 60% |
| SEQ ID-Nr. 4 | 71% | 83% | 95% | | 95% | 95% | 60% |
| SEQ ID-Nr. 5 | 71% | 81% | 99% | 95% | - | 93% | 60% |
| SEQ ID-Nr. 6 | 71% | 83% | 92% | 95% | 93% | - | 61% |
| SEQ ID-Nr. 7 | 64% | 63% | 60% | 60% | 60% | 61% | - |
| SEQ ID-Nr. 8 | 57% | 54% | 54% | 53% | 53% | 53% | 53% |
| SEQ ID-Nr. 9 | 50% | 50% | 53% | 53% | 53% | 55% | 51% |
| SEQ ID-Nr. 10 | 55% | 52% | 55% | 54% | 55% | 53% | 52% |
| SEQ ID-Nr. 11 | 53% | 51% | 53% | 51% | 51% | 52% | 54% |
| SEQ ID-Nr. 12 | 50% | 49% | 50% | 50% | 50% | 49% | 51% |
| SEQ ID-Nr. 13 | 55% | 49% | 51% | 51% | 51% | 52% | 54% |
| SEQ ID-Nr. 14 | 73% | 65% | 69% | 70% | 69% | 68% | 80% |

| | | | | | | | |
|---|---|---|---|---|---|---|---|
| SEQ ID-Nr. 15 | 79% | 68% | 71% | 71% | 71% | 72% | 63% |

| | SEQ ID-Nr. 8 | SEQ ID-Nr. 9 | SEQ ID-Nr. 10 | SEQ ID-Nr. 11 | SEQ ID-Nr. 12 | SEQ ID-Nr. 13 | SEQ ID-Nr. 14 | SEQ ID-Nr. 15 |
|---|---|---|---|---|---|---|---|---|
| SEQ ID-Nr. 1 | 57% | 50% | 55% | 53% | 50% | 55% | 73% | 79% |
| SEQ ID-Nr. 2 | 54% | 50% | 52% | 51% | 49% | 49% | 65% | 68% |
| SEQ ID-Nr. 3 | 54% | 53% | 55% | 53% | 50% | 51% | 69% | 71% |
| SEQ ID-Nr. 4 | 53% | 53% | 54% | 51% | 50% | 51% | 70% | 71% |
| SEQ ID-Nr. 5 | 53% | 53% | 55% | 51% | 50% | 51% | 69% | 71% |
| SEQ ID-Nr. 6 | 53% | 55% | 53% | 52% | 49% | 52% | 68% | 72% |
| SEQ ID-Nr. 7 | 53% | 51% | 52% | 54% | 51% | 54% | 80% | 63% |
| SEQ ID-Nr. 8 | - | 50% | 49% | 51% | 49% | 48% | 83% | 51% |
| SEQ ID-Nr. 9 | 50% | - | 51% | 52% | 69% | 51% | 67% | 51% |
| SEQ ID-Nr. 10 | 49% | 51% | - | 76% | 52% | 52% | 63% | 56% |
| SEQ ID Nr. 11 | 41% | 50% | 76% | - | 52% | 51% | 58% | 52% |
| SEQ ID Nr. 12 | 49% | 52% | 52% | 52% | - | 49% | 71% | 51% |
| SEQ ID Nr. 13 | 48% | 51% | 52% | 51% | 49% | - | 54% | 53% |
| SEQ ID No. 14 | 83% | 67% | 63% | 58% | 71% | 55% | - | 72% |
| SEQ ID | 51% | 51% | 56% | 52% | 51% | 53% | 72% | - |

| | | | | | | | | |
|---|---|---|---|---|---|---|---|---|
| Nr. 15 | | | | | | | | |

Tabelle 2: Prozentuale Sequenzidentität der konservierten Aminosäuresequenzabschnitte A bis O.

| | Sequenzidentität relativ zur Sequenz ID-Nr. 1 | | | | | |
|---|---|---|---|---|---|---|
| Polypeptid | Abschnitt A | Abschnitt B | Abschnitt C | Abschnitt D | Abschnitt E | Abschnitt F |
| SEQ ID-Nr. 1 | 100 % | 100 % | 100 % | 100 % | 100 % | 100 % |
| SEQ ID-Nr. 2 | 59,6 % | 76,9 % | 88,9 % | 87,7 % | 77,3 % | 76,5 % |
| SEQ ID-Nr. 3 | 63,0 % | 76,9 % | 77,8 % | 89,5 % | 86,4 % | 76,5 % |
| SEQ ID-Nr. 4 | 63,0 % | 80,8 % | 77,8 % | 91,2 % | 86,4 % | 76,5 % |
| SEQ ID-Nr. 5 | 63,0 % | 76,9 % | 77,8 % | 87,7 % | 86,4 % | 76,5 % |
| SEQ ID-Nr. 6 | 63,0 % | 80,8 % | 77,8 % | 91,2 % | 86,4 % | 76,5 % |
| SEQ ID-Nr. 7 | 44,7 % | 69,2 % | 77,8 % | 78,9 % | 68,2 % | 64,7 % |
| SEQ ID-Nr. 8 | 40,7 % | 50,0 % | 66,7 % | 82,5 % | 59,1 % | 64,7 % |
| SEQ ID-Nr. 9 | 51,9 % | 57,7 % | 55,6 % | 73,7 % | 45,5 % | 58,8 % |
| SEQ ID-Nr. 10 | 44,4 % | 61,5 % | 77,8 % | 75,4 % | 47,8 % | 76,5 % |
| SEQ ID-Nr. 11 | 44,4 % | 50,0 % | 66,7 % | 71,9 % | 43,5 % | 58,8 % |
| SEQ ID-Nr. 12 | 51,9 % | 53,8 % | 55,6 % | 71,9 % | 50,0 % | 58,8 % |
| SEQ ID-Nr. 13 | 18,5 % | 61,5 % | 55,6 % | 77,2 % | 54,5 % | 52,9 % |
| SEQ ID-Nr. 14 | 55,6 % | 69,2 % | 77,8 % | 84,2 % | 54,5 % | 52,9 % |
| SEQ ID-Nr. 15 | 74,1 % | 86,7 % | 88,9 % | 89,0 % | 77,3 % | 88,2 % |

| | Sequenzidentität relativ zu der SEQ ID-Nr: 1 | | | | | |
|---|---|---|---|---|---|---|
| Polypeptid | Abschnitt G | Abschnitt H | Abschnitt I | Abschnitt J | Abschnitt K | Abschnitt L |
| SEQ ID-Nr. 1 | 100 % | 100 % | 100 % | 100 % | 100 % | 100 % |
| SEQ ID-Nr. 2 | 100 % | 87,5 % | 66,7 % | 85,7 % | 80,0 % | 75,0 % |
| SEQ ID-Nr. 3 | 100 % | 87,5 % | 77,8 % | 57,1 % | 80,0 % | 75,0 % |
| SEQ ID-Nr. 4 | 100 % | 87,5 % | 77,8 % | 57,1 % | 80,0 % | 75,0 % |
| SEQ ID-Nr. 5 | 100 % | 87,5 % | 77,8 % | 57,1 % | 80,0 % | 75,0 % |
| SEQ ID-Nr. 6 | 100 % | 75,0 % | 77,8 % | 85,7 % | 80,0 % | 87,5 % |
| SEQ ID-Nr. 7 | 100 % | 87,5 % | 66,7 % | 71,4 % | 100 % | 50,0 % |
| SEQ ID-Nr. 8 | 100 % | 62,5 % | 44,4 % | 57,1 % | 80,0 % | 62,5 % |

| | | | | | | |
|---|---|---|---|---|---|---|
| SEQ ID-Nr. 9 | 100 % | 12,5 % | 44,4 % | 42,9 % | 60,0 % | 62,5 % |
| SEQ ID-Nr. 10 | 100 % | 62,5 % | 55,6 % | 71,4 % | 80,0 % | 50,0 % |
| SEQ ID-Nr. 11 | 100 % | 50,0 % | 55,6 % | 57,1 % | 80,0 % | 50,0 % |
| SEQ ID-Nr. 12 | 100 % | 12,5 % | 22,2 % | 57,1 % | 80,0 % | 52,5 % |
| SEQ ID-Nr. 13 | 100 % | 50,0 % | 44,4 % | 57,1 % | 80,0 % | 75,0 % |
| SEQ ID-Nr. 14 | 0 % | 8,3 % | 0 % | 14,3 % | 0 % | 25,0 % |
| SEQ ID-Nr. 15 | 100 % | 87,5 % | 100 % | 85,7 % | 100 % | 75,0 % |

| | Sequenzidentität relativ zu der SEQ ID-Nr. 1 | | |
|---|---|---|---|
| Polypeptid | Abschnitt M | Abschnitt N | Abschnitt O |
| SEQ ID-Nr. 1 | 100 % | 100 % | 100 % |
| SEQ ID-Nr. 2 | 87,5 % | 80,0 % | 81,8 % |
| SEQ ID-Nr. 3 | 87,0 % | 80,0 % | 81,8 % |
| SEQ ID-Nr. 4 | 87,5 % | 80,0 % | 81,8 % |
| SEQ ID-Nr. 5 | 87,5 % | 80,0 % | 81,8 % |
| SEQ ID-Nr. 6 | 87,5 % | 80,0 % | 72,7 % |
| SEQ ID-Nr. 7 | 75,0 % | 40,0 % | 36,4 % |
| SEQ ID-Nr. 8 | 75,0 % | 60,0 % | 54,5 % |
| SEQ ID-Nr. 9 | 62,5 % | 40,0 % | 54,5 % |
| SEQ ID-Nr. 10 | 62,5 % | 40,0 % | 54,5 % |
| SEQ ID-Nr. 11 | 75,0 % | 40,0 % | 54,5 % |
| SEQ ID-Nr. 12 | 100 % | 40,0 % | 54,5 % |
| SEQ ID-Nr. 13 | 50,0 % | 40,0 % | 63,6 % |
| SEQ ID-Nr. 14 | 6,2 % | 0 % | 0 % |
| SEQ ID-Nr. 15 | 87,5 % | 80,0 % | 63,6 % |

Beispiel 3: Hydrolyse von ZEN durch Polypeptide in Zelllysaten

Zur Bestimmung ihrer Fähigkeit, ZEN in die nicht bzw. weniger toxischen Metabolite HZEN und DHZEN abzubauen, wurde das Polypeptid mit der Sequenz ID-Nr. 1, codiert durch die Nukleotidsequenz mit der Sequenz ID-Nr. 17, als solcher und mit einem C- bzw. N- terminalen 6xHis Tag in *E. coli,* wie in Beispiel 1 beschrieben, hergestellt. Die Polypeptide mit den Aminosäuresequenzen mit den Sequenz ID-Nrn. 7, 8, 14 und 15, welche durch die Nukleotidsequenzen mit den Sequenz ID-Nrn. 23, 24, 30 und 31 codiert wurden, wurden ausschließlich am C-terminalen Ende 6xHis markiert. Jeweils 100 ml einer *E. coli* Kultur mit einer optischen Dichte (OD600 nm) von 2,0 - 2,5 wurden durch Zentrifugation bei 4 °C geerntet und in 20 ml Brunner Mineralmedium (DSMZ microorganisms medium number 462, 2012) resuspendiert. Die Zellsuspensionen wurden durch 3-malige Behandlung mit einer Frensh Press bei 20.000 psi lysiert. Die so erhaltenen Zelllysate wurden in einer 1:10, einer 1:100 oder einer 1:1.000 Verdünnung, die in Brunner

Mineralmedium inklusive 0,1 mg/ml BSA (Rinderserum Albumin) hergestellt wurde, eingesetzt. Für die ZEN-Abbauversuche wurden 9,9 ml Brunner Mineralmedium inklusive 0,1 mg/ml BSA, 0,1 ml verdünntes Zelllysat und 31 μl ZEN-Substratstammlösung eingesetzt. In Summe wurden die Zelllysate somit 1:1.000, 1:10.000 bzw. 1:100.000 verdünnt. Als ZEN-Substratstammlösung diente eine 2,08 mM ZEN-Lösung (40 Vol.-% ACN + 60 Vol.-% $H_2O$). Zur Herstellung dieser Lösung wurde ZEN in kristalliner Form (Biopure Standard von Romer Labs, Art. Nr. 001109, Reinheit mind. 98 %) entsprechend eingewogen und aufgelöst. Jeder Abbauansatz wurde in 25 ml Glasfläschchen durchgeführt und bei 25 °C unter Schütteln mit 100 Upm für insgesamt 120 h inkubiert. Zu den Zeitpunkten 0; 0,5; 1; 2; 5; 24; 47; 72 und 120 h wurden jeweils eine Probe von 1 ml entnommen, die Polypeptide wurden für 10 min bei 99 °C hitzeinaktiviert und bei -20 °C gelagert. Nach dem Auftauen der Proben wurden die nicht löslichen Bestandteile durch Zentrifugation abgetrennt. ZEN, HZEN und DHZEN wurden mittels LC/MS/MS analysiert. Dazu wurden die Metabolite mittels einer Phenomenex Luna C18(2) Säule mit den Dimensionen 250 mm x 3 mm und einer Partikelgröße von 5 μm chromatographisch getrennt. Als Laufmittel diente ein Acetonitril-Wassergemisch mit einer Ameisensäurekonzentration von 1 ml/l. Das UV-Signal bei 270 nm wurde aufgezeichnet. Als Ionisierungsquelle diente Elektrospray Ionisation (ESI). ZEN, HZEN und DHZEN wurden mittels QTrap/LC/MS/MS (triple quadrupole, Applied Biosystems) im "enhanced Modus" quantifiziert. Nach spätestens 24 Stunden konnten in keinem der Ansätze mehr wesentliche Mengen von ZEN nachgewiesen werden. Der Großteil, über 80 %, von ZEN wurde in HZEN oder DHZEN umgewandelt.

In Fig. 1, die nicht Teil der Erfindung ist, ist der zeitliche Abbau von ZEN und die Zunahme von HZEN sowie DHZEN beispielhaft für eine 1:10.000 verdünnte Zelllysatlösung für nicht getaggtes (Fig. 1A) als auch für C-terminal 6xHis getaggtes (Fig. 1B) und N-terminal 6xHis getaggtes (Fig. 1C) Polypeptid mit der SEQ ID-Nr. 1 zu sehen. Daraus ist klar ersichtlich, dass 1. die Umsetzung von ZEN unmittelbar und vollständig erfolgt, da bereits in der ersten Probe (0 h), die unmittelbar nach dem Versuchsstart gezogen wurden, nahezu kein ZEN mehr detektiert werden konnte und es 2. durch das Anfügen eines Tags, C- oder N- terminal, zu keinen nennenswerten Aktivitätsverlusten kam.

Beispiel 4: Hydrolyse von ZEN Derivaten durch Polypeptide in Zelllysaten

Zur Bestimmung der Fähigkeit der Polypeptide neben ZEN auch ZEN-Derivate in nicht bzw. weniger toxische Metabolite zu transformieren, wurden die Polypeptide mit den Sequenz ID-Nrn. 1, 7, 8, 14 und 15, wie in Beispiel 3 beschrieben mit C-terminalem His-Tag hergestellt und als Zelllysate im Abbau wurden die jeweiligen synthetischen Nukleotidsequenzen mit den Sequenzen mit der Sequenz ID-Nr. 17, 23, 24, 30 und 31 herangezogen.

Die Abbauversuche wurden wie in Beispiel 3 beschrieben durchgeführt, wobei jedes Polypeptid mit jedem ZEN-Derivat gewählt aus der Gruppe α-ZEL, β-ZEL, α-ZAL, β-ZAL, Z14G, Z14S und ZAN, getestet wurde. Die Zelllysate wurden in einer Gesamtverdünnung von 1:10.000 eingesetzt. Als Substratstammlösung wurde anstatt einer 2,08 mM ZEN-Lösung (40 Vol.-% ACN + 60 Vol-.% $H_2O$), äquimolare, d.h. 2,08 mM Lösungen der ZEN-Derivate eingesetzt. α-ZEL, β-ZEL, α-ZAL, β-ZAL und ZAN wurden von Sigma bezogen und als Standards für die Analyse eingesetzt. Z14G und Z14S wurden mit einer Reinheit von wenigstens 90 %, nach den Verfahren wie in P. Krenn et al., 2007 (Mykotoxin Research, 23, 4, 180-184) und M. Sulyok et al., 2007(Anal. Bioanal. Chem., 289, 1505-1523) beschrieben, hergestellt und als Standards für die Analyse eingesetzt. Ein weiterer Unterschied zu Beispiel 3 ist, dass lediglich eine Probe, nämlich nach 24 h, genommen wurde. Die Reduktion der Konzentration der ZEN Derivate während des Abbauversuches wurde mittels LC/MS/MS quantifiziert. α-ZEL, β-ZEL, Z14G und Z14S wurden nach der Methode von M. Sulyok et al. (2010, Food Chemistry, 119, 408-416); α-ZAL, β-ZAL und ZAN wurden nach der Methode von P. Songsermaskul et al. (2011, J. of Animal Physiol. and Animal Nutr., 97, 155-161) gemessen. Überraschenderweise hat sich herausgestellt, dass in allen Abbauversuchen nach 24 h Inkubation, nur noch 0 bis maximal 13 % der Ausgangsmengen der ZEN Derivate vorhanden waren.

Beispiel 5: Spezifische Aktivität sowie enzymkinetische Parameter der Polypeptide sowie von Varianten davon

Die Bestimmung der spezifischen Aktivität der Polypeptide sowie von Varianten davon erfolgte photometrisch, wobei alle eingesetzten Polypeptide einen C-terminalen 6xHis Tag aufwiesen. Die Herstellung, Anreicherung und Reinigung der Polypeptide bzw. von Varianten davon erfolgte wie in Beispiel 1 beschrieben. Der Abbau von ZEN zu HZEN wurde über die Abnahme der Adsorption bei der Wellenlänge von 315 nm gemessen. Die molaren Extinktionskoeffizienten [ε] von ZEN und HZEN wurden experimentell bestimmt und betragen 0,0078895 L $\mu mol^{-1}$ $cm^{-1}$ und 0,0030857 L $\mu mol^{-1}$ $cm^{-1}$. Die Extinktionskoeffizienten sind stark pH-abhängig und daher muss die Aktivitätsmessung immer bei exakt demselben pH-Wert und vorzugsweise auch in derselben Matrix durchgeführt werden. Die Messungen wurden in einer 50 mM Tris-HCl pH = 8,2 Pufferlösung in Quarzküvetten in einem Wellenlängenbereich von 200 bis 2500 nm in einem UV-VIS Photometer (Hitachi U-2001) bei 32 °C durchgeführt.

Als ZEN-Substratstammlösung diente eine 2,08 mM ZEN-Lösung (40 vol-% ACN + 60 vol-% $H_2O$). Zur Herstellung dieser Lösung wurde ZEN in kristalliner Form (Biopure Standard von Romer Labs, Art. Nr. 001109, Reinheit mind. 98 %) entsprechend eingewogen und aufgelöst. Die ZEN-Substratverdünnungen (0,79 μM, 1,57 μM, 2,36 μM,

3,14 µM, 4,71 µM, 6,28 µM, 7,85 µM, 9,42 µM, 10,99 µM, 12,56 µM, 14,13 µM, 15,71 µM, 17,28 µM, 18,85 µM) wurden mit 50 mM Tris-HCl pH = 8,2 hergestellt. Die Polypeptidlösungen wurden mit 50 mM Tris-HCl Puffer pH = 8,2 auf eine Endkonzentration von ca. 70 ng/ml verdünnt. Die ZEN-Substratverdünnungen wurden im Wasserbad auf 32 °C vorgewärmt.

100 µl der jeweiligen ZEN-Substratverdünnung wurden mit 0,2 µl Polypeptidlösung versetzt und die Absorption wurde für 5 min gemessen, wobei jede Kombination aus Polypeptidlösung - ZEN-Substratverdünnung mindestens zweifach gemessen wurde.

Unter Berücksichtigung der Extinktionskoeffizienten von ZEN und HZEN wurde über die Steigung der Absorption im zeitlichen Verlauf die Reaktionsgeschwindigkeit für jede Substratkonzentration errechnet.

Die Bezeichnungen "$K_M$ - Wert" oder "Michaelis-Menten Konstante" beziehen sich auf einen Parameter zu Beschreibung der enzymatischen Affinität, der die Einheiten [µM] oder [mM] besitzt und mit Hilfe des linearen Hanes Plots nach H. Bisswang (2002, Enzyme Kinetics, ISBN 3-527-30343-X, Seite 19) berechnet wird, wobei hierfür bevorzugt die Funktion "Enzymkinetik, Single Substrat" des Programms SigmaPlot 12.0 verwendet wird. Die Bezeichnungen "katalytische Konstante der Enzymreaktion" oder der "$k_{cat}$-Wert" beziehen sich auf einen Parameter zur Beschreibung der Umsatzrate eines Polypeptids bzw. Enzyms, der in $[s]^{-1}$ angegeben ist und bevorzugt mit Hilfe der Funktion "Enzymkinetik, Single Substrat" des Programms SigmaPlot 12.0 berechnet wird. Die "maximale Enzymgeschwindigkeit" oder der "$v_{max}$-Wert" wird in den Einheiten [µM/s] oder [mM/s] angegeben und analog zum $K_M$-Wert mit Hilfe des linearen Hanes Plot ermittelt, wobei hierfür bevorzugt die Funktion "Enzymkinetik, Single Substrat" des Programms SigmaPlot 12.0 verwendet wird.

Mittels $v_{max}$ und der eingesetzten Enzymkonzentration wurde die spezifische Aktivität gemäß der Formel

$$\text{spezifische Aktivität [U/mg]} = \frac{\text{vmax [µM/s] x 60 [s/min]}}{\text{Enzymkonzentration [mg/l]}}$$

berechnet, wobei eine Unit als Hydrolyse von 1 µmol ZEN pro Minute bei 32 °C in 50 mM Tris-HCl-Pufferlösung, pH = 8,2 definiert ist.

Nachfolgend sind die Rohdaten für die Bestimmung der Enzymparameter $K_M$, $v_{max}$, $k_{cat}$ sowie der spezifischen Aktivität beispielhaft für das Polypeptid mit der Sequenz ID-Nr: 1 angeführt. Tabelle 3 zeigt die Reaktionsgeschwindigkeiten bei den jeweiligen ZEN-Substratkonzentrationen, Fig. 2 die zugehörigen Michaelis-Menton Graphiken und in Tabelle 4 sind die entsprechenden enzymkinetischen Parameter angegeben. Die eingesetzte Enzymlösung hatte eine Konzentration von 68 ng/l.

Tabelle 3: Reaktionsgeschwindigkeiten des Polypeptids mit der SEQ ID-Nr: 1 bei unterschiedlichen ZEN-Konzentrationen.

| ZEN-Substratverdünnung [µM] | Messung 1 Reaktionsgeschwindigkeit [µM/s] | Messung 2 Reaktionsgeschwindigkeit [µM/s] |
|---|---|---|
| 0,79 | 0,0073 | 0,0071 |
| 1,57 | 0,0087 | 0,0082 |
| 2,36 | 0,0095 | 0,0080 |
| 3,14 | 0,0101 | 0,0073 |
| 4,71 | 0,0103 | 0,0087 |
| 6,28 | 0,0096 | 0,0088 |
| 7,85 | 0,0084 | 0,0088 |
| 9,42 | 0,0111 | 0,0087 |
| 10,99 | 0,0093 | 0,0081 |
| 12,56 | 0,0100 | 0,0086 |
| 14,13 | 0,0089 | 0,0101 |
| 15,71 | 0,0089 | 0,0090 |
| 17,28 | 0,0100 | 0,0074 |
| 18,85 | 0,0100 | 0,0085 |

Tabelle 4: Enzymkinetische Parameter des Polypeptids mit der Sequenz ID-Nr. 1.

| | $V_{max}$ [µM / s] | | $K_M$ [µM] | | $k_{cat}$ [$s^{-1}$] | | sp. Aktivität [U/mg] | |
|---|---|---|---|---|---|---|---|---|
| Messung | 1 | 2 | 1 | 2 | 1 | 2 | 1 | 2 |
| Wert | 0,00993 | 0,008756 | 0,2172 | 0,1898 | 5,44 | 4,79 | 8,76 | 7,73 |
| Mittelwert | 0,009343 | | 0,2035 | | 5,12 | | 8,25 | |

Die spezifische Aktivitäten der untersuchten Polypeptide beträgt für Sequenz ID-Nr. 1 8,25 U/mg, für Sequenz ID-Nr. 7 3,83 U/mg, für Sequenz ID-Nr. 8 2,57 U/mg, für Sequenz ID-Nr. 14 3,33 U/mg und 7,76 U/mg für Sequenz ID-Nr. 15.

Beispiel 6: Abbau von ZEN und ZEN-Derivaten in kontaminiertem Mais

Zur Bestimmung der Fähigkeit der Polypeptide natürlich vorkommendes ZEN und ZEN-Derivate in einer komplexen Matrix und bei niedrigem pH-Wert abzubauen, wurde kontaminierter Mais beispielhaft mit unterschiedlichen Konzentrationen von jeweils einem der Polypeptide mit der Sequenz ID-Nr. 1 bis 6 versetzt und der Abbau von ZEN und ZEN - Derivaten verfolgt.

Der kontaminierte Mais wurde gemahlen und im Abbauversuch eingesetzt, wobei ein Ansatz aus 1 g gemahlenem, kontaminiertem Mais, 8,9 ml 100 mM Acetatpuffer pH = 4,0 und 0,1 ml Polypeptidlösung bestand. Es wurden angereicherte und gereinigte Polypeptidlösungen, wie in Beispiel 5 beschrieben, hergestellt, wobei diese auf eine Konzentration von 10 mU/ml, 100 mU/ml bzw. 1.000 mU/ml verdünnt wurden. Im Ansatz wurden somit absolut 1 mU (= 1 mU pro Gramm Mais), 10 mU (= 10 mU pro Gramm Mais) bzw. 100 mU (= 100 mU pro Gramm Mais) eingesetzt. Jeder Abbauansatz wurde in 25 ml durchgeführt und bei 37 °C unter Schütteln mit 100 Upm inkubiert. Vor der Enzymzugabe bzw. nach 1 stündiger Inkubation wurde jeweils eine Probe von 1 ml entnommen, das Polypeptid für 10 min bei 99 °C hitzeinaktiviert und die Probe bei -20 °C gelagert. Nach dem Auftauen der Probe wurden die nicht löslichen Bestandteile durch Zentrifugieren abgetrennt. Konzentrationen an ZEN sowie an ZEN-Derivaten wurden mittels LC/MS/MS, wie in M. Sulyok et al. (2007, Anal. Bioanal. Chem., 289, 1505-1523) beschrieben, gemessen. Der Gehalt an ZEN und ZEN-Derivaten in diesem Mais betrug für ZEN 238 ppb, für α-ZEL 15 ppb, für β-ZEL 23 ppb, für Z14G 32 ppb und für Z14S 81 ppb. In Tabelle 5 ist die prozentuale Abnahme des Gehalts an ZEN und an ZEN-Derivaten im Abbauversuch dargestellt.

Tabelle 5: Reduktion an ZEN und ZEN-Derivaten in Prozent bezogen auf den Startgehalt des Abbauversuches von unterschiedlichen Polypeptiden und Polypeptidmengen.

| Polypeptid | Menge im Ansatz | ZEN | α-ZEL | β-ZEL | Z14G | Z14S |
|---|---|---|---|---|---|---|
| SEQ ID Nr: 1 | 0,1 mU | 83% | ≥ 80% | 70% | 78% | 80 % |
| | 1 mU | 96% | ≥ 80 % | 76 % | ≥ 80% | 92% |
| | 10 mU | 97% | ≥ 80% | ≥ 85% | ≥ 80 % | 94% |
| SEQ ID Nr. 2 | 0,1 mU | 87% | ≥ 80% | 73% | ≥ 80% | 84% |
| | 1 mU | 97% | ≥ 80 % | 78 % | ≥ 80% | 90% |
| | 10 mU | 99% | ≥ 80% | ≥ 85 % | ≥ 80% | 96% |
| SEQ ID Nr. 3 | 0,1 mU | 79% | 79% | 67% | 73% | 75% |
| | 1 mU | 85 % | ≥ 80% | 72 % | 79% | 82 % |
| | 10 mU | 92% | ≥ 80% | 78% | ≥ 80% | 88% |
| SEQ ID Nr. 4 | 0,1 mU | 82% | 78% | 65 % | 76 % | 80 % |
| | 1 mU | 89 % | ≥ 80% | 73 % | ≥ 80 % | 86 % |
| | 10 mU | 93% | ≥ 80% | 82 % | ≥ 80% | 91 % |

(fortgesetzt)

| Polypeptid | Menge im Ansatz | ZEN | α-ZEL | β-ZEL | Z14G | Z14S |
|---|---|---|---|---|---|---|
| SEQ ID Nr. 5 | 0,1 mU | 79% | 76 % | 66 % | 78 % | 80 % |
| | 1 mU | 83 % | ≥ 80% | 73 % | ≥ 80% | 81 % |
| | 10 mU | 91 % | ≥ 80% | 79 % | ≥ 80 % | 86% |
| SEQ ID Nr. 6 | 0,1 mU | 93 % | ≥ 80% | 75 % | ≥ 80 % | 90% |
| | 1 mU | 95 % | ≥ 80% | 82 % | ≥ 80% | 92 % |
| | 10 mU | 98 % | ≥ 80 % | ≥ 85% | ≥ 80 % | 96 % |

Beispiel 7: Polypeptid enthaltende Zusatzstoffe zur hydrolytischen Spaltung von ZEN und/oder ZEN-Derivaten

Zur Herstellung von Zusatzstoffen zur hydrolytischen Spaltung von ZEN wurden beispielhaft Fermentationsüberstände von mittels *P. pastoris* exprimierten Polypeptiden mit den Sequenz ID Nrn. 1, 2, 6 und 13 mittels Mikrofiltration und Ultrafiltration (Ausschlussgrenze: 10 kDa) unter Standardbedingungen gereinigt und bis zu einer Trockensubstanzkonzentration von etwa 9 Gew.-% konzentriert. Im Anschluss wurden diese polypeptidhältigen Lösungen mit einem Sprühtrockner, (Mini B290 von Büchi) ebenfalls unter Standardbedingungen zu trockenen Pulvern weiterverarbeitet. Diese vier Pulver werden in weiterer Folge als Z1, Z2, Z6 und Z13 bezeichnet. Z1, Z2, Z6 bzw. Z13 wurden des Weiteren mit Bentonit mit einer durchschnittlichen Korngröße ca. 1 μm, in einem Verhältnis von 1 Gew.-% Zusatzstoff Z1, Z2, Z6 bzw. Z13 und 99 Gew.-% Bentonit in einem Überkopfschüttler, gemischt. Die so enthaltenen Zusatzstoffe werden als Zusatzstoff Z1.B, Z2.B, Z6.B und Z13.B bezeichnet. Des Weiteren wurden Z1, Z2, Z6 und Z13 mit Bentonit und einem Vitamin-Spurenelemente Konzentrat in einem Verhältnis von 0,1 Gew.-% Zusatzstoff Z1, Z2, Z6 bzw. Z13, 0,9 Gew.-% Vitamin-Spurenelemente Konzentrat und 99 Gew.-% Bentonit in einem Überkopfschüttler, gemischt. Die so enthaltenen Zusatzstoffe werden als Zusatzstoff Z1.BVS, Z2.BVS, Z6.BVS und Z13.BVS bezeichnet. 100 g der Zusatzstoffe Z1.BVS, Z2.BVS, Z6.BVS und Z13.BVS enthielten 200 mg Eisensulfat, 50 mg Kupfersulfat, 130 mg Zinnoxid, 130 mg Manganoxid, 2,55 mg Kalzimcarbonat, 160 mg Vitamin E, 6,5 mg Vitamin K3, 6,5 mg Vitamin B1, 14 mg Vitamin B2, 15 mg Vitamin B6, 0,15 mg Vitamin B12, 150 mg Nicotinsäure, 30 mg Pantothensäure und 5,3 mg Folsäure.

Die Zusatzstoffe wurden in einem 50 mMTris-HCl Puffer pH = 8,2 für 30 Minuten extrahiert und im selben Puffer so weiterverdünnt, dass die Endkonzentration an Polypeptid bei ca. 70 ng/ml lag.

Im Anschluss wurde die Zearalenon abbauende Wirkung dieser Lösungen, wie in Beispiel 5 beschrieben, bestimmt. Die entsprechenden Aktivitäten waren für Z1 8.230 U/g, für Z2 9.310 U/g, für Z6 9.214 U/g, für Z1.B 83 U/g, für Z2.B 92 U/g, für Z2.C 90 U/g, für Z13.B 57 U/g, für Z1.BVS 8 U/g, für Z2.BVS 9 U/g, für Z6.BVS 9 U/g und für Z13.BVS 6 U/g.

Die Fähigkeit zum Abbau der ZEN-Derivate α-ZEL, β-ZEL, α-ZAL, β-ZAL, Z14G, Z14S und ZAN durch die Zusatzstoffe Z1, Z2, Z6, Z13, Z1.B, Z2.B, Z6.B, Z13.B, Z1.BVS, Z2.BVS, Z6.BVS und Z13.BVS, wurde, wie in Beispiel 4 beschrieben, durchgeführt, jedoch wurden anstatt 100 μl eines Zelllysats 100 μl einer Polypeptidlösung mit einer Polypeptidkonzentration von ca. 70 ng/ml eingesetzt. Nach 6 stündiger Inkubation lagen nur noch maximal 15 % der Ausgangsmenge als nicht hydrolysiertes ZEN-Derivat vor.

Beispiel 8: Temperaturoptimum

Zur Bestimmung des Temperaturoptimums der Polypeptide mit den SEQ ID-Nrn. 1, 7 und 15 wurden diese mit einem C-terminalen 6xHis-Tag wie in Beispiel 1 beschriebenen kloniert, in E. coli exprimiert und aufgereinigt. In Vorversuchen wurde für jedes Polypeptid jene Konzentration ermittelt bei der unter den Versuchsbedingungen (Teorell Stenhagen Puffer (Teorell und Stenhagen, Ein Universalpuffer für den pH-Bereich 2.0 bis 12.0. Biochem Ztschrft, 1938, 299: S. 416-419), pH 7.5, mit 0.1 mg/ml BSA bei 30°C) nach einer Versuchsdauer von 3 h eine vollständige Umsetzung von ZEN gewährleistet werden konnte. Die Präparationen wurden in den ermittelten Konzentrationen in die Abbauansätze zur Bestimmung des Temperaturoptimums eingesetzt. Die Versuche durchgeführt wurden in einem PCR Cycler (Eppendorf) unter Verwendung der Temperaturgradienten-Funktion bei 20°C +/- 10°C, bei 40°C +/- 10°C und falls erforderlich bei 60°C +/-10°C (10 Temperaturen im jeweiligen Bereich; Temperaturen vom PCR Cycler vordefiniert). Für die Ansätze wurde Teorell-Stenhagen Puffer beim jeweiligen pH Optimum mit der entsprechenden Enzymkonzentration sowie 0,1 mg/ml BSA und 5 ppm ZEN versetzt. Als Negativkontrollen dienten Ansätze mit 0,1 mg/ml BSA und 5 ppm ZEN ohne Enzymzugabe. Nach 0 h, 0,5 h, 1 h, 2 h und 3 h Inkubationszeit wurde jeweils eine Probe pro Inkubationstemperatur gezogen, für 10 min bei 99°C hitzeinaktiviert und bei -20°C gelagert. Nach dem Auftauen wurden die Proben in HPLC-Vials überführt. ZEN, HZEN und DHZEN wurden mittels HPLC-DAD analysiert. Dazu wurden die Metabolite mittels einer Zorbax SB-Aq C18 Säule mit den Dimensionen 4.6 mm x 150 mm und einer Partikelgröße von 5 μm chromatographisch getrennt. Als Laufmittel diente ein Methanol-Wassergemisch mit 5 mM Ammoniumacetat. Das UV-Signal bei 274 nm wurde aufgezeichnet. Die Quantifizierung der Metabolite erfolgte unter Einbeziehung mitgeführter Standardreihen. Die Ermittlung der Temperaturoptima erfolgte über die ermittelten Steigungen der Abbaukurven, wobei die Temperatur, bei der die Steigung am größten war als das Temperaturoptimum definiert wurde. Die Temperaturoptima sind in Tabelle 6 gezeigt.

Tabelle 6: Temperaturoptima der Polypeptide

| SEQ ID-Nr. 1 | SEQ ID-Nr. 7 | SEQ ID-Nr. 15 |
|---|---|---|
| 38°C | 31°C | 41°C |

Beispiel 9: Temperaturstabilität

Zur Bestimmung der Temperaturstabilität der Polypeptide mit den SEQ ID-Nrn. 1, 7 und 15 wurden diese mit einem C-terminalen 6xHis-Tag wie in Beispiel 1 beschriebenen kloniert, in E. coli exprimiert und aufgereinigt. Diese wurden im PCR Cycler mit Gradientenfunktion beim jeweiligen Temperaturoptimum +/- 10°C inkubiert. Nach 0 min, 15 min, 30 min und 60 min wurde pro Ansatz und pro Temperatur eine Probe gezogen. Diese vorinkubierten Proben wurden im Anschluss in einem Abbauversuch in Teorell-Stenhagen Puffer beim jeweiligen pH-Optimum mit 0,1 mg/ml BSA und 5 ppm ZEN im Ansatz eingesetzt. In Vorversuchen wurde für jedes Polypeptid jene Konzentration ermittelt bei der unter den Versuchsbedingungen (Teorell Stenhagen Puffer, pH 7.5, mit 0.1 mg/ml BSA bei 30°C) nach einer Versuchsdauer von 3 h eine vollständigen Umsetzung von ZEN gewährleistet werden konnte. Die jeweils ermittelte Enzymkonzentration wurde in den Ansätzen eingesetzt. Die Abbauansätze wurden bei 30 °C inkubiert. Probenahmen erfolgten nach 0 h, 0,5 h, 1 h, 2 h und 3 h Inkubationszeit. Im Anschluss wurden die Polypeptide bei 99°C für 10 min hitzeinaktiviert und die Proben bei -20°C gelagert. Nach dem Auftauen wurden die Proben in HPLC-Vials überführt und mittels HPLC-DAD wie in Beispiel 8 beschrieben analysiert.

Die Temperaturstabilität ist definiert als die Temperatur, bei der die Polypeptide nach 15-minütiger Vorinkubation eine 50 %ige Restaktivität verglichen mit dem Temperaturoptimum besitzen. Als Maß für die Aktivität wird die Steigung der Abbaukurven herangezogen. Die Temperaturstabilitäten sind in Tabelle 7 gezeigt.

Tabelle 7: Temperaturstabilitäten der Polypeptide (50% Restaktivität nach 15- minütiger Vorinkubation)

| SEQ ID-Nr. 1 | SEQ ID-Nr. 7 | SEQ ID-Nr. 15 |
|---|---|---|
| 38°C | 28°C | 49°C |

Beispiel 10: pH-Optimum

Zur Bestimmung des pH-Optimums der Polypeptide mit den SEQ ID-Nrn. 1, 7 und 15 wurden diese mit einem C-terminalen 6xHis-Tag wie in Beispiel 1 beschriebenen kloniert, in *E. coli* exprimiert und aufgereinigt. In Vorversuchen wurde für jedes Polypeptid jene Konzentration ermittelt bei der unter den Versuchsbedingungen (Teorell Stenhagen Puffer, pH 7.5, mit 0.1 mg/ml BSA bei 30°C) nach einer Versuchsdauer von 3 h eine vollständigen Umsetzung von ZEN gewährleistet werden konnte. Die jeweils Enzymkonzentration wurde in den Ansätzen eingesetzt. Die Abbauansätze wurden in Teorell-Stenhagen Puffer bei pH-Werten von 3,0, 4,0, 5,0, 5,5, 6,0, 6,5, 7,0, 7,5, 8,0, 8,5, 9,0, 9,5, 10,0, 11,0 und 12,0 durchgeführt. Für die Die Abbauansätze mit 0,1 mg/ml BSA und 5 ppm ZEN wurden bei 30°C inkubiert. Als Negativkontrollen dienten Ansätze in Teorell-Stenhagen Puffer bei pH 3,0, pH 7,0 und pH 12,0 mit 0,1 mg/ml BSA und 5 ppm ZEN. Probenahmen erfolgten nach 0 h, 0,5 h, 1 h, 2 h und 3 h Inkubationszeit. Im Anschluss wurden die Polypeptide bei 99°C für 10 min hitzeinaktiviert und die Proben bei -20°C gelagert. Nach dem Auftauen wurden die Proben in HPLC-Vials überführt und mittels HPLC-DAD wie in Beispiel 8 beschrieben analysiert. Die Ermittlung der pH-Optima erfolgte über die ermittelten Steigungen der Abbaukurven, wobei der pH-Wert, bei dem die Steigung am größten war als pH-Optimum definiert wurde. Die pH-Optima sind in Tabelle 8 gezeigt.

Tabelle 8: pH Optima der Polypeptide

| SEQ ID-Nr. 1 | SEQ ID-Nr. 7 | SEQ ID-Nr. 15 |
|---|---|---|
| 8,2 | 7,5-8,5 | 7,5 |

Beispiel 11: pH-Stabilität bei pH 5,0

Zur Bestimmung der pH-Stabilität wurden die Polypeptide aus Beispiel 10 in Teorell-Stenhagen Puffer bei pH 5,0 und dem jeweiligen pH-Optimum für 1 h bei 25°C inkubiert. Diese vorinkubierten Proben wurden in einen Abbauversuch in den gleichen Konzentrationen des jeweiligen Polypeptides wie sie zur Bestimmung des pH-Optimums verwendet wurden in 100 mM Tris-HCl-Puffer beim jeweiligen pH-Optimum mit 0,1 mg/ml BSA und 5 ppm ZEN im Ansatz eingesetzt. Die Ansätze wurden beim jeweiligen Temperaturoptimum inkubiert. Probenahmen erfolgten nach 0 h, 0,5 h, 1 h, 2 h und 3 h Inkubationszeit. Im Anschluss wurden die Polypeptide bei 99°C für 10 min hitzeinaktiviert und die Proben bei -20°C gelagert. Nach dem Auftauen wurden die Proben in HPLC-Vials überführt und mittels HPLC-DAD wie in Beispiel 8 beschrieben analysiert. Die pH Stabilität ist definiert als die prozentuale Restaktivität der Polypeptide bei pH 5,0 relativ zu der Aktivität beim jeweiligen pH Optimum. Die pH Stabilitäten für pH 5,0 sind in Tabelle 9 gezeigt.

Tabelle 9: pH Stabilität der Polypeptide bei pH 5,0

| SEQ ID-Nr. 1 | SEQ ID-Nr. 7 | SEQ ID-Nr. 15 |
|---|---|---|
| 3% | 100 % | 19% |

Beispiel 12: ZEN-Abbauversuch

Der Abbau von ZEN zu HZEN und DHZEN wurde beispielhaft für die Polypeptide mit den Sequenz ID-Nrn. 1, 7 und 15 durchgeführt. Die Abbauansätze wurden in Teorell Stenhagen Puffer, pH 7.5, mit 0.1 mg/ml BSA und 5 ppm ZEN durchgeführt. Die Abbauansätze wurden bei 30 °C inkubiert. Probenahmen erfolgten nach 0 h, 0,5 h, 1 h, 2 h und 3 h Inkubationszeit. Im Anschluss wurden die Polypeptide bei 99°C für 10 min hitzeinaktiviert und die Proben bei -20°C gelagert. Nach dem Auftauen wurden die Proben in HPLC-Vials überführt und mittels HPLC-DAD wie in Beispiel 8 beschrieben analysiert. Die Polypeptidkonzentration wurde so gewählt, dass ein vollständiger Abbau nach ca. 3 Stunden erreicht wurde. Die Abbaukinetiken sind in Fig. 3 dargestellt, wobei die y-Achse die Konzentration von ZEN, HZEN und DHZEN in Mikromol pro Liter (μmol/l) und die x-Achse die Inkubationszeit in Stunden (h) zeigt.
* μM bedeutet mikromolar und entspricht der Einheit μMol/l

**Patentansprüche**

1. Verfahren zur hydrolytischen Spaltung von Zearalenon und/oder wenigstens einem Zearalenon Derivat mittels eines hydrolytisch spaltenden Polypeptids, **dadurch gekennzeichnet, dass** Zearalenon und/oder wenigstens ein Zearalenon Derivat durch das Polypeptid, das eine Hydrolase mit einer Aminosäuresequenz gewählt aus der Gruppe der Sequenz ID-Nrn. 7, 8, 14 und 15, insbesondere der Sequenz ID Nr. 14 oder eine funktionelle Variante davon ist, hydrolysiert wird, wobei eine Sequenzidentität zwischen der funktionellen Variante und wenigstens einer der Aminosäuresequenzen wenigstens 70 % beträgt.

2. Verfahren nach Anspruch 1, **dadurch gekennzeichnet, dass** das Polypeptid wenigstens einen konservierten Aminosäuresequenzabschnitt oder eine funktionelle Variante davon enthält, wobei die funktionelle Variante des Aminosäuresequenzabschnitts eine Sequenzidentität von wenigstens 70 %, bevorzugt wenigstens 84 %, noch bevorzugter wenigstens 92 % und am bevorzugtesten wenigstens 98 % aufweist und der wenigstens eine konservierte Aminosäuresequenzabschnitt aus der Gruppe der Aminosäuresequenzen +24 bis +50, +52 bis +77 +79 bis +87, +89 bis +145, +150 bis +171, +177 bis +193, +223 bis +228, +230 bis +237, +239 bis +247, +249 bis +255, +257 bis +261, +263 bis +270, +272 bis +279, +297 bis +301 der Sequenz mit der Sequenz ID-Nr. 1 gewählt wird.

3. Verfahren nach Anspruch 1 oder 2, **dadurch gekennzeichnet, dass** das Polypeptid wenigstens eine Mutation der Aminosäuresequenz in Bezug auf Sequenz ID-Nr. 1 an wenigstens einer der folgenden Positionen gewählt aus der Gruppe: 22, 23, 25, 26, 27, 29, 31, 32, 35, 37, 42, 43, 46, 51, 53, 54, 57, 60, 69, 72, 73, 78, 80, 84, 88, 95, 97, 99, 114, 118, 119, 123, 132, 141, 146, 148, 149, 154, 163, 164, 165, 169, 170, 172, 176, 180, 182, 183, 190, 191, 194, 196, 197, 198, 201, 204, 205, 206, 207, 208, 209, 210, 212, 213, 214, 216, 217, 220, 221, 222, 229, 231, 233, 238, 240, 244, 245, 246, 248, 249, 251, 254, 256, 260, 262, 263, 266, 269, 271, 277, 280, 281, 282, 283, 284, 285, 286, 287, 292, 296, 298, 302, 307, 308, 309, 311, 314, 317, 319, 321, 323, 325 und 326 aufweist.

4. Verfahren nach einem der Ansprüche 1, 2 oder 3, **dadurch gekennzeichnet, dass** das Polypeptid wenigstens eine Mutation der Aminosäuresequenz in Bezug auf Sequenz ID-Nr. 1 gewählt aus der Gruppe D22A, S23Q, S23L, N25D, I26V, F27Y, F27H, S29P, R31A, F32Y, R35K, R35Q, V37A, V42I, V43T, F46Y, S51E, S51D, D53G, N54M, N54R, L57V, L60I, S69G, P72E, V73A, A78S, N80H, F84Y, I88L, T95S, T97A, R99K, I114M, 1118V, K119R, V123I, L132V, A141S, I146V, I146L, A148G, A149V, A154P, P163T, A164T, Y165C, Y165H, V169I, L170R, A172G, A176M, A176V, Y180F, D182T, F183Y, I190V, G191S, K194T, K194E, F196Y, V197C, V197R, E198R, E198S, K201D, K201G, P204S, P204A, A205S, K206P, A207M, M208A, Q209R, L210A, L210S, ∆P212, T213V, P214A, E216T, E216G, A217I, N220H, L221M, K222R, K222Q, G229A, A231V, F233W, F233Y, F233H, A238G, H240N, H240S, D244E, R245Q, M246L, S248T, S248N, S248G, Q249R, K251N, I254V, I256L, A260M, T262D, T262G, I263T, E266D, E269H, E269N, L271V, L277E, E280A, E280L, H281R, H281Q, A282V, Q283R, D284L, D284R, I285L, I286M, R287E, R287D, R292K, R292T, Q296A, Q296E, H298V, L302S, L307Q, F308S, D309A, A311P, A314V, L317F, S319Q, S319P, S319R, S321A, S321T, T323A, P325A, A326P aufweist.

5. Verfahren nach einem der Ansprüche 1 bis 4, **dadurch gekennzeichnet, dass** das Polypeptid wenigstens eines der folgenden Aminosäuremotive gewählt aus der Gruppe der Sequenz ID-Nrn. 32 bis 69 enthält.

6. Verfahren nach einem der Ansprüche 1 bis 4, **dadurch gekennzeichnet, dass** das Polypeptid in einem Zearalenon und/oder wenigstens ein Zearalenon Derivat spaltendem, Hilfsstoffe enthaltenden, Zusatzstoff zu Futtermitteln für Schweine, Geflügel und Aquakultur, in Nahrungsmittel oder in Trockenschlempe eingesetzt wird.

7. Verfahren nach Anspruch 6 , **dadurch gekennzeichnet, dass** die in dem Zusatzstoff enthaltenen Hilfsstoffe aus

wenigstens einem inerten Träger, sowie gegebenenfalls weiteren Bestandteilen, wie Vitamine und/oder Mineralstoffe und/oder Enzyme und/oder weitere Komponenten zur Detoxifizierung von Mykotoxinen gewählt werden.

8. Verfahren nach Anspruch 6 oder 7, **dadurch gekennzeichnet, dass** das Polypeptid in dem Zusatzstoff in einer Konzentration von höchstens 10.000 U/g, bevorzugt höchstens 1.000 U/g, noch bevorzugter höchstens 100 U/g und am bevorzugtesten höchstens 10 U/g eingesetzt wird.

9. Verfahren nach Anspruch 6, 7 oder 8, **dadurch gekennzeichnet, dass** der Zusatzstoff in verkapselter oder gecoateter Form eingesetzt wird.

10. Verfahren nach einem der Ansprüche 1 bis 9, **dadurch gekennzeichnet, dass** das Polypeptid oder der Zusatzstoff mit einem mit Zearalenon und/oder mit wenigstens einem Zearalenon-Derivat kontaminierten Futter- oder Nahrungsmittel versetzt wird, dass das kontaminierte Futter- oder Nahrungsmittel mit Feuchtigkeit in Kontakt gebracht wird und dass das Polypeptid oder der Zusatzstoff das im kontaminierten Futter- oder Nahrungsmittel enthaltene Zearalenon und/oder wenigstens ein Zearalenon-Derivat hydrolysiert.

11. Verfahren nach einem der Ansprüche 1 bis 10, **dadurch gekennzeichnet, dass** wenigstens 70 %, bevorzugt wenigstens 80 %, insbesondere wenigstens 90 % des Zearalenons und/oder wenigstens eines Zearalenon Derivats hydrolysiert werden.

12. Verwendung eines wenigstens ein Polypeptid mit einer Aminosäuresequenz gewählt aus der Gruppe der Sequenz ID-Nrn. 7, 8, 14 und 15, insbesondere Sequenz ID Nr. 14 oder einer funktionellen Variante davon, wobei eine Sequenzidentität zwischen der funktionellen Variante und wenigstens einer der Aminosäuresequenzen wenigstens 70% beträgt, enthaltenden Zusatzstoffs zur hydrolytischen Spaltung von Zearalenon und/oder wenigsten einem Zearalenon-Derivat in Futtermitteln, insbesondere für Schweine, Geflügel und Aquakultur, in Nahrungsmittel oder in Trockenschlempe.

## Claims

1. A method for hydrolytic cleavage of zearalenone and/or at least one zearalenone derivative by means of a hydrolytically cleaving polypeptide, **characterized in that** the zearalenone and/or at least one zearalenone derivative is hydrolysed by the polypeptide, which is a hydrolase having an amino acid sequence selected from the group of sequence ID numbers 7, 8 14 and 15, in particular sequence ID No. 14 or a functional variant thereof, wherein a sequence identity of at least 70 % exists between the functional variant and the amino acid sequence.

2. The method according to claim 1, **characterized in that** the polypeptide has at least one conserved amino acid sequence segment or a functional variant thereof, wherein the functional variant of the amino acid sequence segment has a sequence identity of at least 70 %, preferably at least 84 %, more preferably at least 92 % and most preferably at least 98 %, and at least one conserved amino acid sequence segment is selected from the group of amino acid sequences +24 to +50, +52 to +77, +79 to +87, +89 to +145, +150 to +171, +177 to +193, +223 to +228, +230 to +237, +239 to +247, +249 to +255, +257 to +261, +263 to +270, +272 to +279, +297 to +301 of the sequence having the sequence ID no. 1.

3. The method according to claim 1 or 2, **characterized in that** the polypeptide has at least one mutation of the amino acid sequence with respect to sequence ID no. 1 in at least one of the following positions selected from the group: 22, 23, 25, 26, 27, 29, 31, 32, 35, 37, 42, 43, 46, 51, 53, 54, 57, 60, 69, 72, 73, 78, 80, 84, 88, 95, 97, 99, 114, 118, 119, 123, 132, 141, 146, 148, 149, 154, 163, 164, 165, 169, 170, 172, 176, 180, 182, 183, 190, 191, 194, 196, 197, 198, 201, 204, 205, 206, 207, 208, 209, 210, 212, 213, 214, 216, 217, 220, 221, 222, 229, 231, 233, 238, 240, 244, 245, 246, 248, 249, 251, 254, 256, 260, 262, 263, 266, 269, 271, 277, 280, 281, 282, 283, 284, 285, 286, 287, 292, 296, 298, 302, 307, 308, 309, 311, 314, 317, 319, 321, 323, 325 and 326.

4. The method according to any one of claims 1, 2 or 3, **characterized in that** the polypeptide has at least one mutation of the amino acid sequence with respect to sequence ID no. 1 selected from the group D22A, S23Q, S23L, N25D, I26V, F27Y, F27H, S29P, R31A, F32Y, R35K, R35Q, V37A, V42I, V43T, F46Y, S51E, S51D, D53G, N54M, N54R, L57V, L60I, S69G, P72E, V73A, A78S, N80H, F84Y, I88L, T95S, T97A, R99K, I114M, 1118V, K119R, V123I, L132V, A141S, 1146V, I146L, A148G, A149V, A154P, P163T, A164T, Y165C, Y165H, V169I, L170R, A172G, A176M, A176V, Y180F, D182T, F183Y, I190V, G191S, K194T, K194E, F196Y, V197C, V197R, E198R, E198S, K201D,

K201G, P204S, P204A, A205S, K206P, A207M, M208A, Q209R, L210A, L210S, ΔP212, T213V, P214A, E216T, E216G, A217I, N220H, L221M, K222R, K222Q, G229A, A231V, F233W, F233Y, F233H, A238G, H240N, H240S, D244E, R245Q, M246L, S248T, S248N, S248G, Q249R, K251N, I254V, I256L, A260M, T262D, T262G, I263T, E266D, E269H, E269N, L271V, L277E, E280A, E280L, H281R, H281Q, A282V, Q283R, D284L, D284R, I285L, I286M, R287E, R287D, R292K, R292T, Q296A, Q296E, H298V, L302S, L307Q, F308S, D309A, A311P, A314V, L317F, S319Q, S319P, S319R, S321A, S321T, T323A, P325A, A326P.

5. The method according to any one of claims 1 to 4, **characterized in that** the polypeptide contains at least one of the following amino acid motifs selected from the group of sequence ID numbers 32 to 69.

6. The method according to one of claims 1 to 4, **characterized in that** the polypeptide is used in a zearalenone and/or zearalenone derivate cleaving, adjuvant-containing additive for feed for pigs, poultry and aquaculture, in food or in dry pasture.

7. The method according to claim 6, **characterized in that** the adjuvants contained in the additive are selected from at least one inert carrier as well as optional additional ingredients, such as vitamins and/or minerals and/or enzymes and/or other components for detoxifying mycotoxins.

8. The method according to claim 6 or 7, **characterized in that** the polypeptide is contained in the additive in a concentration of at most 10,000 U/g, preferably at most 1000 U/g, more preferably at most 100 U/g and most preferably at most 10 U/g.

9. The method according to claim 6, 7 or 8, **characterized in that** the additive is present in encapsulated or coated form.

10. The method according to one of claims 1 to 9, **characterized in that** the polypeptide or the additive is mixed with a foodstuff or animal feed product contaminated with zearalenone and/or with at least one zearalenone derivative; that the contaminated foodstuff or animal feed product is brought in contact with moisture, and that the polypeptide or the additive hydrolyses the zearalenone and/or at least one zearalenone derivative present in contaminated foodstuff or animal feed product.

11. The method according to any one of claims 1 to 10, **characterized in that** at least 70 %, preferably at least 80 %, in particular at least 90 % of the zearalenone and/or at the least one zearalenone derivative is/are hydrolysed.

12. A use of at least one polypeptide having an amino acid sequence selected from the group of sequence ID Nos. 7, 8 14 and 15, in particular sequence ID No. 14 or a functional variant thereof, whereby a sequence identity between the functional variant and at least one amino acid sequence amounts to at least 70 %, containing additives for hydrolytic cleavage of zearalenone and/or at least one zearalenone derivate in animal feed products, in particular pigs, poultry and aquaculture in food items or in distillers dried grain.

**Revendications**

1. Procédé pour la désagrégation par hydrolyse de zéaralénone et/ou au moins d'un dérivé de zéaralénone, **caractérisé en ce que** du zéaralénone et/ou au moins un dérivé de zéaralénone sont hydrolysés par un polypeptide avec une séquence d'acides aminés choisie parmi le groupe de séquence ID n° 7, 8, 14 et 15, en particulier la séquence ID n° 14 ou une variante fonctionnelle de ceux-ci, dans lequel une identité de séquence entre la variante fonctionnelle et au moins une séquence d'acides aminés représente est au moins 70 %.

2. Procédé selon la revendication 1, **caractérisé en ce que** le polypeptide contient au moins un segment de séquences d'acides aminés conservé ou une variante fonctionnelle de celui-ci, dans lequel la variante fonctionnelle du segment de séquence d'acides aminés présente une identité de séquence d'au moins 70 %, préférentiellement d'au moins 84 %, encore plus préférentiellement d'au moins 92 % et le plus préférentiellement d'au moins 98 % et l'au moins un segment de séquence d'acides aminés conservé est choisi parmi le groupe des séquences d'acides aminés +24 à 50, +52 à +77, +79 à +87, +89 à +145, +150 à +171, +177 à +193, +223 à +228, +230 à +237, +239 à +247, +249 à +255, +257 à +261, +263 à +270, +272 à +279, +297 à +301, de la séquence avec la séquence ID n° 1.

3. Procédé selon la revendication 1 ou 2, **caractérisé en ce que** le polypeptide présente au moins une mutation de la séquence d'acides aminés par rapport à la séquence ID n° 1 sur au moins une des positions suivantes choisies

parmi le groupe : 22, 23, 25, 26, 27, 29, 31, 32, 35, 37, 42, 43, 46, 51, 53, 54, 57, 60, 69, 72, 73, 78, 80, 84, 88, 95, 97, 99, 114, 118, 119, 123, 132, 141, 146, 148, 149, 154, 163, 164, 165, 169, 170, 172, 176, 180, 182, 183, 190, 191, 194, 196, 197, 198, 201, 204, 205, 206, 207, 208, 209, 210, 212, 213, 214, 216, 217, 220, 221, 222, 229, 231, 233, 238, 240, 244, 245, 246, 248, 249, 251, 254, 256, 260, 262, 263, 266, 269, 271, 277, 280, 281, 282, 283, 284, 285, 286, 287, 292, 296, 298, 302, 307, 308, 309, 311, 314, 317, 319, 321, 323, 325 et 326.

**4.** Procédé selon l'une quelconque des revendications 1, 2 ou 3, **caractérisé en ce qu'**il présente au moins une mutation de la séquence d'acides aminés par rapport à la séquence ID n° 1 choisie parmi le groupe D22A, S23Q, S23L, N25D, I26V, F27Y, F27H, S29P, R31A, F32Y, R35K, R35Q, V37A, V42I, V43T, F46Y, S51E, S51D, D53G, N54M, N54R, L57V, L60I, S69G, P72E, V73A, A78S, N80H, F84Y, I88L, T95S, T97A, R99K, I114M, I118V, K119R, V123I, L132V, A141S, 1146V, I146L, A148G, A149V, A154P, P163T, A164T, Y165C, Y165H, V169I, L170R, A172G, A176M, A176V, Y180F, D182T, F183Y, I190V, G191S, K194T, K194E, F196Y, V197C, V197R, E198R, E198S, K201D, K201G, P204S, P204A, A205S, K206P, A207M, M208A, Q209R, L210A, L210S, ∆P212, T213V, P214A, E216T, E216G, A217I, N220H, L221M, K222R, K222Q, G229A, A231V, F233W, F233Y, F233H, A238G, H240N, H240S, D244E, R245Q, M246L, S248T, S248N, S248G, Q249R, K251N, I254V, I256L, A260M, T262D, T262G, I263T, E266D, E269H, E269N, L271V, L277E, E280A, E280L, H281R, H281Q, A282V, Q283R, D284L, D284R, I285L, I286M, R287E, R287D, R292K, R292T, Q296A, Q296E, H298V, L302S, L307Q, F308S, D309A, A311P, A314V, L317F, S319Q, S319P, S319R, S321A, S321T, T323A, P325A, A326P.

**5.** Procédé selon une des revendications 1 à 4, **caractérisé en ce que** le polypeptide présente au moins un des motifs d'acides aminés suivants choisi parmi le groupe de la séquence ID n° 32 à 69.

**6.** Procédé selon une des revendications 1 à 4, **caractérisé en ce que**, le polypeptide capable d'entraîner un clivage de la zéaralénone et/ou de dérivés de zéaralénone est contenu dans un additif pour l'alimentation animale pour des porcs, des volailles et pour l'aquaculture.

**7.** Procédé selon la revendication 6, **caractérisé en ce que** les adjuvants contenus dans l'additif pour l'alimentation animale sont choisis parmi au moins un porteur inerte, ainsi qu'éventuellement d'autres constituants, tels que des vitamines et/ou des minéraux et/ou des enzymes et/ou d'autres composants servant à la détoxification de mycotoxines.

**8.** Procédé selon la revendication 6 ou 7, caractérisé en ce le polypeptide est contenu dans l'additif en une concentration de maximum 10 000U/g, préférentiellement au maximum de 1000 U/g, encore plus préférentiellement au maximum de 100 U/g et le plus préférentiellement au maximum de 10 U/g.

**9.** Procédé selon l'une quelconque des revendications 6, 7 ou 8, **caractérisé en ce que** l'additif est présent sous une forme encapsulée ou enrobée.

**10.** Procédé selon l'une quelconque des revendications 1 à 9, **caractérisé en ce que** le polypeptide ou l'additif est mélangé à un aliment pour animaux ou à une denrée alimentaire contaminé(e) par de la zéaralénone et/ou par au moins un dérivé de zéaralénone, que l'aliment pour animaux ou la denrée alimentaire contaminé(e) est amené(e) en contact avec de l'humidité, et que le polypeptide ou l'additif hydrolyse le zéaralénone et/ou au moins un dérivé de zéaralénone contenu dans l'aliment pour animaux ou la denrée alimentaire contaminé(e).

**11.** Procédé selon l'une quelconque des revendications 1 à 10, **caractérisé en ce qu'**au moins 70 %, préférentiellement au moins 80 %, en particulier au moins 90 % du zéaralénone et/ou au moins d'un dérivé de zéaralénone sont hydrolysés.

**12.** Utilisation d'un additif pour la désagrégation par hydrolyse de zéaralénone et/ou au moins d'un dérivé de zéaralénone dans des aliments pour animaux, en particulier pour des porcs, des volailles et pour l'aquaculture, dans des denrées alimentaires ou dans du lisier séché contenant au moins un polypeptide avec une séquence d'acides aminés choisie parmi le groupe de séquence ID n 7, 8, 14 et 15, en particulier la séquence ID n° 14 ou une variante fonctionnelle de ceux-ci, dans laquelle une identité de séquence entre la variante fonctionnelle et au moins une séquence d'acides aminés représente est au moins 70 %.

Fig. 1

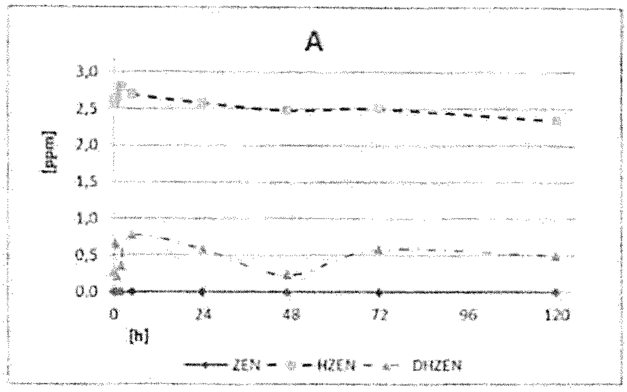


Fig. 1A

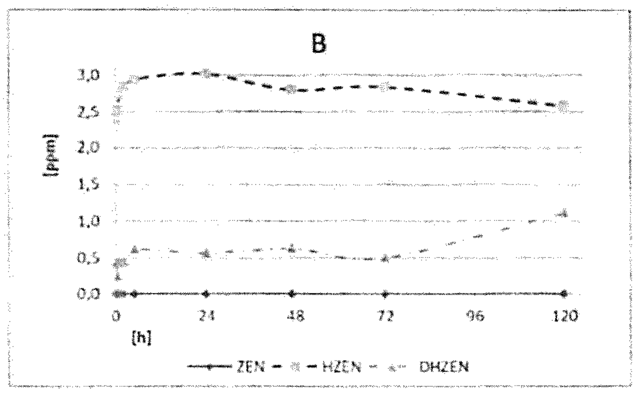


Fig. 1B

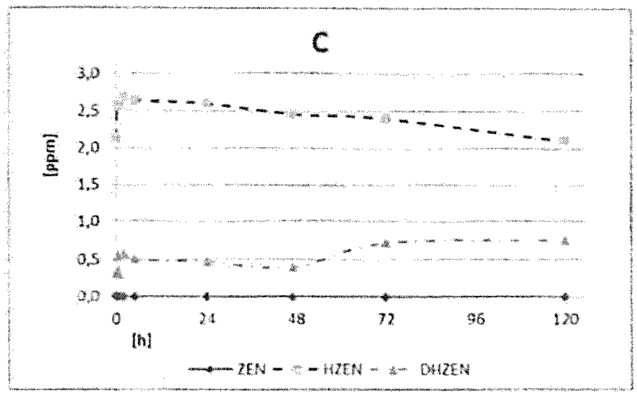


Fig. 1C

Fig. 2

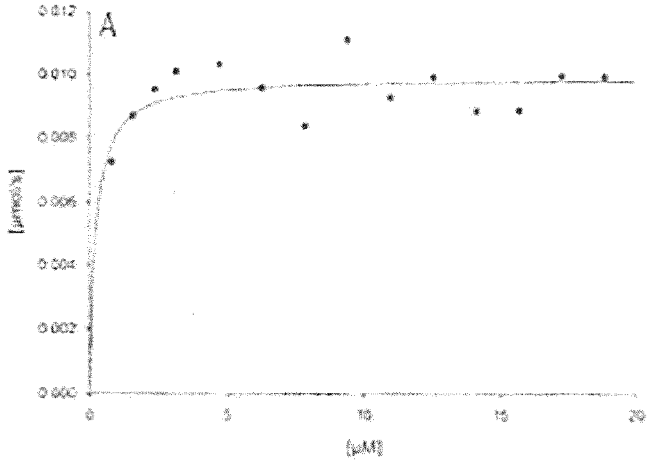


Fig. 2A

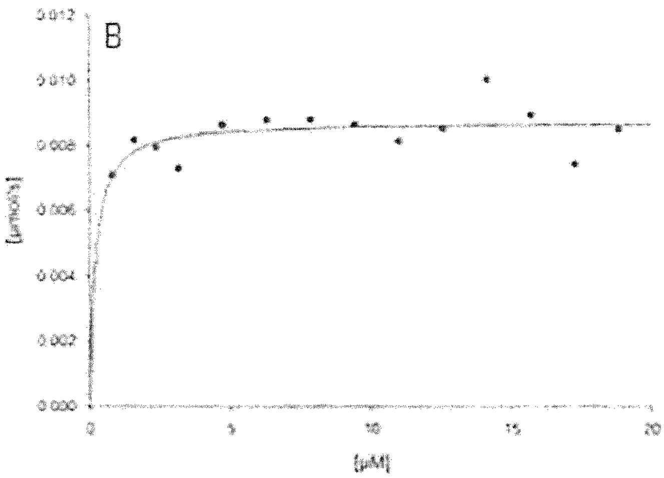


Fig. 2B

Fig. 3A

SEQ 1

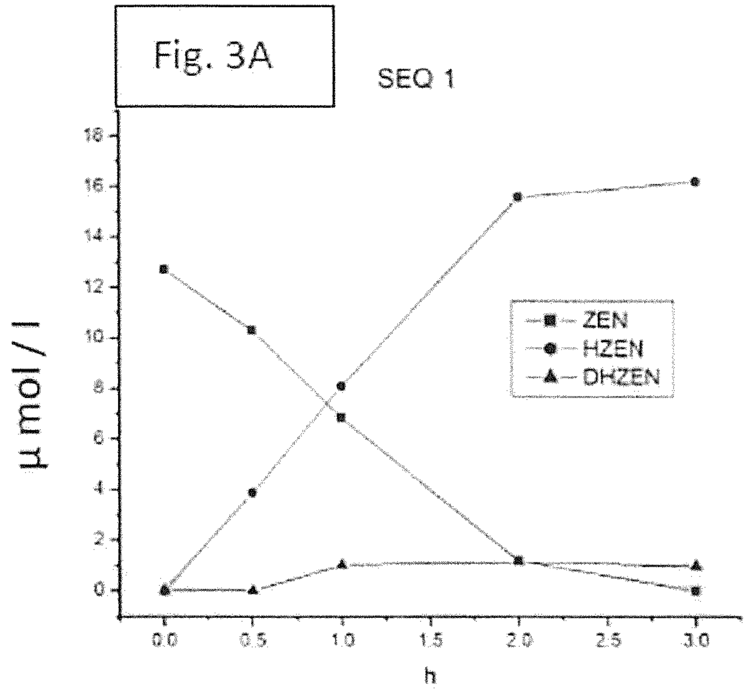


Fig. 3E

SEQ 7

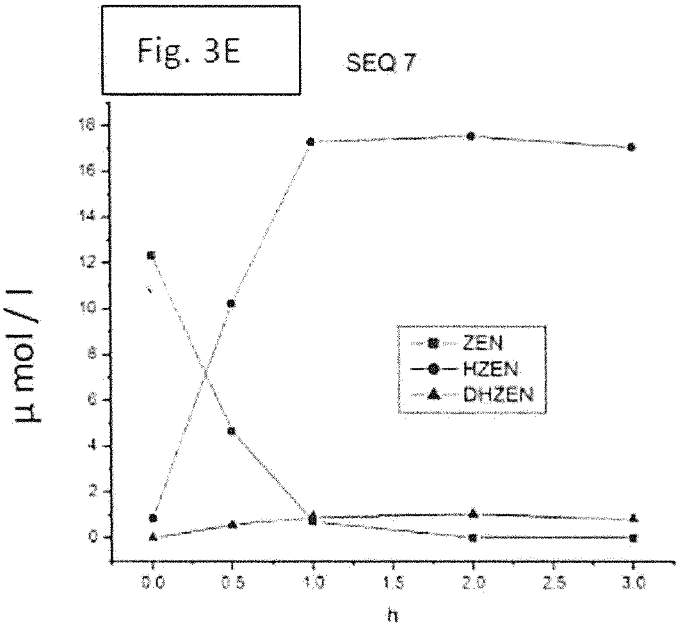

Fig. 3I

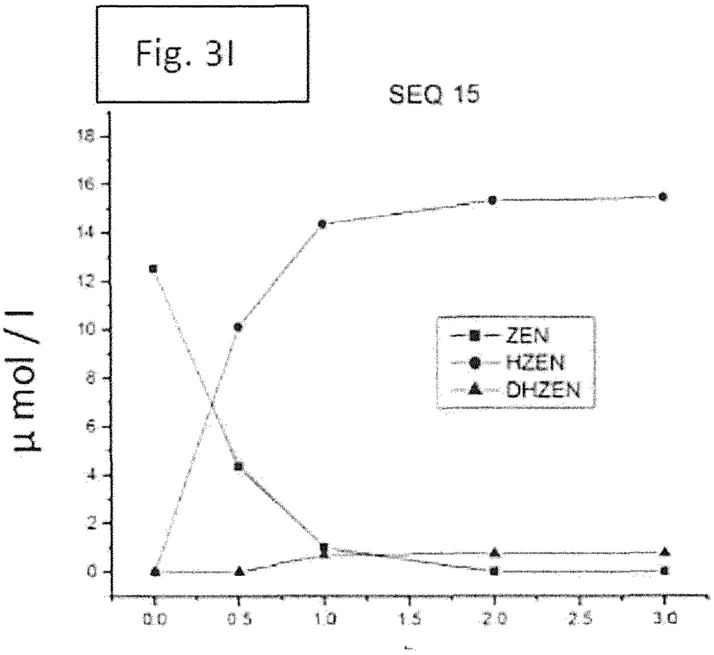
SEQ 15
µ mol / l
ZEN
HZEN
DHZEN
0
2
4
6
8
10
12
14
16
18
0.0
0.5
1.0
1.5
2.0
2.5
3.0

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0938575 B1
- WO 02076205 A
- WO 2012113827 A
- WO 9212645 A
- EP 3039134 A


### In der Beschreibung aufgeführte Nicht-Patentliteratur


- **E. VEKIRU et al.** *Appl. and Environ. Microb.,* 2010, vol. 76 (7), 2353-2359
- *SubName: Full=Alpha/beta hydrolase fold containing protein {ECO:0000313|EMBL:AEM80235.1,* 16. November 2011
- **VON ALTSCHUL et al.** *Nucleic Acids Res.,* 1997, vol. 25, 3389-3402
- **J.M. CREGG.** Pichia Protocols. 2007
- **J. SAMBROOK et al.** Molecular Cloning, A Laboratory Manual. Cold Spring Harbor, 2012
- **GASTEIGER E. et al.** *Protein Identification and Analysis Tools on the ExPASy Server*
- The Proteomics Protocols Handbook. Humana Press, 2005, 571-607
- **J.S. PAPADOPOULOS ; R. AGARWALA.** COBALT: constraint-based alignment tool for multiple protein sequences. *Bioinformatics,* 2007, vol. 23, 1073-79
- **P. KRENN et al.** *Mykotoxin Research,* 2007, vol. 23 (4), 180-184
- **M. SULYOK et al.** *Anal. Bioanal. Chem.,* 2007, vol. 289, 1505-1523
- **VON M. SULYOK et al.** *Food Chemistry,* 2010, vol. 119, 408-416
- **VON P. SONGSERMASKUL et al.** *J. of Animal Physiol. and Animal Nutr.,* 2011, vol. 97, 155-161
- **H. BISSWANG.** *Enzyme Kinetics,* 2002, ISBN 3-527-30343-X, 19
- **TEORELL ; STENHAGEN.** Ein Universalpuffer für den pH-Bereich 2.0 bis 12.0. *Biochem Ztschrft,* 1938, vol. 299, 416-419